# EUROPEAN PATENT APPLICATION

(11) **EP 1 905 439 A1**
(43) Date of publication of application: **02.04.2008**
(21) Application number: 07114786.2
(22) Date of filing: 22.08.2007
(51) Int. Cl.: A61K 31/337, A61K 31/56, A61K 31/573, A61K 33/24, A61P 35/00, A61K 31/137, A61K 31/475, A61K 31/565

(54) **Second line treatment of metastatic hormone refractory prostate cancer using satraplatin**

(30) Priority: 24.09.2006 US 846968 P; 22.02.2007 US 903008 P; 23.02.2007 US 903180 P
(71) Applicant: GPC Biotech AG, 82152 Planegg-Martinsried (DE)
(72) Inventor: McKearn, Thomas J., New Hope, Pennsylvania 18938 (US); Rozencweig, Marcel, Princeton, NJ 08540 (US)
(74) Representative: Dörries, Hans Ulrich

(57) **Abstract**

The instant invention relates to methods using satraplatin, packaged-pharmaceutical-products that include satraplatin and uses of satraplatin to prepare pharmaceutical compositions for the 2^{nd} line treatment of metastatic hormone refractory prostate cancer.

## Description

### Background to the invention

### Prostate Cancer

Worldwide, prostate cancer ranks as the second most common cancer in males, after lung cancer, and in the United States (U.S.) prostate cancer is the second leading cause of death from cancer in men. There were over 180,000 new cases and 29,000 deaths reported in the U.S. in the year 2002 (American Cancer Society). The frequency of patients presenting at each stage of disease has changed remarkably with introduction of prostate specific antigen (PSA) screening in the early 1990s.

Approximately 30-35% of patients with prostate cancer will present with regional or metastatic tumors, while an additional 25% will develop metastases in the course of the disease. Metastases are commonly to the bone, where the lesions can be seen on X-ray as osteosclerotic lesions or on a bone scan as areas of increased activity or "hot spots." In patients presenting with metastatic disease and receiving androgen ablation, median survival is 2.5 years (Sternberg, 1992). In many such patients, bone pain and decreased performance status are predominant. Relief from these symptoms is as important as prolongation of survival. As a result, assessment of these symptoms, including pain, has become a fundamental part of many prostate cancer studies.

Patients with metastatic disease are initially treated with hormone therapy such as luteinizing hormone releasing hormone (LHRH) agonists, diethylstilbestrol (DES), orchiectomy, and/or anti-androgens. The development of hormonal resistance occurs in most patients after androgen deprivation. The term "hormone-refractory prostate cancer" (HRPC) is used by physicians to describe prostate cancer disease that progresses despite castrate levels of serum testosterone.

The median time to progression to HRPC is 18 months from the time of initiation of hormonal therapy against prostate cancer. Responses to current second line hormonal therapies are temporary and do not impact upon survival. The median survival after developing HRPC has been 12 to 18 months, and until recently, there was no clearly effective systemic treatment for this condition. With recent advances in the understanding of HRPC, novel treatment regimens are being identified. In the past, all treatments involving cytotoxic chemotherapy were considered inactive, but newer chemotherapy drugs and drug combinations are now demonstrating improved response rates (Kelly, 2000) and improved survival (Petrylak, 2004; Eisenberger, 2004).

Evaluation of tumor response in advanced prostate cancer has been difficult due to the predominance of non-measurable bony metastases and the infrequent presence of measurable lesions. More recently, PSA level has been used to evaluate the disease status of patients with prostate cancer.

PSA level is generally considered to be a useful surrogate measure in patients receiving hormonal therapy (Bubley, 1999; Miller, 1992), and it may be useful as well in patients receiving therapy for hormone refractory disease (Bubley, 1999; Kelly, 1993). There may be limitations for use of PSA levels to monitor disease in this population, however, since any new therapy may modulate PSA production by tumor cells independently of its effect or lack of effect on tumor growth (Eisenberger, 1996).

### Satraplatin

Satraplatin (INN / USAN), also known as JM-216, or bis (acetato) ammine dichloro (cyclohexylamine) platinum (IV), is a member of a novel class of platinum (IV) compounds that are absorbed by the oral route. The lipophilic properties of these compounds, and hence their absorption, are largely determined by the nature of the axial acetate ligands. Unlike the square planar platinum (II) complexes cisplatin and carboplatin, satraplatin is an octahedral platinum (IV) compound.

The molecular formula for satraplatin is C₁₀H₂₂N₂Cl₂O₄Pt. Its molecular weight is 500.29. Its chemical structure is:

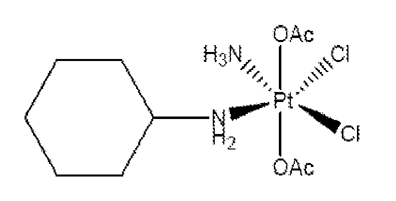

Satraplatin can be synthesised according to the method disclosed in U.S. Patent No. 5,072,011 and 5,244,919 or by appropriate modification of the method disclosed in US 6,518,428.

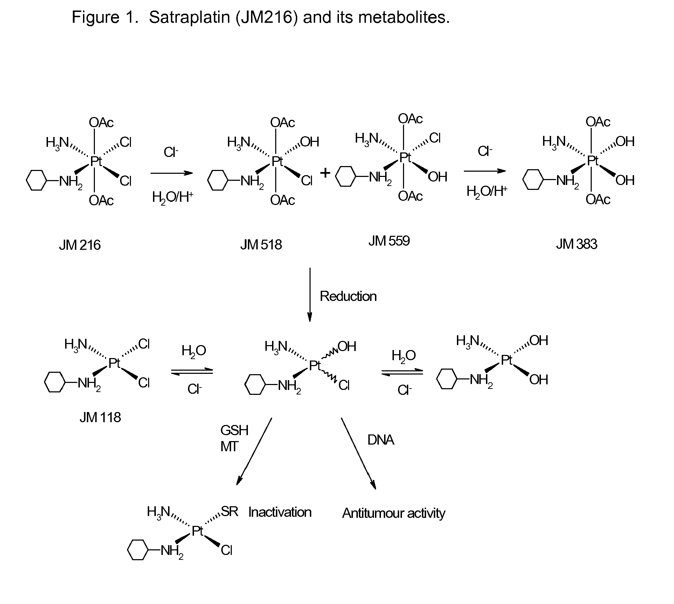

Upon administration of satraplatin to a cell, animal or a human patient, a number of metabolites may be formed. Figure 1 (taken from Raynaud et al. 1996 Cancer Chemother. Phamacol. 38: 155-162) shows exemplary metabolites of satraplatin (JM216), and depicts JM118, JM383, JM518, JM559 and JM149.

### Differences between satraplatin and other platinum-containing therapeutic agents

Satraplatin is a third-generation platinum compound studied in a variety of tumors. Since their original discovery, platinum compounds (cisplatin, carboplatin, oxaliplatin) have emerged as important agents for the therapy of several human tumors including testicular, bladder, lung, colorectal, head and neck, ovarian, and cervical cancer (Rozencweig, 1977; Loehrer, 2984; Prestayko, 1979). Cisplatin, used as single agent, has been evaluated in several trials for the treatment of hormone refractory carcinoma of the prostate (e.g. Rossof, 1979; Merrin, 1979; Yagoda, 1979(I); Yagoda, 1979(II); Qazi, 1983; Soloway, 1983; Moore, 1986). The primary endpoints in these studies were response rate in measurable disease. The response rates to single agent cisplatin are generally low or poor (see below) and comparable to those seen with other agents in this disease (Rossof, 1979; Yagoda, 1993). Furthermore, cisplatin was repeatedly shown not to be effective against prostate cancer. Qazi & Khandekar (Am. J. Clin. Oncol. (1983) 6, 203) demonstrated in a phase II trial that cisplatin is not effective in patients with metastatic prostatic carcinoma. Hasegawa et al. (Cancer & Chemother. (1987) 14, 3279) reported that the range of effective dose was wider for other platinum agents like carboplatin than for cisplatin. Even in combination treatment, cisplatin-comprising regimens demonstrate limited activity, e.g. in combination with mitoxantrone in metastatic prostate cancer (Osborne et al., Eur. J. Cancer (1992) 28, 477). Therefore, cisplatin has not been established as compound for chemotherapy of prostate cancer.

Although (i) pre-clinical studies with satraplatin demonstrated cytotoxic and anti-tumor activities comparable to cisplatin or carboplatin, and (ii) early clinical studies demonstrated its activity against platinum-sensitive tumors of the ovary and lung in addition to the prostate; satraplatin shows considerable and significant differences to other platinum agents, like e.g. cisplatin. It has shown activity in some platinum resistant tumor models *in vitro,* and unlike other platinum compounds, it is absorbed when administered orally. Using a panel of ovarian cancer carcinoma cell lines, Kelland et al. (Cancer Res (1992), 52, 822) demonstrated that satraplatin is significantly more cytotoxic than cisplatin, and that satraplatin exhibits selective cytotoxic effects against intrinsically cisplatin-resistant cell lines. Loh et al. (Br. J. Cancer (1992) 66, 1109) confirmed these findings. Loh et al. furthermore came to the conclusion that the increased accumulation of satraplatin, which is a result of its enhanced lipophilicity, accounts for the dramatic increase of the potency of satraplatin over cisplatin. Other studies reporting on the activity of satraplatin towards cell lines with acquired or intrinsic resistance to cisplatin are those of Mellish et al. (Br J Cancer (1993) 68, 240), using human cervical squamous cell carcinoma cell lines, and Orr et al. (Br J Cancer (1994) 70, 415), using murine leukemia cell lines. In the latter report the cell lines used were not just resistant to cisplatin, but also to tetraplatin and carboplatin.

Twentyman et al. (Cancer Res (1992) 52, 5674) investigated the sensitivity of human lung cancer cell lines with acquired or inherent resistance to cisplatin, to a series of novel platinum compounds, including satraplatin. In this study, cisplatin and carboplatin were found to act very similar, whereas satraplatin did not.

In spite of different routes of administration Kelland et al. (Int. J. Oncol. (1993) 2, 1043) demonstrated the surprising finding that the efficacy of orally administered satraplatin is comparable to that of cisplatin and carboplatin administered intravenously, as determined in human ovarian carcinoma xenograft models. These findings were confirmed by Rose et al. (Cancer Chemother. Pharmacol. (1993) 32, 197), using murine and human tumor models. McKeage et al. (Cancer Res. (1994) 54, 4118) investigated the differences of the schedule dependencies associated with these routes of administration.

In another study by Kelland et al. (Cancer Res. (1993) 53, 2581) many of the above mentioned differences between satraplatin and cisplatin were confirmed. Furthermore it was found, that the cytotoxicity of satraplatin was dependent on the time of drug exposure. Again, it was confirmed that satraplatin does not exhibit cross resistance to cisplatin, whereas other platinum agents, e.g. tetraplatin, do. Without being bound to any particular theory, satraplatin circumvents transport-determined acquired resistance to cisplatin.

Mellish et al. (Cancer Res. (1994) 54, 6194) investigated the mechanisms of acquired resistance to satraplatin in two human ovarian carcinoma cell lines. They found that, in contrast to cisplatin, acquired resistance to satraplatin is not mediated through reduced drug accumulation, but by increased intracellular GSH levels or increased DNA repair.

Sharp et al. (Clin. Cancer Res. 1995, 1, 981) compared the transport of cisplatin and satraplatin in human ovarian carcinoma cell lines. Cisplatin transport in the parental cell lines occurs via passive diffusion and active/facilitated transport, whereas in a cisplatin-resistant cell lines cisplatin enters cells by passive diffusion only. Without being bound to any particular theory, satraplatin circumvents cisplatin resistance by increasing the drug uptake. The mechanism of satraplatin transport across cell membranes is through passive diffusion, predominantly as a result of its enhanced lipophilicity.

Fink et al. (Cancer Res (1996) 56, 4881) investigated the effect of the loss of DNA mismatch repair activity on the sensitivity to cisplatin, satraplatin and other platinum agents. In contrast to cisplatin and carboplatin, which form the same type of adducts in DNA, there was no difference in sensitivity between mismatch repair-proficient and mismatch repair-deficient cell lines for satraplatin.

Perego et al. (Mol. Pharmacol. 1998, 54, 213) investigated the sensitivity of strains of Schizosaccharomyces pombe to cisplatin, satraplatin and other platinum compounds. The panel of the 23 yeast strains tested comprised many mutants in genes that affect the response to radiation. Whereas the mutants fell into three groups with respect to their sensitivity to cisplatin (minimal change in sensitivity, hypersensitivity, and marked hypersensitivity), none of the mutants demonstrated an appreciable change in sensitivity to satraplatin.

Leyland-Jones et al. (Amer. J. Pathol. 1999, 155, 77) investigated genomic imbalances associated with acquired resistance to platinum analogues. Using three ovarian carcinoma cell lines they identified differences between the three platinum compounds cisplatin, satraplatin and AMD473 (picoplatin).

Amorino et al. (int. J. Radiation Oncol. Biol. Phys. 1999, 44, 399) investigated radiopotentiation by satraplatin and the role of repair inhibition. They found that satraplatin can potentiate the effects of radiation in human lung cancer cells, and that the mechanism of this effect is probably inhibition of DNA repair by satraplatin. Differences to other platinum drugs like cisplatin and carboplatin are indicated.

Vaisman et al. (Biochemistry 1999, 38, 11026) reported on the effects of DNA polymerases and high mobility group protein 1 on the carrier ligand specificity for translesion synthesis past platinum-DNA adducts, with respect to different platinum compounds.

Screnci et al. (Br J Cancer (2000) 82, 966) investigated the relationship between hydrophobicity, reactivity, accumulation and peripheral nerve toxicity of a series of platinum compounds. According to Screnci et al. the hydrophilicity of platinum drugs correlates with platinum sequestration in the peripheral nervous system, but not with neurotoxicity.

Wei et al. (J. Biol. Chem. 2001, 276, 38774) reported on the effect of ligands on the specific recognition of intrastrand platinum-DNA cross-links by high mobility group box and TATA-binding proteins, with respect to different platinum compounds.

Fokkema et al. (Biochem. Pharmacol. 2002, 63, 1989) analysed in detail the satraplatin-, JM118-, and cisplatin-induced cytotoxicities in relation to various parameters like platinum-DNA adduct formation, glutathione levels and p53 status in human tumor cell lines with different sensitivities to cisplatin. It was confirmed that satraplatin and JM118 can partially circumvent intrinsic and acquired resistance to cisplatin. At equimolar basis, satraplatin induced lower levels of platinum-DNA adducts in the cell lines tested compared to cisplatin.

Taken together, fundamental differences exist between satraplatin and other platinum agents, such as cisplatin. These differences are the basis, lead to or play a role in many of the different characteristics of satraplatin, including different pharmacokinetic properties, different efficacy, a different toxicology profile, different ADME properties and different mechanisms that lead to drug resistance, only to name a few.

### Relevant in-vitro & pre-clinical investigations with satraplatin

A number of preclinical investigations have been conducted using satraplatin during its development as a chemotherapeutic. In particular, the results of the following investigations have been published:

### Satraplatin in models of prostate cancer and PSA response

Wosikowski et al (AACR meeting: Basic, translational, and clinical advances in prostate cancer in Florida. November 17-21, 2004) reported that treatment of prostate cancer cells with satraplatin or an active metabolite, JM118, resulted in tumor cell kill. The androgen-insensitive prostate cancer cell lines PC-3 and DU 145 was shown to be more sensitive to satraplatin than the androgen-sensitive LNCaP cell line. JM118 and JM518 were the most active metabolites of satraplatin and up to 16-fold more active than satraplatin.

Jung et al. (57. Jahrestagung der Deutschen Gesellschaft für Urologie, September 2005) and Wosikowski et al. (AACR meeting: Basic, translational, and clinical advances in prostate cancer in Florida. November 17-21, 2004) reported that treatment of LNCaP cells for 42 hours with 12 µM satraplatin or 0.7 µM JM-118 resulted in a decrease in cell number (56% and 61% of control, respectively) and decrease in secreted PSA protein level (58% and 61% of control, respectively). However, there was no effect on PSA mRNA transcription (90% of control).

The pre-clinical evaluation of satraplatin and JM118 in human prostate cancer cell lines was also described by Wosikowski et al. on the Prostate Cancer ASCO meeting; San Francisco, Feb 24-26, 2006.

Lamphere et al have reported: (i) the synergistic antitumor activity of the combination of satraplatin (S) and docetaxel (D) in H460 human non-small cell lung carcinoma (NSCLC) xenografted in nude mice (AACR April 1-5, 2006 Washington, D.C. USA); and (ii) the antitumor activity of satraplatin in combination with paclitaxel in the H460 human non small cell lung carcinoma (NSCLC) xenografted in nude mice (AACR-NCI-EORTC International Conference on Molecular Targets and Cancer Therapeutics: Discovery, Biology, and Clinical Applications, Nov 14-18, 2005, Philadelphia, PA).

Further, Lamphere et al have reported the synergistic antitumor activity of the combination of satraplatin (S) and paclitaxel (P) and the combination of satraplatin (S) and docetaxel (D) in prostate carcinoma models (ASCO 2006 Annual meeting, Atlanta, 2-6 June 06; AACR-NCI-EORTC International Conference on Molecular Targets and Cancer Therapeutics: Discovery, Biology, and Clinical Applications, Nov 14-18, 2005, Philadelphia, PA).

PCT/EP2006/060615 describes that in various *in-vitro* and xenograft models of cancers including prostate cancer, satraplatin acts synergistically with certain other non-platinum-containing chemotherapeutic agents including taxanes such as paclitaxel (Taxol®) and docetaxel (Taxotere®).

Obermayr et al. (AACR-NCI-EORTC International Conference on Molecular Targets and Cancer Therapeutics: Discovery, Biology, and Clinical Applications, Nov 14-18, 2005, Philadelphia, PA) reported the synergistic in vitro anticancer activity with sequential schedules of JM-118, a metabolite of satraplatin, in combination with erlotinib, paclitaxel or 5-FU.

EP 05024701.4 describes that in various *in-vitro* models of cancers, satraplatin acts synergistically with certain other non-platinum-based chemotherapeutic agents that include (i) inhibitors of receptors of the EGFR family, such as herceptin and erlotinib, and (ii) active pyrimidine analogues, such as gemcitabine, 5FU or prodrugs thereof.

### Satraplatin in models of resistant or refractory cancers

Cisplatin, carboplatin and oxaliplatin have shown clinical activity in testicular, ovarian, head and neck, small and non-small cell lung, and colon carcinomas. However, the effectiveness of these compounds has been limited due to intrinsic or acquired resistance. Proposed mechanisms of cisplatin resistance include increased DNA tolerance, reduced cellular accumulation of cisplatin and enhanced cellular detoxification of platinum complexes.

Various *in-vitro* studies show that satraplatin and JM-118 are able to overcome several of the mechanisms of resistance to cisplatin including those attributed to alterations in the DNA repair processes and platinum transport, and to mechanism associated with the resistance of cancers to certain non-platinum chemotherapeutic compounds. In particular: (i) satraplatin does not exhibit cross-resistance to a number of cisplatin-resistant cell lines; and (ii) resistance mechanisms that confer resistance to non-platinum based chemotherapeutic agents, i.e., taxanes, doxorubicin, vincristine, etoposide, mitoxantrone and camptothecin, generally do not confer cross-resistance to satraplatin or JM-118.

Kishimoto et al (2006 AACR meeting in Washington, 1-5 April 06) reported the differences in the mechanisms of resistance to cisplatin and to JM-118, an active metabolite of satraplatin.

Wosikowski et al. (AACR Annual Meeting in Anaheim, 16-20 April 05) reported the efficacy of satraplatin (JM216) and JM118 in certain drug resistant cells, and in combination with docetaxel.

WO 05/077385 describes that satraplatin is effective in the treatment of models of cancers and tumors that are resistant or refractory to certain other chemotherapeutic agents, including: (i) those cancers and tumors wherein the resistance mechanism is mediated by multidrug resistance mechanisms such as ABC transporters; (ii) cancers and tumors wherein the resistance mechanism is mediated by tubulin; and (iii) cancers and tumors wherein the resistance mechanism is mediated through topoisomerase. In particular, satraplatin was shown to be effective in models of cancer refractory or resistant to certain taxanes, including paclitaxel and docetaxel.

### Clinical studies of the efficacy of satraplatin against hormone resistant prostate cancer

A number of clinical studies have been conducted with satraplatin, and the results of these are summarised in Sternberg et al (BJU International, 2005, p.990-994). Many of such clinical studies have investigated the pharmacology, toxicology and other safety of satraplatin in human subjects. Others clinical trials have tested the efficacy of satraplatin against a number of different cancers. The results of some of such clinical trials that set out to test the efficacy of satraplatin against hormone resistant prostate cancer have been published and are described below.

### Study 1

A pilot multicenter open-label phase II study to evaluate the efficacy and safety of satraplatin as a single-agent for first-line treatment of patients with hormone refractory prostate cancer was conducted in the U.S. (Peereboom et al: Proc. Am. Soc. Clin. Oncol. 16: 339a, 1997 & Latif et al; Investigational New Drugs 23: 79, 2005). Satraplatin was administered daily for 5 days every 4 weeks at a starting dose of 120 mg/m² per day. An interim analysis (Peereboom et al: Proc. Am. Soc. Clin. Oncol. 17: 314a, 1998) concluded that satraplatin is an active and convenient drug against HPRC and has manageable toxicities, whilst Latif et al concluded that although satraplatin had moderate activity in HRPC, it is associated with significant treatment-related toxicities in this patient population. The trial however was open-labeled, and hence any effect observed, is confounded by placebo effect.

### Study 2

A multicenter, randomized phase III study was designed to evaluate the efficacy and safety of satraplatin for first-line treatment of patients with HRPC (Sternberg et al: Proc. Am. Soc. Clin. Oncol. 22:395, 2003; Sternberg et al: Oncology 2005; 68:2-9). Patients were randomized between satraplatin 100 mg/m2 for 5 days plus prednisone 10 mg orally BID or prednisone alone. After 50 randomized patients, the trial was closed to further accrual by the sponsoring company. Median overall survival was 14.9 months (95% Cl: 13.7-28.4) on the satraplatin plus prednisone arm and 11.9 months (95% Cl: 8.4-23.1) on prednisone alone (hazard ratio, HR = 0.84, 95% Cl: 0.46-1.55). A >50% decrease in prostate specific antigen (PSA) was seen in 9/27 (33.3%) in the satraplatin plus prednisone arm vs. 2/23 (8.7%) on the prednisone alone arm. Progression-free survival was 5.2 months (95% Cl: 2.8-13.7) on the satraplatin plus prednisone arm as compared to 2.5 months (95% Cl: 2.1- 4.7) on the prednisone alone arm (HR = 0.50, 95% Cl: 0.28-0.92). This randomized comparison of a combination of satraplatin and prednisone versus prednisone alone was suggestive of the antitumor activity of the combination. It was concluded that a role for satraplatin in the treatment of HPRC remains to be elucidated in an appropriate phase III setting. Other factors and parameters like pain progression and PSA levels were not followed up in this trial and no conclusion were made in these respects.

### Combination studies of satraplatin against cancer

As described above, satraplatin has shown increased efficiency when used in combination with certain other chemotherapeutics in a number of pre-clinical models of cancer. In certain early-phase clinical studies, satraplatin has been studied and when used in combination with the other therapeutics, including paclitaxel (Jones et al; Invest New Drugs 20: 55.61, 2002). In certain studies of the efficacy of satraplatin against hormone refractory prostate cancer (including that described as "Study 2 above), it has been used in combination with prednisone, a corticosteroid. Such trials have been conducted as so called "two-arm" trails, with one set of patients being treated with satraplatin plus prednisone, and the other set of patients treated with placebo plus prednisone. It has been considered unethical for clinical studies of HRPC to be conducted as "three-arm" trials; that is a trial in which a third arm is used to investigate and compare the efficacy of satraplatin alone (plus a placebo for the prednisone). Hence, it has been ethically impossible, and shall remain so, to investigate in clinical studies the potential synergy of such combinations by the use of such appropriate experimental design.

In summary, there is substantive preclinical *in vitro* and *in vivo* information for satraplatin, including information from *in vitro* or *in vivo* models for prostate cancer, and there are substantive results obtained in various clinical studies using satraplatin in various oncology indications, including as a first-line treatment of hormone refractory prostate cancer in combination with prednisone. Based on these suggestive studies, the SPARC ("Satraplatin and Prednisone Against Refractory Cancer") phase III clinical study described in the Exemplification was started. However, while one could *hope* that the SPARC trial would be successful, one *would not* have had an *expectation* of actually achieving a statistically significant positive outcome in light of (i) the limitations in making predictions for clinical study results based on preclinical information, (ii) the known failure rate for phase III clinical trials, (iii) the complexity and severity of the underlying condition to be treated, and (iv) the limited success in treating hormone refractory prostate cancer (see below).

### Chemotherapy for Hormone Refractory Prostate Cancer

Until recently, the response of HRPC to cytotoxic agents, both singly and in combination, has been less than satisfactory (Pienta, 1994; Dawyson, 1993; Eisenberger, 1985; Yagoda, 1993). Objective disease regression occurs in approximately 10% to 20% of cases. Most responses are only partial. In a literature review of 3184 patients, the overall response rate (CR + PR) was only 7% (Yagoda, 1993). When the stable category was added, this figure increased by only 15% to 22%.

In the United States, there are two regimens approved for the first line treatment of HRPC: mitoxantrone plus a corticosteroid (e.g. prednisone) and docetaxel plus prednisone. Mitoxantrone is an anthracenedione that is effective, when combined with prednisone, in producing a palliative response using pain response criteria, in symptomatic patients (29% versus 12% with prednisone alone, p = 0.01) (Tannock, 1996). The mitoxantrone plus corticosteroid regimen was thus approved as palliative treatment based on improvement in pain (Tannock, 1996). Despite the improvement in pain symptoms, however, no improvement in survival was observed with the combination therapy.

Prednisone therapy alone has been associated with an improved survival duration when compared to liarozole, a retinoic acid metabolism-blocking agent, for patients with hormone refractory prostate cancer (Oncology Drug Advisory Committee to the Food & Drug Administration, June 1997). Prednisone is normally used in combination with mitoxantrone at the dose of 5 mg twice daily for patients with symptomatic hormone refractory prostate cancer.

It has been recognized for many years that corticosteroids, such as prednisone, have a definite palliative and sometimes objectively beneficial effect on the clinical course of patients with hormone-refractory prostate cancer. Among the corticosteroids other than prednisone that have been investigated for use in therapies against HRPC are dexamethasone (Nelius et al., BJU Int. 2006, 98, 580-5; Odrazka et al., Oncol. Rep. 2005, 14, 1077-81; Storlie et al., Cancer 1995, 76, 96-100), hydrocortisone (Abratt et al., Ann. Oncol. 2004, 15, 1613-21; Kruit et al., Anticancer Drugs 2004,15, 843-7), cortisone acetate (Ponder et al., Br. J. Cancer 1984, 50, 757-63), and prednisolone (Heidenreich, J. Urol. Urogynäkol. 2004, 11, special edition 6 (edition for Austria), 15-19).

Estramustine is a mixed hormonal and alkylating agent. It is available in Europe, Australia and the U.S. for palliative treatment of patients with metastatic and/or progressive carcinoma of the prostate. Recent reports from clinical trials suggest that the combination of estramustine with either paclitaxel or docetaxel is well tolerated and produces a decrease of > 50% in serum PSA levels in more than 50% of hormone refractory prostate cancer treated patients (Hudes, 1997; Petrylak, 1999; Hussain, 1999).

The taxanes, Taxol® (paclitaxel) and Taxotere® (docetaxel), have activity in hormone refractory prostate cancer when used alone or in combination with other cytotoxic agents (Hudes, 1997; Petrylak, 1999; Petrylak, 2004; Eisenberger, 2004). The results of two recent studies, SWOG 99-16 and TAX327, demonstrate a survival advantage in the docetaxel arms compared to mitoxantrone and prednisone (Petrylak, 2004; Eisenberger, 2004). Taxotere was recently approved by the FDA for use as first-line chemotherapy in patients with HRPC in combination with prednisone. The efficacy data generated through these two phase 3 randomized trials demonstrated for the first time a clinical benefit (survival advantage) for patients treated with chemotherapy for HRPC versus prednisone alone.

SWOG 99-16 was a randomized phase 3 trial of docetaxel and estramustine versus mitoxantrone and prednisone in men with androgen-independent prostate cancer (Petrylak, 2004). The median survival of men treated on the docetaxel/estramustine arm was 18 months and on the mitoxantrone/prednisone arm was 15 months. This difference was statistically significant (log rank p = 0.008). The docetaxel/estramustine arm also demonstrated a superior median time to progression (6 months) compared to the mitoxantrone/prednisone arm (3 months), which was also statistically significant (log rank p < 0.0001).

TAX327 was an international, multicenter phase 3 trial comparing docetaxel and prednisone, given either on an every 3 week schedule or a weekly schedule (5 of 6 weeks), to mitoxantrone and prednisone in patients with HRPC (Eisenberger, 2004; Dagher, 2004).The median survival in the every 3 weeks docetaxel arm was 18.9 months versus 16.5 months in the mitoxantrone arm. This difference was statistically significant (p = 0.009). The median survival in weekly docetaxel schedule was 17.4 months. This was not statistically different compared to the mitoxantrone arm. However, when comparing the median survival of both docetaxel arms together (18.3 months) to the mitoxantrone arm, this difference was statistically significant (p = 0.04).

In the TAX327 trial, the use of prednisolone, the active metabolite of prednisone, was allowed as a replacement of prednisone in case that oral tablets of prednisone were not marketed in the country where the trial took place (see Approval Package for Application Number 20-449/S-028: Medical Review(s) dated May 18, 2004). Correspondingly in Europe, the Committee for Medicinal Products for Human Use (CHMP) adopted a positive opinion to recommend the variation to the terms of the marketing authorisation for Taxotere (docetaxel) to add that Taxotere in combination with prednisone or prednisolone is indicated for the treatment of patients with hormone refractory metastatic prostate cancer.

Thus, chemotherapy is now an established treatment for HRPC, but the duration and response to first-line chemotherapy is limited, and a substantial number of patients will fail first-line therapy after an initial improvement of symptoms and modestly improved survival. There is a medical need for chemotherapeutic agents that may provide continued palliation and improved survival. Randomized trials must continue in order to identify new agents for the treatment of HRPC.

### The challenges for any future HRPC treatment

Due to the favorable results of the trials that led to approval of docetaxel (Taxotere®) for treatment of HRPC as first-line chemotherapy, it is anticipated that the number of patients treated with chemotherapy in the first-line setting of this disease will increase substantially. Once HRPC fails such first-line chemotherapy, subsequent treatment is needed for these patients. Currently there is no therapy approved for those patients for who the HRPC disease progresses despite such first-line chemotherapy; patients who have a median survival of only around 18 months. Considering the extent of the unmet medical need in HRPC, and following the approval of docetaxel for first-line chemotherapy the increasing number of HRPC patients in need of such second-line chemotherapy for HRPC, there is an urgent need for new therapies that can show significantly significant effects in clinical trials of hormone refractory prostate cancer in patients who were treated with a first-line cytotoxic chemotherapy regime.

Any therapy for second-line HRPC that shows statistically significantly efficacy in clinical trials, can lead to approval by FDA or other international drug-regulatory agencies, and the introduction and use of such therapy on a larger scale. Such use has the potential to materially improve the prospects of life-expectancy or life-quality of many men throughout the world. Any therapy that would enable such sick men to conduct the remainder of their life with as much dignity and convenience as possible, especially a therapy that could be practiced largely at home or in a more convenient setting, would have great significance and be of immense advantage to such men.

### Summary of the invention

We have invented that satraplatin in combination with prednisone is effective in the treatment of an individual suffering from metastatic hormone refractory prostate cancer, where such individual was treated with previous chemotherapy against such disease.

Thus, one aspect of the present invention relates to a method of treating an individual suffering from metastatic hormone refractory prostate cancer comprising administration of a therapeutically effective amount of satraplatin to said individual, wherein:
(a) said individual was treated with previous chemotherapy for metastatic hormone refractory prostate cancer; and
(b) said method further comprises the administration of prednisone to said individual in combination with said administration of satraplatin.

In another aspect, the invention relates to a packaged-pharmaceutical-product comprising a pharmaceutical composition that includes satraplatin, wherein said packaged-pharmaceutical-product further comprises instructions to conduct administration of a therapeutically effective amount of said satraplatin included in said pharmaceutical composition to an individual suffering from metastatic hormone refractory prostate cancer, wherein said instructions further include:
(a) an instruction to conduct said administration of satraplatin to an individual who was treated with previous chemotherapy for metastatic hormone refractory prostate cancer; and
(b) an instruction to conduct said administration of satraplatin in combination with administration of prednisone;

Such aspects include certain embodiments, wherein the packaged-pharmaceutical-product further comprises a second pharmaceutical composition that includes prednisone.

Another aspect of the present invention relates to a use of satraplatin for the preparation of a pharmaceutical composition including satraplatin for administration of a therapeutically effective amount of satraplatin to an individual suffering from metastatic hormone refractory prostate cancer, wherein:
(a) said individual was treated with previous chemotherapy for metastatic hormone refractory prostate cancer; and
(b) said individual is administered prednisone in combination with said administration of satraplatin.

Other features and advantages of the invention will be apparent from the following detailed description and from the claims.

### Brief description of the drawings

### Figure 1.

Exemplary metabolites of satraplatin (JM216), depicting JM118, JM383, JM518, JM559 and JM149 (taken from Raynaud et al. 1996 Cancer Chemother. Phamacol. 38: 155-162).

### Figure 2.

Study schema of the "SPARC" trial (Satraplatin and Prednisone Against Refractory Cancer)

### Figure 3.

Representation of data to demonstrate significant efficacy of satraplatin, in combination with prednisone, against metastatic hormone resistant prostate cancer in patients having previous chemotherapy treatment. Kaplan Meier plot of Progression-Free Survival (as adjudicated by the IRC) for the ITT Population - SPARC Study: satraplatin (plus prednisone) arm, compared to placebo (plus prednisone) arm.

### Figure 4.

Demographic and Disease Characteristics - SPARC Study.

### Figure 5.

Kaplan Meier plot of Progression-Free Survival (as adjudicated by the IRC) for the subset of ITT Population who had received prior docetaxel - SPARC Study: satraplatin (plus prednisone) arm, compared to placebo (plus prednisone) arm.

### Figure 6.

Hazard ratios for PFS (and 95% confidence intervals) in various prognostic subsets - SPARC Study: satraplatin (plus prednisone) arm, compared to placebo (plus prednisone) arm. In the plot shown, estimated hazard ratio is depicted by a circle and the 95% confidence interval for the hazard ratio by the horizontal line.

### Figure 7.

(a) Kaplan Meier plot of Progression-Free Survival (as adjudicated by the IRC) for the subset of ITT Population who had disease-related pain at baseline (PPI score 1-5) - SPARC Study: satraplatin (plus prednisone) arm, compared to placebo (plus prednisone) arm; (b) Kaplan Meier plot of Progression-Free Survival (as adjudicated by the IRC) for the subset of ITT Population who were asymptomatic at baseline (PPI score 0) - SPARC Study: satraplatin (plus prednisone) arm, compared to placebo (plus prednisone) arm.

### Figure 8.

(a) Grade 3/4 haematological toxicity; (b) Grade 3/4 non-haematological toxicity.

### Figure 9.

Number of treatment cycles for patients in the SPARC Trial.

### Figure 10.

Time to Pain Progression Analysis for the Intent-to-Treat Population.

### Exhibit A.

Public disclosure of results from SPARC trial.

### Exhibit B.

Public disclosure II of results from SPARC trial.

### Detailed description of the invention

### Definitions

The terms "administered", "administration", or "administering" a compound is understood by skilled artisans, such as clinical oncologists, and refers to providing a compound, such as a therapeutic agent including but not limited to satraplatin, prednisone or granisetron, to an individual in need of treatment by bringing such individual in contact with, or otherwise exposing such individual to, such compound. Compounds may be administered as a pharmaceutical composition or formulation.

The term "antiemetic agent" is understood by skilled artisans, such as clinical oncologists, and refers to any anti-emetic agent known to the skill artisan, including, but not limited to, serotonin-3 receptor antagonists like granisetron, dolasetron, ondansetron and tropisetron, NK1 receptor antagonists, antihistamines such as cinnarizine, cyclizine and promethazine, histamine H2 receptor antagonists such as ranitidine (Zantac), phenothiazines such as chlorpromazine, droperidol, haloperidol, methotrimeprazine, perphenazine, trifluoperazine and prochlorperazine, domperidone, and metoclopramide.

The term "chemotherapy" is understood by skilled artisans, such as clinical oncologists, and refers to the treatment of cancer with chemical compounds that have a specific toxic effect upon the cancer, e.g. by interfering with cell reproduction. By way of non-limiting example, compounds useful for chemotherapy of metastatic prostate cancer include taxanes such as paclitaxel and docetaxel, mitoxantrone, viniblastine and estramustine.

The term "in combination" when used in reference to administration is understood by skilled artisans, such as clinical oncologists, and refers to the essentially simultaneous or sequential administration of at least two compounds, including but not limited to the two compounds satraplatin and prednisone. Such compounds may be administered sequentially with each other, with the term "in combination" not being limited in the sequence of administration; encompassing when a compound is administered either prior to or after administration of another compound. By way of non-limiting example, satraplatin and prednisone are considered to be administered "in combination" during the treatment regime using such compounds that is set out with in the exemplification. A compound may also be administered "in combination" with another compound when both are administered essentially at the same time or simultaneously, including when appropriate when both compounds are formulated as single dosage form.

The term "corticosteroid" is understood by skilled artisans, such as clinical oncologists, and refers to a family of semisynthetic and synthetic compounds that mimic the anti-inflammatory effects of cortisol. The most commonly prescribed agents include cortisone acetate, hydrocortisone, prednisone, dexamethasone, and prednisolone.

The term "cytotoxic" is understood by skilled artisans, such as clinical oncologists, and refers to the property of e.g. a compound to be toxic to cells, including the ability to kill a cell.

The term "cytotoxic chemotherapy regime" is understood by skilled artisans, such as clinical oncologists, and refers to a treatment procedure or regime that uses, performs or requires chemotherapy that involves at least one compound that is believed to be cytotoxic, e.g. by administering a certain dosage or dosages of such compound at, or over, a defined period of time, in one or more cycles, with or without concomitant or sequential administration of additional cytotoxic compounds, or, for example, analgesic or antiemetic compounds.

The term "bone pain" is understood by skilled artisans, such as clinical oncologists, and also by patients, and refers herein to a pain commonly associated with metastatic cancer such as metastatic prostate cancer, and is felt in (or has the sensation of stemming from) bones of the patient. Bone pain can be referred to as "ostealgia" or "osteodynia" by skilled artisans. Without being bound by theory, the pain occurs due to the disruption of the balance of normal cellular activity in the bones, causing damage to the bone tissue. Normal bone is constantly being remodeled, or broken down and rebuilt. Cancer cells that have spread to the bone disrupt this balance between the activity of osteoclasts and osteoblasts, resulting in either weakened or excessively built-up bone. This damage can either stretch the periosteum or stimulate nerves within the bone, and is a major cause of such pain.

The term "lymph pain" is understood by skilled artisans, such as clinical oncologists, and also by patients, and refers herein to a pain or discomfort felt in (or has the sensation of stemming from) a lymph node. For pain associated with metastatic cancer, such pain can felt in lymph nodes that are regional or distant to the primary cancer or tumour. In the case of prostate cancer for example, regional lymph nodes can be those nodes found in the groin, while distant lymph nodes can be those in the neck or under-arm region. Lymph nodes are often one of the first organs of the body in which metastases of a primary cancer are found. Without being bound by theory, metastases that establish in or near lymph nodes can cause swelling of or pressure on such nodes. Such swelling or pressure can cause discomfort and pain.

As used herein the term "packaged-pharmaceutical-product" refers to any packaging system for storing and dispensing individual doses of medication, including such a system storing for and dispensing to the patient who ultimate consumes the medication. The packaged-pharmaceutical-product can contain sufficient daily dosage units appropriate to the treatment period or regime, or in amounts which facilitate the patient's compliance with the regimen. In certain embodiments, the packaged-pharmaceutical-product comprises one or more vessels that include the compound to be used in the treatment according to the present invention. Such vessel can be a unit dosage form such as a capsule or pill, or may be a container such as a bottle, vial or syringe. The compound may be provided in the vessel in a pharmaceutically acceptable form or may be provided, for example, as a lyophilized powder. In further embodiments, the packaged-pharmaceutical-product may further include a solvent to prepare the compound for administration. In certain embodiments, the compound may be already provided in a delivery device, such as a syringe, or a suitable delivery device may be included in the pack. The packaged-pharmaceutical-product may comprise pills, liquids, gels, tablets, dragees or the pharmaceutical preparation in any other suitable form. The packaged-pharmaceutical-product may contain any number of daily pharmaceutical dosage units, or a number of dosage units sufficient for multiple days of a treatment regime. The package may be of any shape, and the unit dosage forms may be arranged in any pattern, such as circular, triangular, trapezoid, hexagonal or other patterns. One or more of the doses or subunits may be indicated, for example to aid the doctor, pharmacist or patient, by identifying such dose or subunits, such as by employing color-coding, labels, printing, embossing, scorings or patterns. The packaged-pharmaceutical-product may also comprise instructions for the patient, the doctor, the pharmacist or any other related person.

Some embodiments comprise the administration of more than one active ingredient, including compounds as disclosed herein. Such administration may occur concurrently or sequentially. The active ingredients may be formulated together such that one administration delivers both components. Alternatively the active ingredients may be formulated separately. In certain such embodiments, the packaged-pharmaceutical-product may comprise: (i) a compound used in the present invention and any the other pharmaceutical ingredient in a single formulation (i.e., they are formulated together), or (ii) such compound used in the present invention and the other pharmaceutical ingredient in individual formulations (i.e., they are formulated separately). Each formulation may comprise a compound used in the present invention and any other pharmaceutical ingredient in individual dosage amounts (in approximately equal or unequal amounts).

As used herein, the term "instructions" means a product label and/or documents describing relevant materials, methodologies or information pertaining to assembly, preparation or use of a packaged-pharmaceutical-product or any component contained therein. For example, such instructions may include details of the indications and usage of such component, therapeutic procedure or regime to be followed, with appropriate doses and mode of administrations that provide therapeutically effective amounts of any compounds used in such therapeutic regime, dosage modifications, warnings and precautions and other information pertinent for the safe and effective application of the packaged-pharmaceutical-product in the area of health-care. These materials, methodologies or information may include any combination of the following: background information, steps or procedures to follow, list of components, proposed dosages for therapeutically effective amounts, warnings regarding possible side effects, instructions for administering the drug, technical support, and any other related documents. Instructions can be supplied in printed form, such as a package label or a package insert. Non-limiting example of "instructions" in the form of a package insert, can be obtained from the Center for Drug Evaluation and Research of the U.S. FDA, including via http://www.accessdata.fda.gov/scripts/cder/drugsatfda/index.cfm. Such form of instructions can be required to be approved before use by a drug regulatory authority, such as the FDA, and only after appropriate clinical trials have been conducted that show significantly significant effects following treatment with the drug. Alternatively, instruction may also be stored in electronic form, e.g., on a computer-readable storage medium such as a computer-readable memory device, a centralized database, magnetic media such as hard disks, floppy disks, and magnetic tape; optical media such as compact discs, CD-ROMs and holographic devices; magneto-optical media such as floptical disks; and hardware devices that are specially configured to store and execute program code, such as application-specific integrated circuits (ASICs), programmable logic devices (PLDs) and ROM (read only memory) and RAM (random access memory) devices. Instructions may comprise a web address of an internet website from which more detailed instructions may be downloaded, or a recorded presentation. Instructions can contain one or multiple documents or future updates.

The term "taxane" is understood by skilled artisans, such as clinical oncologists, and is meant to include any member of the family of terpenes, including, but not limited to paclitaxel (Taxol) and docetaxel (Taxotere), which were derived primarily from the Pacific yew tree, *Taxus brevifolia,* and which have activity against certain tumors, particularly breast, lung and ovarian tumors (See, for example, Pazdur et al. Cancer Treat Res. 1993.19:351; Bissery et al. Cancer Res. 1991 51:4845). In particular embodiments of the methods, uses and packaged-pharmaceutical-products of the present invention, taxanes are paclitaxel, docetaxel, deoxygenated paclitaxel, TL-139 and their active derivatives. See Annu. Rev. Med. 48:353-374 (1997).

The term "taxane" as used herein includes naturally occurring or partly or fully chemically synthesized paclitaxel, which is sold as TAXOL® by Bristol-Myers Oncology, as well as terpene compounds derived from, or related to, paclitaxel, or other derivatives thereof, including deoxygenated paclitaxel compounds, such as those described in U.S. Pat. Nos. 5,440,056 and 4,942,184, which are herein incorporated by reference. Paclitaxel has been approved for clinical use in the treatment of refractory ovarian cancer in the United States (Markman et al., Yale Journal of Biology and Medicine, 64:583, 1991; McGuire et al., Ann. Intern. Med., 111:273, 1989). It is effective for chemotherapy for several types of neoplasms including breast (Holmes et al., J. Nat. Cancer Inst., 83:1797, 1991) and has been approved for treatment of breast cancer as well. It is a potential candidate for treatment of neoplasms in the skin (Einzig et al., Proc. Am. Soc. Clin. Oncol., 20:46, 2001) and head and neck carcinomas (Forastire et al. Sem. Oncol., 20:56, 1990). The compound also shows potential for the treatment of polycystic kidney disease (Woo et al, Nature, 368:750, 1994), lung cancer and malaria. Docetaxel (N-debenzoyl-N-tert-butoxycarbonyl-10-deacetyl paclitaxel) is produced under the trademark TAXOTERE® by Sanofi-Aventis. In addition, other taxanes are described in "Synthesis and Anticancer Activity of Taxol other Derivatives," D. G. 1. Kingston et al., Studies in Organic Chemistry, vol. 26, entitled "New Trends in Natural Products Chemistry" (1986), Atta-ur-Rahman, P. W. le Quesne, Eds. (Elvesier, Amsterdam 1986), pp 219-235 are incorporated herein. Various taxanes are also described in U.S. Patent No. 6,380,405, the entirety of which is incorporated herein.

Without being bound by theory, taxanes exert their cytotoxic effect on cells, including cancer and tumour cells, by binding to tubulin, thereby causing the formation of unusually stable microtubules. The ensuing mitotic arrest triggers the mitotic spindle checkpoint and results in apoptosis. Other mechanisms that mediate apoptosis through pathways independent of microtubule dysfunction have been described as well, including molecular events triggered by the activation of Cell Division Control-2 (cdc-2) Kinase, phosphorylation of BCL-2 and the induction of interleukin 1β (IL-1β) and tumour necrosis factor-α (TNF-α). Furthermore, taxanes have been shown to also exert anti-tumour activity via mechanisms other than the direct activation of the apoptotic cascade. These mechanisms include decreased production of metalloproteinases and the inhibition of endothelial cell proliferation and motility, with consequent inhibition of angiogenesis.

The term "therapeutically effective amount" of a compound, including an active ingredient, therapeutic agent or drug, is understood by skilled artisans, such as clinical oncologists, and refers to an amount of a compound to be administered to an individual in need of therapy or treatment, as required by any particular dosage, therapeutic or administration regimen or procedure, and as according to clinically acceptable standards for the disease, disorder, symptom or condition to be treated, or at a reasonable benefit/risk ratio applicable to such treatment. In the case of metastatic hormone resistant prostate cancer, such amount is reasonably in accordance with the amount of such compound that has been demonstrated to have the desired therapeutic effect with statistic significance in a clinical trial, such as in the SPARC phase III clinical trial shown herein in the Exemplification.

It is well known to anyone of ordinary skill that for a given individual, the therapeutically effective amount, dosage form and timing and form of administration of such therapeutically effective amount, will be determined by a qualified physician, or other person having appropriate knowledge and qualification, based on one or more of: (i) the dosage, dosage form and timing and form of administration used in the clinical study that has demonstrated the statistically significant clinical efficacy for the respective treatment, (ii) recommendations for the dosage, dosage form and timing and form of administration provided in any instructions provided with the pharmaceutical form of the compound, including the approved product label or insert for such treatment, and (iii) factors specific for such individual that may influence the actual dose or amount to be administered to the individual. Thus, the dosage administered will, of course, vary depending upon known factors such as the pharmacodynamic characteristics of the particular compound and its mode and route of administration; age, sex, health, weight, body surface area, neutrophil count, of the individual to be treated; nature and extent of symptoms; kind of concurrent treatment, frequency of treatment and the effect desired. Furthermore, scientific or medical publications or reports on additional clinical studies, especially those related to efficiency or safety of the compound when used in other setting, may influence the determination of a dosage, dosage form, or and timing and form of administration in order to determine an amount reasonably expected to be a therapeutically effective amount for any given individual.

The term "time to disease progression", is understood by skilled artisans, such as clinical oncologists, and refers to the time from initiation of a particular therapy or treatment regime or protocol for an individual, such as administration of satraplatin to patients suffering from metastatic hormone resistant prostate cancer, to when disease progression is then first observed in such individual, as determined from one or more symptoms or characteristics of the individual. Time to disease progression can be abbreviated to "TTP". By way of example, "time to disease progression" in the SPARC trial was used to refer to the time period described in section 10.3.1 of the clinical protocol.

The term "progression-free survival" is also understood by skilled artisans, such as clinical oncologists, and refers to the time from initiation of a particular therapy or treatment regime or protocol for an individual, such as administration of satraplatin to patients suffering from metastatic hormone resistant prostate cancer, to the earlier of: (i) when disease progression is then first observed in such individual, as determined from one or more symptoms or characteristics of the individual; or (ii) death of the individual. Progression-free survival can be abbreviated to "PFS". By way of example, "progression-free survival" in the SPARC trial was used to refer to the time period described in section 10.3.2 of the clinical protocol. The term "time to pain progression" is also understood by skilled artisans, such as clinical oncologists, and refers to the time from initiation of a particular therapy or treatment regime or protocol for an individual, such as administration of satraplatin to patients suffering from metastatic hormone resistant prostate cancer, to when pain-related progression is then first observed in such individual. Time to pain progression can be abbreviated to "TPP". By way of example, "time to pain progression" in the SPARC trial was used to refer to the time period described in section 10.7.3 of the clinical protocol.

The term "overall survival" is also understood by skilled artisans, such as clinical oncologists, and refers to the time from initiation of a particular therapy or treatment regime or protocol for an individual, such as administration of satraplatin to patients suffering from metastatic hormone resistant prostate cancer, to death of such individual.

The term "chemotherapy holiday" is also understood by skilled artisans, such as clinical oncologists, and refers to the use of intermittent chemotherapy - whereby during the chemotherapy (such as chemotherapy with docetaxel) breaks or "holidays" in the chemotherapy are given (for example, Br J Cancer 2003; 89:968-970). Although chemotherapy drugs can be effective, side effects can accumulate when such drugs are used for prolonged periods of time, and it is unrealistic to continue the treatment indefinitely. Indeed, patients are often unable to tolerate continuous ongoing chemotherapy, such as therapy with docetaxel, and chemotherapy can be administered intermittently: patients take a break (a "chemotherapy holiday") from treatment and resume at a specified point in the future.

### Particular embodiments

In certain embodiments, prednisone is administered in a therapeutically effective amount.

In one embodiment, the individual to be treated in accordance with the present invention has a diagnosis of Stage D2 adenocarcinoma of the prostate that is unresponsive to hormone therapy.

In another embodiment, the individual has failed treatment with previous chemotherapy.

In yet another embodiment, the individual has taken a chemotherapy holiday from said previous chemotherapy.

In certain embodiments, the chemotherapy was a cytotoxic chemotherapy regime.

In certain embodiments, the individual has suffered disease progression or PSA progression after a minimum of two courses of one prior cytotoxic chemotherapy regime for metastatic hormone refractory prostate cancer.

In another embodiment, the chemotherapy or cytotoxic chemotherapy regime used a compound selected from mitoxantrone, viniblastine, estramustine and a taxane, including embodiments where the compound is a taxane, including paclitaxel and docetaxel.

In a certain embodiment, the taxane is docetaxel.

In a certain embodiment, the previous chemotherapy did not use a platinum-containing compound, including satraplatin. In a related embodiment, the individual has not had prior treatment with a platinum-containing compound, including satraplatin.

In an alternative embodiment, the previous chemotherapy did not use mitoxantrone in combination with a corticosteroid.

In yet another embodiment, the individual is administered satraplatin orally at a dose of between about 30 mg/m² and about 140 mg/m² per day over between 3 and 7 days, including administration at a dose of between about 40 mg/m2 and about 100 mg/m², or at a dose of between about 50 mg/m² and about 90 mg/m², in each case per day, over between 3 and 7 days.

In another embodiment, the individual is administered satraplatin orally at a dose of about 40 mg/m² per day, at a dose of about 60 mg/m², or at a dose of about 80 mg/m², in each case over between 3 and 7 days.

In a certain embodiment, the actual amount or dose of satraplatin administered orally to the individual is rounded to the nearest 10 mg.

In a certain embodiment, the individual is administered satraplatin daily for about five consecutive days, with the cycle repeated about every 35 days. In an alternative certain embodiment, the individual is not administered satraplatin with such five consecutive days for no more than two days, and satraplatin is administered for a further number of days equal to the such number of days the individual is not administered satraplatin. In another certain embodiment, the cycle is repeated after about 38 days.

In another embodiment, the individual is examined after an appropriate period of time following the administration of satraplatin. Such examination can include the examination or assessment of one or more of: History and Physical (H&P), Weight and Performance Status ("PS"), Toxicity Assessment, PSA, Bone scan, Tumor Assessment, Complete Blood Count (CBC), platelets, absolute neutrophil count ("ANC"), Serum Chemistry, Chest X-ray, Electrocardiogram, Present Pain Intensity ("PPI") Diary or Analgesic Diary. Such examinations or assessments can be conducted using methodologies that are known to skilled artisans, such as clinical oncologists, for example, as described in the Exemplification.

In a certain embodiment, the individual is examined or assessed for at least one of neutropenia, thrombocytopenia or non-hemotologic toxicity.

In one embodiment, the individual is retreated with satraplatin if the absolute neutrophil count is greater than or equal to about 1.5 x 10⁹/L, and platelets are more than or equal to about 100 x 10⁹/L. In another embodiment, the individual is retreated if no non-hematological toxicity that is ascribed to the therapy resolves to base line of greater than or equal to grade 1, for example as graded according to the NCI Common Toxicity Criteria Version 2.0. In certain such embodiments, the individual is retreated with a dose of satraplatin at about 100 mg/m² per day.

In an alternative embodiment, the individual is retreated with a decreased dose of satraplatin if the absolute neutrophil count is less than about 1.5 x 10⁹/L, platelets are less than about 100 x 10⁹/L, or the individual shows non-hematological toxicity that is ascribed to the therapy. In certain such embodiments, the individual is retreated with a reduced dose of satraplatin at about administered a dose of satraplatin at about 60 mg/m² or 40 mg/m² per day.

In a particular embodiment of the invention, the individual is not retreated with satraplatin if upon examination or assessment if one or more of the following observations are made in the individual: (i) neutropenia (neutrophil count is less than about 0.5 x 10⁹/L) or thrombocytopenia (platelets less than about 25 x 10⁹/L) despite dose reduction to 40 mg/m² per day; (ii) grade 3 or 4 hepatic (lasting >7 days), renal, cardiac, pulmonary, or neurological toxicity; or (iii) grade 4 vomiting or diarrhea that cannot be controlled by medical treatment and that occurs after one dose reduction.

In another embodiment, no food is taken by the individual for at least about one hour before, and for at least about 2 hours after administration of satraplatin.

In yet another embodiment, administration of satraplatin is to the individual on an empty stomach.

In other embodiment, the individual is administered prednisone orally with an amount of between 2 mg and 10 mg twice per day, including with an amount of 5 mg twice per day.

In certain embodiments, the individual is administered prednisone orally about one hour prior to administration of satraplatin orally and about eight hours after administration of satraplatin orally.

In certain embodiments, the individual is administered prednisone in the morning and the evening on those days of a cycle when satraplatin is not administered. In particular such embodiments, the individual is administered prednisone in the morning and the evening without administration of satraplatin for about 30 consecutive days.

In certain embodiment of all aspects of the invention, the individual is administered a number of cycles of treatment, wherein such number is greater than 3, 4 or 5 cycles. In particular such embodiments, such number is greater than 7, 9 or 11 cycles. In other particular embodiments, such number is greater than 16, 18 or 20 cycles. In yet other particular embodiments, such number is greater than 5, 9 or 16, but less than 90, 60 or 30 cycles, including where such number of cycles is between 5 and about 35 cycles, or between 17 and about 28 cycles. In particular such embodiments, the individual has one or more cycle delayed by one week or more, including 1, 2 or 3 such cycles delayed by about 1 week. In other particular such embodiments, the individual has two or more cycle delayed by one week or more, including by about 1 week., including 2, 3 or 4 cycles being so delayed.

In yet another embodiment, the individual is further administered an antiemetic agent on the same day of administration of satraplatin, including embodiments wherein the antiemetic agent is administered about one hour prior to administration of satraplatin orally and about eight hours after administration of satraplatin orally.

In certain embodiments, the antiemetic agent is administered in a therapeutically effective amount.

In a related embodiment, the individual is premedicated with an antiemetic agent.

In certain embodiments, the antiemetic agent is a 5-HT3 blocker or inhibitor, including ondansetron, tropisetron, or dolasetron, and further including embodiments wherein the antiemetic agent is granisetron. In certain of these embodiments, granisetron is administered orally with an amount of between 0.2 mg and 5 mg, including embodiments where granisetron is administered with an amount of 1 mg.

In another embodiment, the method of the present invention comprises the steps of: (a) to said individual, on each of days 1 to 5, the administration of prednisone (5 mg) and antiemetic agent (1 mg) orally, followed after about 1 hour by the administration of satraplatin orally at a dose of about 80 mg/m², followed after about 8 hours by the administration of prednisone (5 mg) and antiemetic agent (1 mg) orally; (b) to said individual, on each of days 6 to 35 the administration of prednisone (5 mg) twice daily in the morning and evening; and (c) repeating (a) and (b) at least one time.

In a certain embodiment, the instructions included in the packaged-pharmaceutical-product of the present invention comprises instructions to conduct the steps of: (a) to said individual, on each of days 1 to 5, the administration of prednisone (5 mg) and antiemetic agent (1 mg) orally, followed after about 1 hour by the administration of satraplatin orally at a dose of about 80 mg/m², followed after about 8 hours by the administration of prednisone (5 mg) and antiemetic agent (1 mg) orally; (b) to said individual, on each of days 6 to 35 the administration of prednisone (5 mg) twice daily in the morning and evening; and (c) repeating (a) and (b) at least one time.

An embodiment of the use of the present invention is further characterised as: (a) to said individual, on each of days 1 to 5, prednisone (5 mg) and antiemetic agent (1 mg) is administered orally, followed after about 1 hour by the administration of satraplatin orally at a dose of about 80 mg/m², followed after about 8 hours by the administration of prednisone (5 mg) and antiemetic agent (1 mg) orally; (b) to said individual, on each of days 6 to 35 prednisone (5 mg) is administered twice daily in the morning and evening; and (c) repeating (a) and (b) at least one time.

In certain such embodiments, the individual is examined or assessed for at least one of neutropenia, thrombocytopenia or non-hemotologic toxicity after (b) and before (c). In a particular such embodiment, (c) is conducted if the absolute neutrophil count is greater than or equal to about 1.5 x 10⁹/L, and platelets are more than or equal to about 100 x 10⁹/L.

In other such embodiments, the satraplatin is administered to the individual on an empty stomach. In a related such embodiment, the individual had not received food for one hour before or two hours after the administration of satraplatin.

In certain embodiments of all aspects of the invention, the administration of satraplatin results in an extension, elongation or prolongation of the time to disease progression.

In certain embodiments of all aspects of the invention, the administration of satraplatin results in an extension, elongation or prolongation of the progression-free survival.

In a particular embodiment, the administration of satraplatin results in a extension, elongation or prolongation of the progression-free survival of between about 5 weeks to about 50 weeks In another particular embodiment, such extension, elongation or prolongation of progression-free survival is between about 8 weeks and about 25 weeks, including a extension, elongation or prolongation of the progression-free survival of between about 10 weeks to about 20 weeks.

In a certain embodiment, the administration of satraplatin results in a progression-free survival of between about 10 weeks to about 50 weeks. In a particular such embodiment, such progression-free survival is between about 15 weeks and about 40 weeks, including a progression-free survival of between about 20 weeks to about 35 weeks.

In another certain embodiment, the administration of satraplatin results in between about 10% to about 80% lower risk of the progression-free survival. In a particular such embodiment, such lower risk of progression-free survival is between about 15% to about 50%, while in another particular such embodiment, such lower risk of progression-free survival is between about 20% to about 35%, including a lower risk of about 30% to about 35% of the progression-free survival.

In certain embodiments of all aspects of the invention, the administration of satraplatin results in an extension, elongation or prolongation of the overall survival.

In certain embodiments of all aspects of the invention, the individual suffering from metastatic hormone refractory prostate cancer is suffering from pain.

In certain such embodiments, the pain is cancer-related pain.

In other such embodiments, the pain is pain associated with metastatic hormone refractory prostate cancer.

In certain such embodiments, the pain is caused by metastases.

In certain such embodiments, the pain is bone pain or lymph pain.

In certain such embodiments, the administration of satraplatin results in relief or alleviation of the pain, in stable, or in stabilization of, pain, or in an extension, elongation or prolongation of the time to pain progression.

In a particular embodiment, the administration of satraplatin results in a extension, elongation or prolongation of the time to pain progression of between about 5 weeks to about 50 weeks In another particular embodiment, such extension, elongation or prolongation of time to pain progression is between about 10 weeks and about 30 weeks, including a extension, elongation or prolongation of the time to pain progression of between about 15 weeks to about 20 weeks.

In certain embodiment, the administration of satraplatin results in a time to pain progression of between about 20 weeks to about 100 weeks In a particular such embodiment, such time to pain progression is between about 30 weeks and about 80 weeks, including a time to pain progression of between about 40 weeks to about 60 weeks.

In another certain embodiment, the administration of satraplatin results in a lower risk of pain progression of between about 15% to about 50%. In a particular such embodiment, the administration of satraplatin results in a lower risk of pain progression of between about 20% to about 40%, including a lower risk of pain progression of between about 30% to about 35%.

In yet another certain embodiment, the administration of satraplatin results in relief of pain, including embodiments where such relief lasts for between about 15 weeks to about 80 weeks, between about 25 weeks to about 60 weeks or between about 30 weeks to about 56 weeks.

In other such embodiments, the individual does not show an increase in PPI score or analgesic consumption. In a particular such embodiment, the individual does not experience an increase cancer related pain, of at least one point from baseline or at least 2 points compared with the nadir, observed for at least 2 weeks (based on 2 or more consecutive weekly PPI determinations), or the individual does not show an increase in average analgesic score of greater than 25% compared with base line that is maintained for more than 2 consecutive weeks. In particular such embodiments of the invention, the individual shows a decrease in PPI score or analgesic consumption.

In another embodiment of all aspects of the invention, the individual does not show: (i) a decrease in ECOG performance status of greater than 2 units compared to baseline attributable to cancer for longer than about two weeks; and (ii) weight loss of greater than 10% of initial body weight attributable to cancer. In particular such embodiments, the individual shows an increase in ECOG performance status or a weight gain.

In certain embodiments of all aspects of the invention, the individual: (i) suffers from Stage D2 adenocarcinoma of the prostate that is unresponsive to hormone therapy; (ii) has shown progression of such disease after 1 prior cytotoxic chemotherapy regimen (prior prednisone therapy permitted); (ii) is classified as Eastern Cooperative Oncology Group (ECOG) performance status ≤2; (iii) has no history of major gastrointestinal surgery or conditions that may impair absorption; (iv) shows no symptoms of active gastric or duodenal ulcer; and/or (v) does not suffer from uncontrolled insulin-dependent diabetes.

In other certain embodiments of all aspects of the invention, the individual is an asymptomatic patient, including patients that are asymptomatic for pain (for example with a PPI score of 0).

In yet other certain embodiments of all aspects of the invention, the individual has not shown progression of HRPC as determined by pain progression, while in another alternative embodiment of all aspects of the invention, the individual has not shown progression of such disease as determined by PSA level, increase in PSA or rate of ("velocity") of PSA increase. In alterative embodiments of all aspects of the invention, the individual has HRPC that has progressed as determined by pain progression, while in another alternative embodiment of all aspects of the invention, the individual has shown progression of such disease as determined by PSA level, increase in PSA or rate of ("velocity") of PSA increase.

In yet other embodiments of all aspects of the invention, the individual is older than 50 years, is between about 50 and about 95 years or is between about 60 and about 90 years, including individuals older than 65 years and younger than about 85 years.

In yet another embodiment of all aspects of the invention, the individual is administered satraplatin together with another therapy, such as chemotherapy, including embodiments where the other therapy and the satraplatin is administered is within about 35 days, 28 days, 14 days, 7 days or 2 days of each other. In particular embodiments, the other therapy and the satraplatin is administered on the same day, or effectively at the same place. In certain embodiments, the other therapy uses active ingredients to relieve pain, including bisphosphonates or opioid analgesics, or to control or ameliorate diarrhea. In certain other embodiments, the other therapy is chemotherapy that does not use a compound that is a taxane, such as paclitaxel or docetaxel, mitoxantrone, viniblastine or estramustine. In other certain embodiments, the chemotherapy is radiation therapy or uses a radionuclide. In yet another embodiment, the chemotherapy uses a compound selected from: altretamine, busulfan, chlorambucil, cyclophosphamide, ifosfamide, mechlorethamine, melphalan, thiotepa, cladribine, fluorouracil, floxuridine, capecitabine, gemcitabine, thioguanine, pentostatin, methotrexate, 6-mercaptopurine, cytarabine, carmustine, lomustine, streptozotocin, carboplatin, cisplatin, oxaliplatin, picoplatin, LA-12, iproplatin, tetraplatin, lobaplatin, fludarabine, aminoglutethimide, flutamide, goserelin, leuprolide, megestrol acetate, cyproterone acetate, tamoxifen, anastrozole, bicalutamide, dexamethasone, diethylstilbestrol, prednisone, bleomycin, dactinomycin, daunorubicin, doxirubicin, erlotinib, idarubicin, mitoxantrone, losoxantrone, mitomycin-c, plicamycin, paclitaxel, docetaxel, topotecan, irinotecan, 9-amino camptothecan, 9-nitro camptothecan, GS-211, etoposide, teniposide, vinblastine, vincristine, vinorelbine, procarbazine, asparaginase, pegaspargase, octreotide, estramustine, and hydroxyurea, and in yet another embodiment, the chemotherapy uses a compound that is a non-small molecule therapeutic, including but not limited to antibodies, e.g., 1D09C3 and other anti-HLA-DR antibodies as described in WO 01/87337 and WO 01/97338, Rituxan as described in US patents 5,736,137, 5,776,456, 5,843,437, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin ®, rhuMAb), Epratuzumab, Cetuximab (Erbitux ®), Pertuzumab (Omnitarg®, 2C4), R3, CDP860, Bevacizumab (Avastin ®), tositumomab (Bexxar ®), Ibritumomab tiuxetan (Zevalin ®), M195, 1D10, Hu1D10 (Remitogen®, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties, and proteins or peptides, e.g., those described in Trends in Biotechnology (2003), 21(12), p.556-562.

In one embodiment, the other therapy is chemotherapy that uses a compound that is a taxane, such as paclitaxel or docetaxel, mitoxantrone, viniblastine or estramustine, provided that such compound has not been used in the previous chemotherapy or cytotoxic chemotherapy regime for hormone refractory prostate cancer.

In another embodiment, the other therapy is chemotherapy that uses a compound that is a taxane, such as paclitaxel or docetaxel, mitoxantrone, viniblastine or estramustine, where such compound has been used in the previous chemotherapy or cytotoxic chemotherapy regime for hormone refractory prostate cancer.

### Other aspects of the invention

In an alternative aspect of the invention, the prednisone used in any of the methods, packaged-pharmaceutical-products or uses recited above, is replaced with a corticosteroid. In certain embodiments of this aspect, the corticosteroid is selected from dexamethasone, hydrocortisone or cortisone acetate. In another embodiment of this aspect, the corticosteroid is prednisolone.

In yet another alternative aspect of the invention, the individual is not administered a corticosteroid such as prednisone.

In certain embodiments of the methods, packaged-pharmaceutical-products or uses of such aspects, the administration of a therapeutic amount of satraplatin is single-agent administration, or as single-agent chemotherapy, for treating an individual, or to an individual suffering from metastatic hormone refractory prostrate cancer.

For any of these alternate aspects of the invention, further specific and appropriate embodiments can be envisioned by a person of ordinary skill based on the disclosure herein, including from one or more of the particular embodiments of the inventions listed above, including any combination thereof. By way of non-limiting example, the dose of satraplatin to be orally administered in such alternate aspects can be between about 30 mg/m² and about 140 mg/m², and in particular embodiments a therapeutic amount of antiemetic agent may be administered on the same days as administration of satraplatin.

### Formulations, Dosages and Applications

The compositions of this invention can be formulated and administered to treat individuals in need by any means that produces contact of the active ingredient with the agent's site of action in the body of an individual. They can be administered by any conventional means available for use in conjunction with pharmaceuticals, either as individual therapeutic active ingredients or in a combination of therapeutic active ingredients. They can be administered alone, but are generally administered with a pharmaceutically acceptable diluent, excipient or carrier selected on the basis of the chosen route of administration and standard pharmaceutical practice.

Pharmaceutical compositions for use in accordance with the present invention may be formulated in conventional manner using one or more pharmaceutically acceptable diluents, excipients or carriers. The pharmaceutical compositions of the invention can be formulated for a variety of routes of administration, including systemic and topical or localized administration. Techniques and formulations generally may be found in Remington's Pharmaceutical Sciences, Meade Publishing Co., Easton, PA. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, capsules, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intrarectally, for example, as a cream or foam. In certain embodiments, the pharmaceutical preparations may be non-pyrogenic, i.e., do not substantially elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, and/or parenteral administration. The formulations may conveniently be presented in unit dosage form and may be prepared by any methods well known in the art of pharmacy. The amount of active ingredient which can be combined with a carrier material to produce a single dosage form will vary depending upon the individual being treated, as well as the particular mode of administration. The amount of active ingredient which can be combined with a carrier material to produce a single dosage form will generally be that amount of active ingredient which produces a therapeutic effect when administered as a single or small number of such dosage forms. Generally, out of one hundred percent, this amount will range from about 1 percent to about ninety-nine percent of active ingredient, preferably from about 5 percent to about 70 percent, or in particular embodiments from about 10 percent to about 30 percent.

Methods of preparing these formulations or compositions include the step of bringing into association a compound used in the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product. These formulations may be further prepared shortly before administration of the active ingredient. For example, a formulation may be shaken, diluted or dissolved, a pill divided or crushed, or a syringe filled, often in each case only a few moments before administration to the patient.

Pharmaceutical compositions for use in the invention may be formulated to be suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound used in the present invention as an active ingredient. A compound used in the present invention may also be administered as a bolus, electuary or paste.

In formulating the pharmaceutical compositions for use in the invention in solid dosage forms for oral administration (capsules, tablets, pills, dragees, powders, granules and the like), a compound of the invention as active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, high molecular weight polyethylene glycols, and the like.

Gelatin capsules can contain a compound used in the present invention an as active ingredient, together with powdered carriers, such as lactose, starch, cellulose derivatives, magnesium stearate, stearic acid, and the like. Similar carriers can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or crosslinked sodium carboxymethyl cellulose), surface-active or dispersing agent. Molded tablets may be made by molding in a suitable machine a mixture of the powdered inhibitor moistened with an inert liquid diluent.

The tablets and other solid dosage forms of the pharmaceutical compositions used in the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the pharmaceutical compositions of the invention include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the pharmaceutical compositions for oral administration can also include adjuvants such as wetting agents, emulsifying and suspending agents, sweetening, flavoring, coloring, perfuming, and preservative agents.

Suspensions, in addition to the pharmaceutical composition of the present invention, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For buccal administration the pharmaceutical compositions may take the form of tablets or lozenges formulated in a conventional manner.

For administration by inhalation, the pharmaceutical compositions used in the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The pharmaceutical compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions of this invention suitable for parenteral administration comprise one or more compounds used in the invention in combination with one or more pharmaceutically acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions used in the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the pharmaceutical compositions. In addition, prolonged absorption of the injectable pharmaceutical form may be brought about by the inclusion of agents that delay absorption such as aluminum monostearate and/or gelatin.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the pharmaceutical compositions used in the invention are formulated into ointments, salves, gels, or creams as generally known in the art. A wash solution can be used locally to treat an injury or inflammation to accelerate healing.

Pharmaceutical compositions for use in the invention may be formulated for rectal administration as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum cavity and release the active inhibitor.

Dosage forms for the topical or transdermal administration of a compound used in this invention include powders, sprays, ointments, pastes, creams, lotions, gels, solutions, patches and inhalants. Such compound may be mixed under sterile conditions with a pharmaceutically acceptable carrier, and with any preservatives, buffers, or propellants which may be required.

The ointments, pastes, creams and gels may contain, in addition to a compound of the invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound used in the present invention in a polymer matrix or gel.

Ophthalmic formulations, eye ointments, powders, solutions and the like, are also contemplated as being within the scope of this invention.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton forming one example of a packaged-pharmaceutical-product.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions used in the present invention can be used to provide slow or sustained release of one or more of the active ingredients using, for example, hydroxypropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions used in the invention. Thus, single unit dosage forms suitable for oral administration, such as, but not limited to, tablets, capsules, gelcaps, caplets, powders, and the like, that are adapted for sustained release are encompassed by the present invention.

Injectable depot forms are made by forming microencapsulated matrices of a compound or drug used in the invention in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

The formulations will contain an appropriate amount of the active ingredient or compounds used in the invention. Such amount will depend on a number of factors, including the mode of administration, therapeutic regime or procedure. An appropriate number of amount of the formulation will be administered to the patient, to provide a final dose or amount of active ingredient or compound. Exemplary doses include milligram or microgram amounts of the compounds of the present invention per kilogram of individual or patient weight, e.g., about 1 microgram per kilogram body-weight to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface area (BSA) basis. Such dose rates can be used to calculate the amount of a compound to be used in chemotherapy, such as that set out in the clinical trial described in the exemplification. For example, a person of 70 kg has an approximate body surface area of 1.8 square meter, and doses can be expressed as milligram or microgram amounts of the compound per body surface area of subject or sample, e.g. about 50 micrograms per square meter to about 15 grams per square meter, about 5 milligrams per square meter to about 1.5 grams per square meter, or about 50 milligrams per square meter to about 150 milligrams per square meter.

Alternatively, doses of compounds to be administered to individuals in need thereof, can be expressed as absolute amounts, such as 5 mg prednisone, 1 mg granisetron or 160 mg satraplatin.

### Exemplification

### Clinical Protocol for the SPARC Trial ("Satraplatin and Prednisone Against Refractory Cancer")

### PROTOCOL SYNOPSIS

- **Protocol Title:**: A Multi-Center, Multi-National, Double-Blind, Randomized Phase III Study of Satraplatin Plus Prednisone or Placebo Plus Prednisone in Patients with Hormone Refractory Prostate Cancer Previously Treated with One Cytotoxic Chemotherapy Regimen
- **Development Phase:**: Phase III
- **Primary Endpoints:**: Time to disease progression
Progression-free survival
- **Secondary Endpoints:**: Time to pain progression
Overall survival
- **Study Design:**: Multi-center, multi-national, double-blind, randomized, placebo-controlled study. There are two treatment groups, with a 2:1 ratio, as follows: satraplatin plus prednisone, and placebo plus prednisone. Patients will be stratified according to:
• Performance Status (ECOG 0 - 1 versus 2)
• Average baseline Present Pain Intensity (PPI) score (0-1 versus 2-5) rounded to nearest integer
• Type of progression (Tumor progression versus PSA progression) after prior first-line cytotoxic chemotherapy for metastatic disease. Patients with both tumor progression and PSA progression will be stratified as tumor progression
- **Key Inclusion Criteria:**: • Patients with a diagnosis of Stage D2 adenocarcinoma of the prostate that is unresponsive to hormone therapy
• Disease progression or PSA progression after a minimum of two courses of one prior cytotoxic chemotherapy regimen for metastatic disease (first-line treatment)
- **Key Exclusion Criteria:** Patient must not have:: • More than one prior cytotoxic chemotherapy regimen for metastatic disease
• Prior treatment with satraplatin or other platinum agents
• A history of major gastrointestinal surgery or pathology likely to influence absorption
• Active gastric or duodenal ulcer
• Uncontrolled insulin-dependent diabetes
• Concurrent cytotoxic therapy
- **Study Treatment:**: Active or placebo satraplatin 80 mg/m² administered by mouth once daily for 5 consecutive days (days 1-5), active or placebo antiemetic 1 mg administered by mouth twice daily (days 1-5), plus prednisone 5 mg twice daily every day for 35 days. These treatment cycles are repeated every 35 days. Blinding will ensure that only those patients receiving active satraplatin will receive active antiemetic therapy.
- **Patient Accrual:**: 912 study subjects will be enrolled during a 24-month period.
- **Duration of Patient Participation:**: Active or placebo satraplatin will be continued until evidence of disease progression (PSA rise in isolation is not considered evidence of disease progression*), untoward and unmanageable toxicities, withdrawal of informed consent, or noncompliance. If untoward and unmanageable active or placebo satraplatin-associated toxicities are observed - in the absence of disease progression - the patient will continue on prednisone alone. If prednisone is discontinued due to toxicity in the absence of disease progression, the patient will continue on active or placebo satraplatin alone until disease progression or withdrawal of informed consent.
After treatment cessation, patients will be monitored for toxicity for at least 30 days, and for survival every 3 months for the first year, and every 6 months thereafter until death.
****Note: Isolated increase in PSA is not currently accepted as a valid surrogate endpoint for prostate cancer disease progression in this patient population. Therefore, Investigators are encouraged to keep study subjects with such isolated PSA increase on protocol until other evidence of disease progression is confirmed.***
- **Treatment Crossover:**: Crossover to satraplatin is not allowed in patients randomized to the placebo arm.
- **Final Analysis:**: The final analysis will be performed after approximately 700 events of disease progression.

### LIST OF ABBREVIATIONS

- AEs: Adverse Events
- ALT: Alanine Aminotransferase
- ANC: Absolute Neutrophil Count
- ASCO: American Society of Clinical Oncology
- AST: Aspartate Aminotransferase
- BID: Twice Daily
- BSA: Body Surface Area
- BUN: Blood Urea Nitrogen
- CBC: Complete Blood Count
- CFR: Code of Federal Regulations
- CL: Clearance
- CLIA: Clinical Laboratory Improvements Amendments
- CNS: Central Nervous System
- CR: Complete response
- CRA: Clinical Research Associate
- CRF: Case Report Form
- CRO: Contract Research organization
- CT: Computed Tomography
- CTC: Common Toxicity Criteria
- DMB: Data Monitoring Board
- EC: Ethics Committee
- ECOG: Eastern Cooperative Oncology Group
- EMEA: European Medicines Agency
- EORTC: European Organization for Research and Treatment of Cancer
- FDA: Food and Drug Administration
- GCP: Good Clinical Practice
- GI: Gastro-intestinal
- Hgb: Hemoglobin
- HRPC: Hormone Refractory Prostate Cancer
- ICH: International Council for Harmonization
- IND: Investigational New Drug
- IRB: Institutional Review Board
- KPS: Karnofsky Performance Status
- LD: Longest diameter
- LDH: Lactate Dehydrogenase
- LHRH: Luteinizing Hormone Releasing Hormone
- LLN: Lower Limit of Normal
- MedDRA: Medical dictionary for Drug Regulatory Affairs
- mg/dL: milligram per deciliter
- MTD: Maximum Tolerated Dose
- ng/mL: nanogram/milliliter
- NCI: National Cancer Institute
- P: Placebo plus Prednisone
- PD: Progressive Disease
- PFS: Progression-free survival
- PFS + PSA: Progression-free survival or PSA progression, whichever comes first
- PP: Per Protocol
- PPI: Present Pain Intensity
- PR: Partial Response
- PS: Performance Status
- PSA: Prostate Specific Antigen
- RDF: Rapid Data Flow
- S+P: Satraplatin plus Prednisone
- SAEs: Serious Adverse Events
- SD: Stable Disease
- SOC: Systems Organ Class
- Stage D2: Prostate cancer has spread to distant organs. (any Primary Tumor (T), any Lymph Nodes (N), Distant metastases present (M1).
- TTP: Time to disease progression
- TTP + PSA: Time to disease progression or to PSA progression, whichever comes first
- ULN: Upper Limit of Normal

### 1.0 STUDY SCHEMA

This will be a multi-center, multi-national, double-blind, randomized Phase III study to evaluate the efficacy and safety of oral satraplatin plus prednisone or placebo plus prednisone in patients with hormone refractory prostate cancer. Eligible patients must have progressive disease after a minimum of two courses of one prior cytotoxic chemotherapy regimen for metastatic disease.

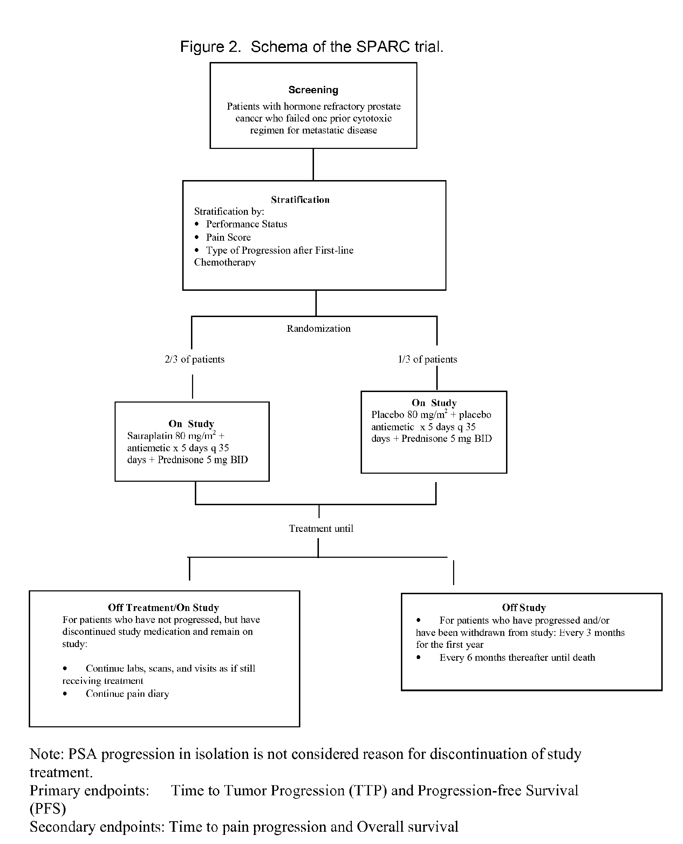

The study schema for this protocol is shown in Figure 2.

### 2.0 OBJECTIVES

The primary objectives for this study are to compare both the time to disease progression (TTP) and the progression-free survival (PFS) in patients with hormone refractory prostate cancer randomized to either satraplatin plus prednisone (S+P) or placebo plus prednisone (P) after failure of one prior chemotherapy regimen for metastatic disease.

The secondary objectives are to compare time to progression of pain and overall survival.

### 3.0 STUDY DESIGN

This is a multi-center, multi-national, double-blind, randomized Phase III study for patients with stage D2 hormone refractory prostate cancer previously treated with one and no more than one prior cytotoxic chemotherapy regimen for metastatic disease. During this study, the efficacy and safety of satraplatin (with antiemetic) plus prednisone or placebo (with placebo antiemetic) plus prednisone will be evaluated. Eligible patients will be randomized to receive either satraplatin plus prednisone or placebo plus prednisone (2:1 ratio). Blinding will ensure that only those patients receiving active satraplatin will receive active antiemetic therapy.

Patients will be stratified according to:
Performance Status (ECOG 0 - 1 versus 2)
Average baseline Present Pain Intensity (PPI) score (0-1 versus 2-5) rounded to nearest integer. The baseline value for the PPI score will be the average of the daily PPI scores recorded for a week before randomization, based on at least 5 assessments during the 7-day period.
Type of progression (PSA progression versus tumor progression on prior cytotoxic therapy). Patients with both PSA progression and tumor progression are will be stratified as tumor progression

For this study, patients will continue to be treated with active or placebo satraplatin (plus active or placebo antiemetic) and prednisone until one of the following occurs:
Disease progression (see Section 10.3)
   - PSA rise in isolation is not considered reason for study drug discontinuation
   - Bone scan progression (see Section 10.4.1) in isolation or in presence of improvement ofPSA and/or symptoms is not cause to stop treatment
Intolerable and unmanageable toxicity to satraplatin and prednisone or prednisone alone (see Section 10)
Patient withdrawal of consent
Noncompliance

If untoward and unmanageable active or placebo satraplatin-associated toxicities are observed, in the absence of progression, the patient will continue on prednisone alone. If prednisone is discontinued due to toxicity in the absence of progression, the patient will continue on active or placebo satraplatin (plus active or placebo antiemetic) alone.

For patients who have not progressed, but discontinue study medication and remain on study (section 9.4.1), the schedule of assessment shown in the table in Section 9.3 should be followed. Patients who withdraw, or are withdrawn from the study (section 9.4.2) for disease progression, will be monitored for toxicity for at least 30 days and for survival every 3 months for the first year, and every 6 months thereafter until death. Patients who are taken off study without evidence of progression will also be followed for survival every 3 months for the first year, and every 6 months thereafter until death.

The final analysis will be performed after approximately 700 events. That number of events takes into consideration the 2:1 randomization scheme. An interim analysis will also be conducted.

### 4.0 INSTITUTIONAL REVIEW BOARD/ETHICS COMMITTEE, INFORMED CONSENT AND DECLARATION OF HELSINKI

The trial will be conducted under appropriate IRB/Ethics Committee approval and supervision, informed consent and in accordance with the Declaration of Helsinki (1964), as amended in Scotland (2000)

### 5.0 INCLUSION/EXCLUSION CRITERIA

To be admitted to the study, patients who qualify for the study must meet all of the following inclusion criteria and none of the following exclusion criteria.

### 5.1 Inclusion Criteria

5.1.1 Patients who meet the diagnostic criteria of stage D2 (Appendix 6) metastatic adenocarcinoma of the prostate that is unresponsive to hormone therapy and progressive after a minimum of two cycles of one prior chemotherapy regimen for metastatic disease that may have also included prednisone therapy.

For the purpose of this study, multiple courses of a taxane-based regimen may count as a single regimen. For example, a patient who was retreated with a taxane-based regimen, following an initial response and a treatment holiday, may be considered as having one course of chemotherapy. Multiple courses of a non-taxane agent or a combination chemotherapy regimen, administered in a similar fashion (i.e. with treatment holidays) may count as a single regimen, but patient eligibility will be determined on a case-by-case basis.

For the purpose of this study, estramustine will be considered a chemotherapeutic agent if it was administered in conjunction with another cytotoxic agent, such as docetaxel, paclitaxel, or vinblastine, or if it was administered as a single agent (for a minimum of 30 consecutive days), following the documentation of hormone-refractory disease. If it was administered as a single agent, prior the documentation of hormone-refractory disease, it will be considered a hormonal agent.

Progression after prior chemotherapy is defined as one of the following:
Tumor progression based on tumor growth according to the RECIST criteria (see Sections 10.4.2). If tumor progression disease is based on bone scan, two or more new lesions are required (see section 10.4.1)

Progression based on increasing PSA⁵ defined as:

| | |
|---|---|
| • For patients with a ≥ 50% decrease in PSA level since the onset of the prior chemotherapy: | An increase of at least 50% of PSA levels above nadir with an increase in the absolute-value PSA level by at least 5 ng/ml confirmed on a second measurement at least one week later. |
| • For patients with less than a 50% decrease in PSA level since onset of the prior chemotherapy: | An increase of at least 25% of PSA levels above nadir or, in the absence of a PSA decrease, an increase of at least 25% of PSA levels above baseline of prior chemotherapy. In either case, the increase in the absolute-value PSA level must be by at least 5 ng/ml and increase must be confirmed on a second measurement at least one week later. |

- 5.1.2: Males older than 18 years
- 5.1.3: Performance Status ECOG ≤ 2 (see Appendix 1)
- 5.1.4: Life expectancy > 3 months
- 5.1.5: Pre-study PSA (within 7 days prior to randomization)
- 5.1.6: All patients must have a chest X-ray and/or a CT scan of the chest and a CT scan of the abdomen and pelvis within 21 days prior to randomization. Patients must also have had a bone scan within 21 days prior to randomization. If a patient has a CT scan of the chest, rather than a chest X-ray at baseline, follow-up tests must continue to be CT scans, not chest X-rays.
- 5.1.7: Patients must have been surgically or medically castrated. If method of castration is LHRH agonists (leuprolide or goserelin), then patient should be willing to continue the use of LHRH agonists during protocol treatment. Castration using LHRH agonists should not be interrupted, and patients who have stopped treatment should be willing to restart. If there is any question regarding the result of medical castration, this will be verified by obtaining a testosterone level (serum testosterone level < 50 ng/dl).
- 5.1.8: Patients may have received prior surgery. However, at least 21 days must have elapsed since completion of surgery, and patient must have recovered from all side effects.
- 5.1.9: Patients must have recovered from all side effects from prior chemotherapy.
- 5.1.10: Adequate bone marrow function (to be determined within 14 days of randomization):
- White blood cell count ≥ 3 x 10⁹/L
- Absolute neutrophil count (neutrophils and bands) ≥ 1.5 x 10⁹/L
- Platelet count ≥ 100 x 10⁹/L
- Hemoglobin >9 g/dL
- 5.1.11: Adequate liver function (to be determined within 14 days of randomization):
• Total bilirubin ≤1.5 x Institutional ULN
• AST or ALT ≤2 x Institutional ULN
- 5.1.12: Adequate renal function (to be determined within 14 days of randomization)
• serum creatinine ≤1.2 x Institutional ULN
- 5.1.13: Patients must be able to swallow capsules.
- 5.1.14: Patients who have already initiated bisphosphonate therapy prior to entry are eligible, provided any bone symptoms have stabilized, and should continue bisphosphonate therapy while on this trial.
- 5.1.15: Patients must give written informed consent before study participation and agree to complete the daily pain and analgesic assessments during the study until the last study visit.
- 5.1.16: Patients must have a stabilized analgesic regimen (see section 10.7.2).

- **5.2**: **Exclusion Criteria**
- 5.2.1: Patients who have received more than one prior cytotoxic chemotherapy regimen for metastatic disease. Prior treatment with satraplatin or other platinum containing compounds will exclude the patient. Previous non-cytotoxic immune therapies will not exclude the patient.
- 5.2.2: Patients with a history of prior malignancy except basal or squamous cell skin cancer. Patients with historically remote malignancies of other types may be entered after consultation with and approval by the Sponsor.
- 5.2.3: Patients who have received radiation therapy to >30% of the bone marrow (see Appendix 2), or who have received strontium-89, rhenium-186, or rhenium-188 will be excluded from this trial. Patients who have received prior radiotherapy must have recovered from acute toxicity due to radiation. At least 28 days must have elapsed since the completion of radiation therapy and the patient must have recovered from side effects. Patients who have received samarium-153 may be considered for the study because samarium is better tolerated than the other radioactive isotopes. Patients who have received samarium-153 must have adequate bone marrow reserve.
- 5.2.4: Patients who, in the opinion of the physician, have a serious concurrent uncontrolled medical disorder.
- 5.2.5: Patients with a history of major gastrointestinal surgery or pathology likely to influence absorption of oral medications.
- 5.2.6: Patients with a disease where corticosteroids are contraindicated, e.g. active gastric or duodenal ulcer, or poorly controlled insulin dependent diabetes. Patients with well-controlled insulin-dependent diabetes may be considered, providing they understand their glucose levels will increase, and their insulin dose will require adjusting.
- 5.2.7: Patients with a known history of brain metastases.
- 5.2.8: Patients who have received any chemotherapeutic or investigational agent given within 21 days prior to randomization. For nitrosoureas or mitomycin C, six weeks should have passed prior to randomization. For patients who have received estramustine as a single agent for more than 1 month continuously prior to randomization, the treatment-free interval until the first day of study drug administration may be reduced to 7 days. For patients who have received estramustine as a single agent for less than 1 month continuously prior to randomization, the treatment-free interval until the first day of study drug administration must be 14 days.
- 5.2.9: Patients receiving flutamide, nilutamide, or another antiandrogen, including ketoconazole, prior to enrollment in this trial will have the agent discontinued for a minimum period of 4 weeks (6 weeks for bicalutamide and nilutamide) See Note below
***Note: Patients Receiving Flutamide or Other Antiandrogens***
***Withdrawal of antiandrogen therapy has been associated with disease responses that are indicated by decreases in PSA and clinical improvement in disease-related symptoms²⁹⁻³¹. For this reason, all patients receiving flutamide, nilutamide, or another antiandrogen prior to enrollment in this trial will have the agent discontinued and then be re-evaluated. Patients will be observed for a minimum period of 4 weeks (6 weeks for bicalutamide and nilutamide) to document disease status following antiandrogen withdrawal. Lack of response to antiandrogen withdrawal will be defined as any increase in PSA and*/*or progression of measurable or non-measurable disease.***

### 6.0 CONCOMITANT THERAPY

Satraplatin is a strong inhibitor of CYP enzymes (cytochrome P450 system). This may result in increased toxicity of certain drugs (see Appendix 5). Patients will be monitored closely for drug interactions or adverse reactions to concomitant medications.
Investigators are cautioned to adjust concomitant medication dosages appropriately.

Loperamide can be given for diarrhea.

Patients will not receive any clinical investigational drugs or any additional antineoplastic therapy (including the new use of estrogens or radiation therapy or PC-SPECS) while participating in this study, nor will they receive corticosteroids (such as dexamethasone for nausea or vomiting) other than that prescribed in the study regimen.

All prescription and over-the-counter concomitant medications taken will be recorded on the Case Report Forms (CRFs).

### 6.1 Agents for Pain

Patients may use any analgesics for the treatment of pain, including narcotic and non-narcotic agents.

### 6.2 Antiemetic Prophylaxis

Premedication will be given to patients in both treatment groups. Active and placebo granisetron 1 mg bid will be provided by the Sponsor.

Patients will receive active or placebo antiemetic therapy 1 hour prior to study therapy dosing and approximately 8 hours after treatment dosing.

If these agents prove ineffective, the Investigator may use another antiemetic regimen (i.e., ondansetron, tropisetron, or dolasetron). The type and period of all antiemetic therapy as well as any other concomitant medication administered will be recorded in the CRFs.

### 6.3 Growth Factors

G-CSF, GM-CSF or other bone marrow stimulants (including erythropoietin) are permitted when clinically indicated. G-CSF and GM-CSF should not be used prophylactically.

### 7.0 RANDOMIZATION

### 7.1 Treatment assignment

After ensuring that the patient meets all eligibility criteria and has given informed consent to participate in the study, the study center will obtain the patient's number and treatment assignment by accessing a centralized call-in system.

The following information is required at the time of randomization:
Protocol number
Treatment center and Principal Investigator's name
Patient's initials and birth date
Caller's name and responsible physician's name, and
Stratification factors

Subjects will be randomized at a ratio of 2:1 to receive either satraplatin plus prednisone or placebo plus prednisone.

Treatment with study drug should begin within 3 days after randomization, and preferably, the same day as randomization.

### 7.2 Stratification Parameters

Performance Status (ECOG 0 - 1 versus ≥2) (See Appendix 1)

Average baseline Present Pain Intensity (PPI) score (0-1 versus 2-5) rounded to nearest integer as follows:
- 0 = No disease related pain
- 1 = Mild disease related pain
- 2 = Discomforting pain resulting from disease
- 3 = Distressing pain resulting from disease
- 4 = Horrible pain resulting from disease, and
- 5 = Excruciating pain resulting from disease
   - Type of progression after prior chemotherapy: isolated PSA rise (see Section 5.1) versus tumor progression (see Section 10.4). Patients with both PSA elevation and tumor progression will be stratified as tumor progression.

### 8.0 TREATMENT PLAN

Patients will be dosed orally with active or placebo satraplatin based on body surface area (BSA).

Using a combination of 50 mg capsules and 10 mg capsules, patients will receive an initial dosage of 80 mg/m². If toxicities occur that warrant a dose reduction (see Section 8.3), the patient will receive a dosage of 60 mg/m². If subsequent toxicities are experienced that warrant a second dose reduction, the patient will receive a dosage of 40 mg/m² (see Section 8.3). There will be no more than two dose reductions allowed. Once a patient has had a dose reduction, no re-escalations will be allowed.

Satraplatin and satraplatin placebo will both be supplied as 50 mg capsules and 10 mg capsules.

### 8.1 Dose Administration

Patients should be observed in the physician's office for at least one hour following the first dose of satraplatin.

### Active or placebo satraplatin plus prednisone

On days 1-5:
- Prednisone 5 mg and active or placebo antiemetic 1 mg administered orally one hour prior to satraplatin
- Active or placebo satraplatin 80 mg/m² orally. Patients should take satraplatin on an empty stomach (no food for 1 hour before or 2 hours after dosing)
- Prednisone 5 mg and active or placebo antiemetic 1 mg administered orally 8 hours after dosing

On days 6-35:
- Prednisone 5 mg in AM and 5 mg in PM

Patient may use 120 mL to 240 mL (4 - 8 fluid ounces) of clear liquids for administration of study medication. Placebo satraplatin and placebo antiemetic will be visually indistinguishable from active satraplatin and active antiemetic respectively.

If active or placebo satraplatin dosing is interrupted during a treatment cycle, it may be restarted as long as the planned 5 days of dosing occurs within an 8-day period (maximum interruption = 3 days). The Investigator may reduce the dose in accordance with Section 8.3.1.

**If there is more than a 3 consecutive day delay, no further doses should be given in the cycle.** The patient will be allowed to begin the next cycle a minimum of 35 days after the first day of the previous cycle if the criteria for re-treatment are met (see Section 8.2).

Prednisone is administered twice daily throughout the study period and is not cycled, in the absence of prednisone toxicity.

The total dose of active or placebo satraplatin delivered should be rounded to the nearest 10 mg (i.e., if total dose < 165 mg deliver 160 mg; if total dose ≥ 165 mg deliver 170 mg) after adjustment for patient body surface area (BSA).

### Antiemetic: Active and placebo (granisetron)

Active and placebo (granisetron) 1 mg bid will be provided by the Sponsor. Patients will receive active or placebo antiemetic therapy daily, for 5 consecutive days every 35 days, at 1 hour prior to study therapy dosing and approximately 8 hours after study therapy dosing.

Blinding will ensure that only those patients receiving active satraplatin will receive active antiemetic therapy.

### 8.2 Retreatment

Patients may be retreated every 35 days if the following criteria are met:
- Absolute neutrophil count is ≥ 1.5 x 10⁹/L and platelets ≥ 100 x 10⁹/L,
- Non-hematologic toxicity ascribed to study drugs resolves to baseline or ≤ grade 1, with the exception of alopecia; or ≤ grade 2 for pain
- There has been no progression: all potential endpoints have been reviewed, and it has been determined that neither tumor progression (section 10.4), nor a skeletal-related event (section 10.5), nor a symptomatic event (section 10.6) has occurred.
**Note: Hematologic values should be established prior to retreatment.**

Subsequent to the retreatment, all laboratory tests must be performed and reviewed by the Investigator at the scheduled study visit before administration of study medications.

When grades 3 or 4 laboratory abnormalities occur, the pertinent test should be regularly repeated until resolution to less than or equal to study subjects baseline. These results are to be entered into the CRF.

Laboratory tests may be performed up to 2 days prior to the scheduled study visit, and if the retreatment criteria are met, the patient may be retreated. If the retreatment criteria are not met, the patient may not be retreated until they are met.

### 8.3 Dose Level Modification of Active or Placebo Satraplatin

### 8.3.1 Dose Reductions for Toxicity and Delayed Recovery

The dose of active or placebo satraplatin will be modified based on the most severe toxicities observed during the previous course:

**Hematologic Toxicity and Delayed Recovery**

| **Variable** | **Finding** | **Dose Modification** |
|---|---|---|
| **Hematologic toxicity** (based on previous cycle nadir)* | Platelets ANC. ≤ 25.0 x 10⁹/L or ≤ 0.5 x 10⁹/L | Decrease by one dose level |
| | | |
| **Delayed recovery for Retreatment due to hematologic toxicity**** | Less than or equal to 6 weeks | No change in dose level |
| | Between 6 and 8 weeks | Decrease by one dose level |
| | More than 8 weeks | Off satraplatin treatment*** |
| **Neutropenic fever** | | Decrease by one dose level |

| | | |
|---|---|---|
| *Complete blood counts must be available on a weekly basis in the previous course ** Time from Day 1 of last course of therapy *** Prednisone will be continued until progression | | |

| **Non-Hematologic Toxicity** | |
|---|---|
| **Non-hematologic toxicity (ascribed to satraplatin)** | **Dose Modification** |
| Gastrointestinal toxicity (nausea, vomiting, diarrhea) ≥ grade 3* | Decrease by one dose level |

| | |
|---|---|
| *Active or placebo satraplatin will be discontinued if grade 4 vomiting or diarrhea occurs despite medical intervention and one dose reduction. | |

Toxicities as described above will determine the necessary dose adjustments:

| **Dose Level** | **Active or Placebo Satraplatin Dose** |
|---|---|
| Starting | 80 mg/m²/d |
| First Reduction | 60 mg/m²/d |
| Second Reduction | 40 mg/m²/d |

No more than two dose reductions are permitted. No dose re-escalation will be allowed after a dose reduction.

*Note: If a patient has had two dosage reductions and has ANC* ≤ *0.5 x 10⁹*/*L and*/*or platelet count ≤ 25 x 10⁹*/*L and*/*or dose delay of greater than 8 weeks, the patient will be discontinued from the study.*

The Investigator may use his/her own judgment as to whether an abnormal finding is sufficient reason to immediately withdraw the patient from the study. If the Investigator judges a laboratory value to be serious and life-threatening, he/she should immediately withdraw the patient from the study and initiate the appropriate therapy (see Section 13).

### 8.3.2 Dose Escalation

The starting dose of active or placebo satraplatin will be 80 mg/m²/day for 5 consecutive days.

An escalation in dose up to 100 mg/m²/day for 5 consecutive days will be allowed as follows:
Patient must have completed at least 2 cycles of active or placebo satraplatin with no incidence of:
   - ≥ grade 2 hematologic toxicities
   - ≥ grade 2 gastrointestinal toxicities (while receiving loperamide treatment); or
   - grade 4 fever toxicities
   - dosing interruption or delay (≥ 6 weeks) due to toxicities

- Patient must have no evidence of disease progression (see Section 10.4)
- Patients will only be allowed one dose escalation

Following dose escalation, patients will have weekly CBCs for the next three treatment cycles.

Patients who have had a dose reduction will not be eligible for re-escalation.

### 8.4 Discontinuation of Active or Placebo Satraplatin

Patients will stop active or placebo satraplatin (and antiemetic) treatment if any of the following occur:
- Disease progression as described in Section 10.3. PSA rise in isolation is not a reason for study drug discontinuation
- Neutropenia (≤ 0.5 x 10⁹/L) or thrombocytopenia (≤ 25 x 10 ⁹/L) despite dose reduction to 40 mg/m²/d
- Grade 3 or 4 hepatic (lasting >7 days), renal, cardiac, pulmonary, or neurological toxicity
- Grade 4 vomiting or diarrhea that cannot be controlled by medical treatment and that occurs after one dose reduction
- Retreatment delay of>8 weeks due to study drug-related hematologic toxicities

If active or placebo satraplatin is discontinued, the patient will continue on prednisone alone until progression (see Section 10.3), intolerable prednisone toxicity, patient withdrawal of consent, or noncompliance (see Section 12.0).

### 8.5 Dose Level Modification of Prednisone for Toxicity and Delayed Recovery

The dose of prednisone may be reduced to 5 mg once daily for ≥ grade 3 hyperglycemia or other toxicity attributed to prednisone. The dose may not be re-escalated following dose reduction.

### 8.6 Discontinuation of Prednisone

Prednisone will be discontinued if untoward and unmanageable toxicity occurs (e.g., GI ulcerations, symptomatic hyperglycemia unresponsive to medical therapy) despite dose reduction as specified in Section 8.5. If prednisone is discontinued in the absence of disease progression (see Section 10.3), the patient will continue on active or placebo satraplatin.

### 8.7 Treatment Duration

In the absence of disease progression (see Section 10.3), treatment will continue until the occurrence of untoward and unmanageable toxicity for both satraplatin (active or placebo) and prednisone (see Section 8.0), or patient withdrawal of consent, or noncompliance (see Section 12.0).

If untoward and unmanageable active or placebo satraplatin associated toxicity is observed in the absence of progression, the patient will continue on prednisone alone. When the patient is treated with prednisone alone, cycles will be continued at 35 day intervals.

If untoward and unmanageable prednisone toxicity is observed, active or placebo satraplatin alone will be continued in 35 day cycle.

The Investigator will determine subsequent therapy after discontinuation from this trial. After discontinuation from the study, patients will be monitored for toxicity for at least 30 days and for survival every 3 months for the first year, and every 6 months thereafter until death.

Crossover to satraplatin is not allowed in patients randomized to the placebo arm.

### 9.0 STUDY SCHEDULE

### 9.1 Subject Evaluation

**Prestudy:** Patients will be evaluated by their physician to determine eligibility prior to enrollment in the study. Baseline evaluation will be performed according to the schedule of assessments in Section 9.3

**Daily:** Patients will keep a *daily* record of their disease-related pain using the 6-point PPI scale (0= no pain to 5 = excruciating pain), together with their *daily* analgesic intake.

**Weekly:** Once enrolled, on a weekly basis for the first 3 courses, patients will be required to undergo blood draws for complete blood count (CBC). If all the laboratory results are within normal range, assessments may be done only on Day 1 of each cycle starting on the 4^{th} course. Following discontinuation of satraplatin, CBCs will be obtained on a weekly basis until bone marrow recovery occurs to CTC toxicity grade ≤ 1, then the CBC may be done every 5 weeks until full recovery. Patients who are dose escalated will also have weekly CBCs for the 3 cycles following such dose escalation.

**Each Cycle:** Prior to starting each dosing cycle, the patients will be required to undergo an evaluation by their physician as detailed in Section 9.3. This includes routine history and physical examination, weight, performance status, pain assessment, serum chemistry, CBC level and a blood draw for PSA determination except day 1 of cycle 1.

**Every 2 Cycles:** After receiving each even numbered cycle of treatment, the patient will be evaluated for tumor response (in addition to the tests scheduled prior to each cycle outlined above). If baseline assessments are negative, subsequent scans for tumor assessment are optional and may be ordered at the physician's request as clinically indicated.

**End of Study:** When a patient discontinues from the study, he will be evaluated for weight and performance status measurements, and pain (PPI and Analgesic Scores), tumor and bone scan assessments, and will undergo a blood draw for PSA, serum chemistry, and CBC.

### 9.2 Patient PPI Assessment and Diary Card

Upon entry to the study, patients will be asked to complete a diary card. They will keep a *daily* record of their disease-related pain using the 6-point PPI scale (0= no pain to 5 = excruciating pain), together with their *daily* analgesic intake. Detailed instructions will be given by the Investigator or nurse to the patients concerning the completion of the diary.
Diaries will be collected from the patient after completion of each cycle. They will be promptly reviewed for compliance by the Investigator. Diaries will be collected until end of study visit.

### 9.3 Study Parameters

The following table summarizes the minimal assessments required for evaluation of the study drug's effects. Measurements for other parameters and/or increased frequency of examination may be required, depending on the findings during the study.

### Schedule of Assessments

| Assessment | Pre-Treatment¹ | On-study Phase | | | | End of study visit | Follow-up visits¹¹ |
|---|---|---|---|---|---|---|---|
| | | Daily | Each week | Prior to each cycle | Every 2 cycles | | |
| History and physical (H&P) | X | | -- | X⁸ | -- | X | X¹⁰ |
| Weight, PS, | X | | -- | X | -- | X | X¹⁰ |
| Toxicity Assessment | Continuous | | | | | | |
| PSA² | X | | | X | -- | X⁹ | -- |
| Bone scan³ | X | | -- | -- | X | X⁹ | -- |
| Tumor Assessment⁴ | X | | -- | -- | X | X | -- |
| CBC, platelets, ANC⁵ | X | | X⁵ | X⁵ | -- | X | -- |
| Serum Chemistry⁶ | X | | -- | X | -- | X | -- |
| Chest X-ray⁷ | X | | -- | -- | X | -- | -- |
| Electrocardiogram⁷ | X | | -- | -- | X | -- | -- |
| PPI Diary | X | X | | | | X | |
| Analgesic Diary | X | X | | | | X | |

| | | | | | | | |
|---|---|---|---|---|---|---|---|
| ¹All pre-treatment procedures and tests must be done within 2 weeks prior to randomization (radiology assessments are to be completed within 21 days prior to randomization). Clinical eligibility, based on H&P, PS, and labs, must be determined prior to radiologic scans. ²Pre-study PSA should be done within one week of randomization. **PSA is not required for Day 1 of Cycle 1 if already done at screening.** ³Bone scan frequency of q 2 cycles for the first 6 cycles then q 3 cycles until 12 cycles, then q 6 cycles thereafter. Bone scans will be repeated even if baseline is negative. ⁴If baseline tumor assessments are positive for measurable and/or evaluable lesions: nuclear medicine scans, chest X-rays, CT/MRI scans of abdomen and pelvis and other imaging techniques will be done every other cycle. The same imaging techniques will be used on repeat assessments. These assessments will be repeated at a 5-weekinterval if > 50% tumor reduction is observed. If baseline assessments are negative, subsequent scans for tumor assessment are optional and may be ordered at the physician's discretion as clinically indicated. Tumor assessments may be performed within 1 week of scheduled study visit. ⁵Weekly for the first 3 courses and weekly for the 3 courses following dose escalation. If all the laboratory results are within normal range during the first 3 courses, assessments may be done on Day 1 of each cycle starting on course #4. Following discontinuation of satraplatin, CBCs will be obtained on a weekly basis until bone marrow recovery occurs to CTC toxicity grade ≤ 1, then the CBC may be done every 5 weeks until full recovery. ⁶Sodium, potassium, bicarbonate*, BUN, creatinine, bilirubin, AST and/or ALT, alkaline phosphatase, LDH, albumin, total protein, calcium, phosphate, urate and glucose. (Bicarbonate may be included or not, as per the local standard.) ⁷Baseline required within 21 days and repeated every other cycle. A chest CT may be used in lieu of chest x-ray. However, the same methodology should be used throughout the study. ⁸Pre-cycle H&P may be done within 3 days of start of cycle. ⁹End of study bone scans and tumor assessment scans must be completed within 21 days of discontinuing study. ¹⁰Patients will be monitored every 3 months for 1 year after off-study and then every 6 months until death. ¹¹This column only refers to patients who are off treatment **and** off study. For patients who discontinue study medication and remain on study, please follow the scheduled assessments as if these patients are still on treatment (see Section 9.4.1 for detail). | | | | | | | |

### 9.4 Follow-Up

### 9.4.1 Patients Who Discontinue Study Medication and Remain On Study

There are a number of circumstances in which a patient may discontinue study medication, yet remain on the study. Such a patient should not have had a documented progression. Possible reasons for discontinuing study medication include experiencing an SAE and not tolerating study medication(s).

For such a circumstance, both the patient AND the investigator should continue to comply with the schedule of assessment shown in the table in Section 9.3. The patient will continue to complete the PPI diary, return to the investigator every 10 weeks for all visits as if he were still on study medication, and continue to receive the scheduled tests and tumor assessment scans. All clinical and laboratory data during this period will continue to be collected as indicated on the schedule. Schedule 9.3 shall be followed until the patient:
**1. experiences disease progression,**
**2. withdraws consent,**
**3. starts a new treatment or goes onto a new study protocol,**
**4. shows non-compliance to the SPARC protocol in a manner that interferes with the evaluation of endpoints,**
**5. dies, or**
**6. the physician determines it is no longer in the patient's best interests to continue in the SPARC Trial.**

Upon the occurrence of any of these circumstances, the patient will be considered to have left the study, and the appropriate written documentation shall be made by the investigator, including, where appropriate, those assessments identified as "end of study visit" in Protocol Section 11.3.

In the alternative circumstance, where a patient on study has not had an progressive event but has stopped taking study drug, and either the patient or the investigator has determined that the patient should leave the study, then this decision to leave the study should be documented in writing, and where feasible, the "end of study visit" assessments conducted and further assessment of the patient provided according to the schedule in Protocol Section 9.4.

### 9.4.2 Patients Who Are Withdrawn from the Study (Off-Study)

A patient may be withdrawn (off-study) from the study for any of the following reasons:
**1. experiences disease progression,**
**2. withdraws consent,**
**3. goes onto a new study protocol,**
**4. shows non-compliance to the SPARC protocol in a manner that interferes with the evaluation of endpoints,**
**5. dies, or**
**6. the physician determines it is no longer in the patient's best interests to continue in the SPARC Trial.**

After a patient is withdrawn from the study, the Investigator will monitor him for new toxicities for at least 30 days, and follow existing adverse events until resolution. The Investigator will monitor for survival every 3 months for the first year, and every 6 months thereafter until death.

### 10.0 CRITERIA FOR EVALUATION AND STUDY ENDPOINTS

**10.1 Primary Endpoints**
   Time to Disease Progression
   Progression-free Survival
**10.2 Secondary Endpoints**
   Time to pain progression
   Overall survival
**10.3 Progression**

### 10.3.1 Time to Disease Progression (TTP)

Time to disease progression is defined as the time from randomization to when disease progression is initially reported. Disease progression will be defined from a composite endpoint based on first occurrence of either one of the following:
- Tumor progression (see Section 10.4)
- Skeletal Events (see Section 10.5)
- Symptomatic Progression (see Section 10.6)

### 10.3.2 Progression-free Survival (PFS)

Progression-free survival is defined as the time from randomization to disease progression or death. Therefore, any TTP event is also a PFS event. The only difference between TTP and PFS occurs when disease progression has not been observed in a patient before his death, and the cause of death of this patient is not malignant disease. In this case, the patient is censored for disease progression on the death date, but the patient has an event for PFS on the death date.

### 10.4 Assessment and Definition of Tumor Progression

The vast majority of indicator lesions are expected to be evaluated radiologically.

Specific considerations are given here for bone lesion evaluations.

### 10.4.1 Bone Lesions

Bone scans should be used to assess bone lesions. Intensity changes will not be used to determine progression, as increased uptake does not constitute unequivocal progression. Progression by bone scan criteria *alone* will require two or more new lesions. Bone scan progression in isolation or in presence of improvement of PSA and/or symptoms is not cause to stop treatment. If only one new lesion is documented, the lesion should be confirmed as being cancerous by additional radiographic studies starting with a plain radiograph. Follow up studies with MRI and/or CT scan may be performed if the plain radiographic is non-diagnostic.

### 10.4.2 Soft Tissue Lesions

Progression in soft tissue lesions will be assessed using the Response Evaluation Criteria In Solid Tumors (RECIST) criteria (Appendix 3). Accordingly, progression of measurable or non-measurable soft tissue lesions is defined as either of the following:
- At least a 20% increase in the sum of the longest diameter of target lesions, taking as reference the smallest sum of the longest diameters recorded since treatment initiation or the appearance of one or more new lesions
- Reappearance of any lesion which had disappeared, OR appearance of one or more new lesions and/or unequivocal progression of existing non-target lesions

### Exceptions:

(1) In cases for which initial tumor flare reaction is possible (hypercalcemia, increased bone pain, erythema of skin lesions), either symptoms must persist beyond four weeks or there must be additional evidence of progression.
(2) Lesions that appear to increase in size due to presence of necrotic tissue will not be considered to have progressed.

### 10.5 Skeletal Related Events

Progression due to skeletal related events is defined as any observation of the following:
- Pathologic bone fracture in the region of cancer involvement
- Radiation therapy to bone
- Cancer related surgery to bone
- Spinal cord or nerve root compression
- Initiation of bisphosphonate therapy in response to new bone pain symptoms
- Change of antineoplastic therapy for bone pain due to prostate cancer

### 10.6 Symptomatic Events

Progression due to Symptomatic Events is defined as any observation of the following:
- Increase in PPI score* or analgesic consumption* (see section 10.7) is defined as either of the following:
   - An increase in cancer related pain, of at least one point from baseline or at least 2 points compared with the nadir, observed for at least 2 weeks (based on 2 or more consecutive weekly PPI determinations), or
   - An increase in the average analgesic score (section 10.7.2) of greater than 25% compared with baseline. This increase should be maintained for a minimum of 2 consecutive weeks.
   *The date of pain progression is defined as the end of the first of the 2 consecutive weeks of increased PPI score or analgesic score.
- An increase in ECOG performance status of ≥2 units compared to baseline attributable to cancer in the Investigator's opinion confirmed by a history exceeding 2 weeks (see Appendix 1)
- Weight loss of greater than 10% of initial body weight attributable to cancer in the Investigator's opinion
- Other clinical events attributable to prostate cancer in the Investigator's opinion requiring intervention such as bladder outlet or ureteral obstruction

### 10.7 Pain Evaluation

Upon entry to the study, patients will be asked to complete a diary card. They will keep a daily record of their disease-related pain, together with their daily analgesic usage. Diaries will be collected from the patient after completion of each cycle and reviewed for compliance by the Investigator. Diaries will be collected until end of study visit.

### 10.7.1 PPI Score

Disease-related pain will be followed in this trial by a patient diary which records the Present Pain Intensity (PPI) score of the McGill-Melzack questionnaire³² on a *daily* basis.

The PPI score is validated and has been used in the registrational studies of mitoxantrone plus corticosteroid in the prostate cancer indication^{16,27-30}.

The PPI scale has verbal descriptors (0 = no pain, 1 = mild pain, 2 = discomforting pain, 3 = distressing pain, 4 = horrible pain, and 5 = excruciating pain); patients will be asked on a daily basis to clarify the average pain level during the previous 24 hours, using the PPI scale. The baseline value for the PPI score will be the average of the daily PPI scores recorded for a week before randomization, based on at least 5 assessments during the 7-day period. The weekly PPI score during the period on-study is the average of the daily PPI scores, based on a minimum of 3 daily PPI assessments during a week's period.

### 10.7.2 Analgesic Score

The amount of analgesics used for disease-related pain will be determined by asking the patient to record the number, types and doses of analgesic drugs in a daily diary. A numeric scale will be used to compute an analgesic score. Patients should be classified as either being on a Chronic Analgesic Regimen (with or without intermittent or prn ("per re nata") dosing) or on an only Intermittent Analgesic Regimen (see 10.7.2.1 and 10.7.2.2). The analgesic score will be determined by the total number of analgesic units (narcotics only, Appendix 4) taken over 1 week's time, based on the recordings in the analgesic diary, divided by the number of days in which a value was recorded. The average score during the week must be based on at least 5 assessments for baseline and at least 3 assessments during treatment; otherwise the score for the week is considered missing.

### 10.7.2.1 Baseline Analgesic Score

### Chronic Analgesic Regimen

For patients on a chronic analgesic regimen (e.g. MS Contin 60 mg PO q12h) with or without pm doses for breakthrough pain, the analgesic score will be calculated as follows: the total score will be determined for the week. The analgesic score will be considered stable if all daily scores during the week differ from the day one score by no more than 25%. If subsequent analgesic scores differ from the day one score by greater than 25% for two or more days, then the score is not yet stable, and it should continue to be monitored until it has become stable during a 7-day period.

The total score for the week will be divided by 7 (or by the number of days in which values were recorded, if less than7) to determine the average daily analgesic score, and this will be considered as the **baseline analgesic score.**

### Intermittent Analgesic Regimen

For patients not an a chronic regimen, taking analgesics only intermittently (prn), the analgesic score will be determined as follows: the total weekly analgesic score will be determined by adding all the scores, on days in which a value was recorded, during 1 week's time. This total will be divided by the number of days in which a value was recorded (including a value of 0). The analgesic score will be considered stable if there is no greater than a 50% increase from the lowest to the highest scores on the days analgesic is taken. If there is a greater than 50% increase, the score is not yet stable, and it should continue to be monitored until it has become stable during a 7-day period. If analgesic is used for a single day during the week, it will be considered as stable. If there is any question regarding stability of analgesic score, this should be presented to one of the GPC Biotech medical monitors.

The total score for the week will be divided by the number of days analgesic was taken and recorded to determine the average daily analgesic score, and this will be considered as the **baseline analgesic score.**

### 12.7.2.2 Average Analgesic Score

### Chronic Analgesic Regimen

For patients on a chronic analgesic regimen, the weekly analgesic score will be determined as follows: the total score, inclusive of prn analgesics, will be determined for the week. This total score will be divided by 7(or by the number of days in which values were recorded, if less than 7) to determine the **average analgesic score.**

### Intermittent Analgesic Regimen

For patients not an a chronic regimen, taking analgesics only intermittently (prn), the total score for the week will be determined by adding all the scores, on days in which a value was recorded, during 1 week's time. This total will be divided, by the number of days in which a value (including 0) was recorded. This will be the average analgesic score.

### 10.7.3 Pain Response

Patients will be considered evaluable for a Pain Response if the baseline PPI and analgesic use was determined, and at least four consecutive weekly assessments of PPI and analgesic score are available during the period after the initiation of therapy until discontinuation of all study medications. The patient's Pain Response will be judged using the weekly PPI and Analgesic Score (see Sections 10.7.1 and 10.7.2).

The criteria for the **Pain Response** are as follows: A 2-point or more reductions in weekly PPI score from baseline (complete loss of pain if baseline PPI score was < 2.0), maintained for at least 5 consecutive weeks, in the setting of a stable or decreasing weekly analgesic score (also compared to baseline). A stable or decreasing weekly analgesic score is defined as no more than 25% increase from the baseline score.

### 10.7.4 Time to Pain Progression*

This is the time from randomization to first observed pain related progression that has been confirmed. Progression due to pain is defined as any observation of the following:
An increase in cancer related pain, of at least one point from baseline or at least 2 points compared with the nadir, observed for at least 2 weeks (based on 2 or more consecutive weekly PPI determinations), or
An increase in the average analgesic score of greater than 25% compared with baseline. This increase should be maintained for a minimum of 2 consecutive weeks.

* The date of pain progression is defined as the end of the first of the 2 consecutive weeks of increased PPI score or analgesic score.

### 10.8 Overall Survival

Overall survival is defined as the time from randomization to death.

### 10.9 Safety

Incidence of adverse events including serious adverse events (*SAEs*) recorded during the study, changes from baseline in vital sign measurements and laboratory values. All patients will be evaluable for toxicity if they have received any study drug. All signs and symptoms will be recorded in the patient's case report form (CRF). Toxic effects of chemotherapy will be assessed using the NCI Common Toxicity Criteria Version 2.0, copies of which are available from various sources including via http.//ctep.cancer.gov/..

### 10.10 Central Adjudication of Disease progression

Disease progression and timing of disease progression will be adjudicated by a committee of experts in a blinded fashion. Bone scans will also be reviewed centrally, in a blinded fashion.

### 11.0 ASSESSMENT OF SAFETY

Safety shall be monitored according to all appropriate procedures, regulations and law. Without limitation: (i) the local clinical laboratory will perform clinical laboratory assessments; (ii) patients will be monitored and questioned at every visit regarding the occurrence and nature of any adverse experiences or events ("AE"s); and (iii) all AEs that are serious, unexpected, and definitely, probably, or possibly related to the study drug must be reported ("SAE"s).

All AEs shall be recorded, monitored and reported according to all appropriate procedures, regulations and law.

Unblinding can only be requested by the Investigator, Regulatory Authority, Chair Data Monitoring Board, Director Pharmacovigilance GPC Biotech Inc. only when the information of study treatment may have a direct impact in the course of action taken for individual or collective benefit of patients, or to follow regulations in jurisdictions where the trial is being conducted.

The DMB will review individual patient data and summary descriptive statistics on adverse events and measures of treatment efficacy. This committee will be composed of at least three experts in biometrics and/or the management of prostate cancer. The frequency of the meeting between these DMB committee members will be based on the number and type of adverse events received at any point in time.

### 12.0 Treatment Discontinuation

Treatment with study drug should be discontinued if it is considered to be in the best interest of the patient. Reasons for treatment discontinuation include:
Disease Progression (see Section 10.3)
Occurrence of intolerable toxicity to both satraplatin and prednisone (see Section 8)
Patient withdrawal of consent or non-compliance

### PSA progression in isolation should not be a cause for patient discontinuation from this protocol.

### Drop-outs

Patients discontinued from the study for reasons unrelated to therapy, such as noncompliance, ineligibility or withdrawal of consent will be considered drop-outs. All of these patients are still evaluable for toxicity and efficacy if they actually received treatment. These patients will be included in the all-randomized analysis.

The reason for patient withdrawal should be included in the CRFs.

The "Intent to Treat" analysis will include all patients randomized, regardless of whether they have received study drug or not.

### Non-compliance

Patients who interrupt study drug intake for greater than 3 days in a single course for two or more consecutive courses or do not complete the patient diary for at least 3 days per week for 2 consecutive weeks will be considered as non-compliant and will be discontinued from the study. These patients will be included in the safety and efficacy assessments.

### 13.0 STATISTICAL CONSIDERATIONS

### 13.1 Overview and Types of Analyses

A detailed statistical analysis plan will be prepared prior to dosing the first patient. The statistical analysis plan will be finalized prior to the interim analysis. Eligible patients at baseline will be randomized to either satraplatin plus prednisone (S+P) or placebo plus prednisone (P) alone in a 2:1 ratio. The primary endpoints are time to disease progression and progression-free survival.

An intent-to-treat analysis is planned as the primary analysis for both efficacy and safety evaluation, where all randomized patients will be included.

In addition, per protocol (PP) analysis may be done as secondary analysis for efficacy. Major differences, if any would be addressed in the appendix. PP analysis will include those patients who a) meet eligibility criteria, b) do not have major protocol violations, and c) meet visit-specific criteria, such as visits occurring within the scheduled visit window and d) complete 12 month follow-up or have died.

### 13.2 Statistical Hypotheses and Level of Significance

The primary and secondary endpoints in the interim and final analyses are listed in Section 10.1 and Section 10.2. For both interim and final analyses, the primary endpoints are time to disease progression and progression-free survival. At the interim analysis, time to pain progression is the secondary endpoint. No regulatory claim will be made at the interim analysis based on the results of time to pain progression unless the primary endpoint is significant. In the final analysis, time to pain progression and overall survival are secondary endpoints.

For each endpoint, the null hypothesis is that there exists no difference between S + P and P in patients' response for that endpoint; the alternative hypothesis is that a difference exists. All tests will be two-sided. For efficacy analysis, a two-sided test result will be claimed significant only if the two-sided p-value is less than or equal to the significance level and if satraplatin is the better arm.

The overall type I error of the interim and final analyses will be controlled at 0.05 level, allocated between the final analysis and the interim analysis.

### 13.3 Statistical Methods

Results will be summarized with descriptive statistics (i.e., sample size, mean, standard deviation, minimum, maximum and median) for continuous variables and with tabulations/frequency tables/case listings for categorical variables. Incidence of adverse events will be tabulated with MedDRA, Preferred Terms within that MedDRA, and may be further tabulated with severity or relatedness. Lab data will also be analyzed and summarized. The statistical methods to be used for efficacy and safety parameters are discussed below.

### Time to Disease Progression, Progression-Free Survival, Time to Pain Progression, and Overall Survival

The log rank test will be used as the primary method to compare the time to disease progression, progression-free survival, time to pain progression, and overall survival time between S+P and P, stratified by the same stratification variables used for randomization. The median and the Kaplan-Meier estimates will be given with 95% CI. Life tables and curves will be provided for each treatment group.

In addition, the Cox model and the non-parametric covariate-adjusted method proposed by Tangen and Koch³³ will be used as secondary methods using various covariates.

### Pain Response, and Best Overall Response for Soft Tissue Lesions

Pain response and best overall response for soft tissue lesions will be performed at the interim and final analyses for exploratory purpose, using Fisher's exact test stratified by the same stratification variables used for randomization.

### Safety Parameters

Incidence of adverse events will be tabulated with MedDRA, Preferred Terms within that MedDRA, and will be further tabulated with severity or relationship to treatment.
Fisher's exact test, stratified by the same stratification variables used for randomization, will be used to compare event rates (grade ≥ 3 versus grade < 3) between treatment groups. Significant p-values (*p* ≤ 0.05) will be flagged if the satraplatin arm has a higher incidence rate.

### 13.4 Sample Size

A total of 912 patients will be enrolled.

### 13.5 Subgroup Analysis

Subgroup efficacy analysis will be done.

### 13.6 Interim Analysis

An interim analysis will be performed when an appropriate number of events of disease progression have been observed.

### 13.7 Final Analysis

The final analysis will be performed after approximately 700 events have been observed.

In the final analysis, the p-values of the secondary efficacy endpoints will be adjusted for multiplicity using the Hochberg method³⁴. The adjustment results using overall type I error of 0.05 will be reported.

### 18.0 REFERENCES

1. Sternberg C. Hormone refractory metastatic prostate cancer. Ann Oncol 1992;3(5):331-5.
2. Kelly W, Slovin S. Chemotherapy for Androgen-independent Prostate Cancer: Myth or Reality. Current Oncol Rep 2000;2:394-401.
3. Petrylak D, Tangen C, Hussain M, et al. SWOG 99-16: Randomized phase III trial of docetaxel (D)/estramustine (E) versus mitoxantrone (M)/prednisone(p) in men with androgen-independent prostate cancer (AIPCA). Proc Am Soc Clin Oncol 2004;23:abstract #3.
4. Eisenberger M, De Wit R, Berry W, et al. A multicenter phase III comparison of docetaxel (D) + prednisone and mitoxantrone (MTZ) + P in patients with hormone-refractory prostate cancer (HRPC). Proc Am Soc Clin Oncol 2004;23:abstract #4.
5. Bubley GJ, Carducci M, Dahut W, et al. Eligibility and Response Guidelines for Phase II Clinical trials in Androgen-Independent Prostate Cancer: Recommendations From the Prostate-Specific Antigen Working Group. J Clin Oncol 1999;17:3461-7.
6. Miller JI, Ahmann FR, Drach GW, Emerson SS, Bottaccini MR. The clinical usefulness of serum prostate specific antigen after hormonal therapy of metastatic prostate cancer. J Urol 1992;147:956-61.
7. Kelly WK, Scher HI, Mazumdar M, Vlamis V, Schwartz M, Fossa SD. Prostate-specific antigen as a measure of disease outcome in metastatic hormone-refractory prostate cancer. J Clin Oncol 1993;11:607-15.
8. Eisenberger MA, Nelson WG. How much can we rely on the level of prostate-specific antigen as an end point for evaluation of clinical trials? A word of caution. J Nat Can Inst 1996;88(12):779-81.
9. Pienta KJ, Redman B, Hussain M, et al. Phase II evaluation of oral estramustine and oral etoposide in hormone-refractory adenocarcinoma of the prostate. J Clin Oncol 1994;12:2005-21.
10. Dawson NA. Treatment of progressive metastatic prostate cancer. Oncology 1993;7:17-27.
11. Eisenberger MA, Simon R, O'Dwyer PJ, Wittes RE, Friedman MA. A reevaluation of non-hormonal cytotoxic chemotherapy in the treatment of prostate cancer. J Clin Oncol 1985;3:827-41.
12. Yagoda A, Petrylak DP. Cytotoxic chemotherapy for advanced hormone-resistant prostate cancer. Cancer 1993;71 (Supplement): 1098-109.
13. Tannock IF, Osoba D, Stockler MR, et al. Chemotherapy with mitoxantrone plus prednisone or prednisone alone for symptomatic hormone-resistant prostate cancer: A Canadian randomized trial with palliative end points. J Clin Oncol 1996;14(6):1756-64.
14. Hudes G, Nathan F, Khater C, et al. Phase II trial of 96-hour paclitaxel plus oral estramustine phosphate in metastatic hormone-refractory prostate cancer. J Clin Oncol 1997;15:3156-63.
15. Petrylak DP, Macarthur R, O'Connor J, et al. Phase I/II studies of docetaxel (Taxotere) combined with estramustine in men with hormone-refractory prostate cancer. Semin Oncol 1999;26(5 Suppl 17):28-33.
16. Hussain M, Petrylak D, Fisher E, Tangen C, Crawford D. Docetaxel (Taxotere) and estramustine versus mitoxantrone and prednisone for hormone-refractory prostate cancer: scientific basis and design of Southwest Oncology Group Study 9916. Semin Oncol 1999;26 (5Suppl 17):55-60.
17. Rozencweig M, Von Hoff DD, Slavik M, Muggia FM. Cis-diaminedichloroplatinum (II) a new anticancer drug. Ann Int Med 1977;86:803-12.
18. Loehrer PJ, Einhorn LH. Cisplatin. Ann Int Med 1984;100:704-13.
19. Prestayko A, D'Aoust J, Issell B, Crooke S. Cisplatin (cis-diaminedichloroplatinum II). Cancer Treat Rev 1979;6:17-39.
20. Rossof AH, Talley RW, Stephens R, et al. Phase II evaluation of cisdichlorodiammineplatinum (II) in advanced malignancies of the genitourinary and gynecologic organs: a Southwest Oncology Group study. Cancer Treat Rep 1979;63:1557-64.
21. Merrin CE. Treatment of genitourinary tumors with cis-dichloroammineplatinum (II): experience in 250 patients. Cancer Treat Rep 1979;63:1579-84.
22. Yagoda A. Phase II trials with cis-dichlorodiammineplatinum (II) in the treatment of urothelial cancer. Cancer Treat Rep 1979;63:1565-72.
23. Yagoda A, Watson R, Natale R. A critical analysis of response criteria in patients with prostate cancer treated with cis-diamminedichlororide platinum (II). Cancer 1979;44:1553-62.
24. Qazi R, Khandekar J-. Phase II study of cisplatin for metastatic prostatic cancer. Am J Oncol 1983;6:203-5.
25. Soloway M, S B, Brady M, et al. A comparison of estramustine phosphate versus cisplatinum alone versus estramustine phosphate plus cisplatinum in patients with advanced hormone refractory prostate cancer who had extensive irradiation to the pelvis or lumbosacral area. J Urol 1983;129:56-61.
26. Moore MR, B TM, deSimone P, Birch R, Irwin L. Phase II evaluation of weekly cisplatin in metastatic hormone-resistant prostate cancer: A Southeastern Cancer Study Group trial. Cancer Treat Rep 1986;70:541-2.
27. Satraplatin. GPC Biotech Inc. IB, 2004.
28. Sternberg C, Hetherington J, Paluchowska B, et al. Randomized phase III trial of a new oral platinum, satraplatin (JM-216) plus prednisone or prednisone alone in patients with hormone refractory prostate cancer. Proc Am Soc Clin Oncol 2003;22: abstract #1586.
29. Scher HI, Kelly WK. Flutamide withdrawal syndrome: Its impact on clinical trials in hormone-refractory prostate cancer. J Clin Oncol 1993;8:1566-72.
30. Figg WD, Sartor O, Cooper MR, et al. Prostate specific antigen decline following the discontinuation of flutamide in patients with stage D2 prostate cancer. Am J Med 1995;98:412-5.
31. Small EJ, Srinivas S. The antiandrogen withdrawal syndrome. Experience in a large cohort of unselected patients with advanced prostate cancer. Cancer 1995;76:1428-34.
32. Melzack R. The McGill Pain Questionnaire: Major properties and scoring methods. Pain 1975;1:277-99.
33. Tangen CM, Koch GG. Nonparametric analysis of covariance for hypothesis testing with logrank and Wilcoxon scores and survival rate estimation in a randomized clinical trial. J Biopharm Stat 1999;9:307-38.
34. Hochberg Y. A sharper Bonferroni procedure for multiple tests of significance. Biometrika 1988;75:800-2.

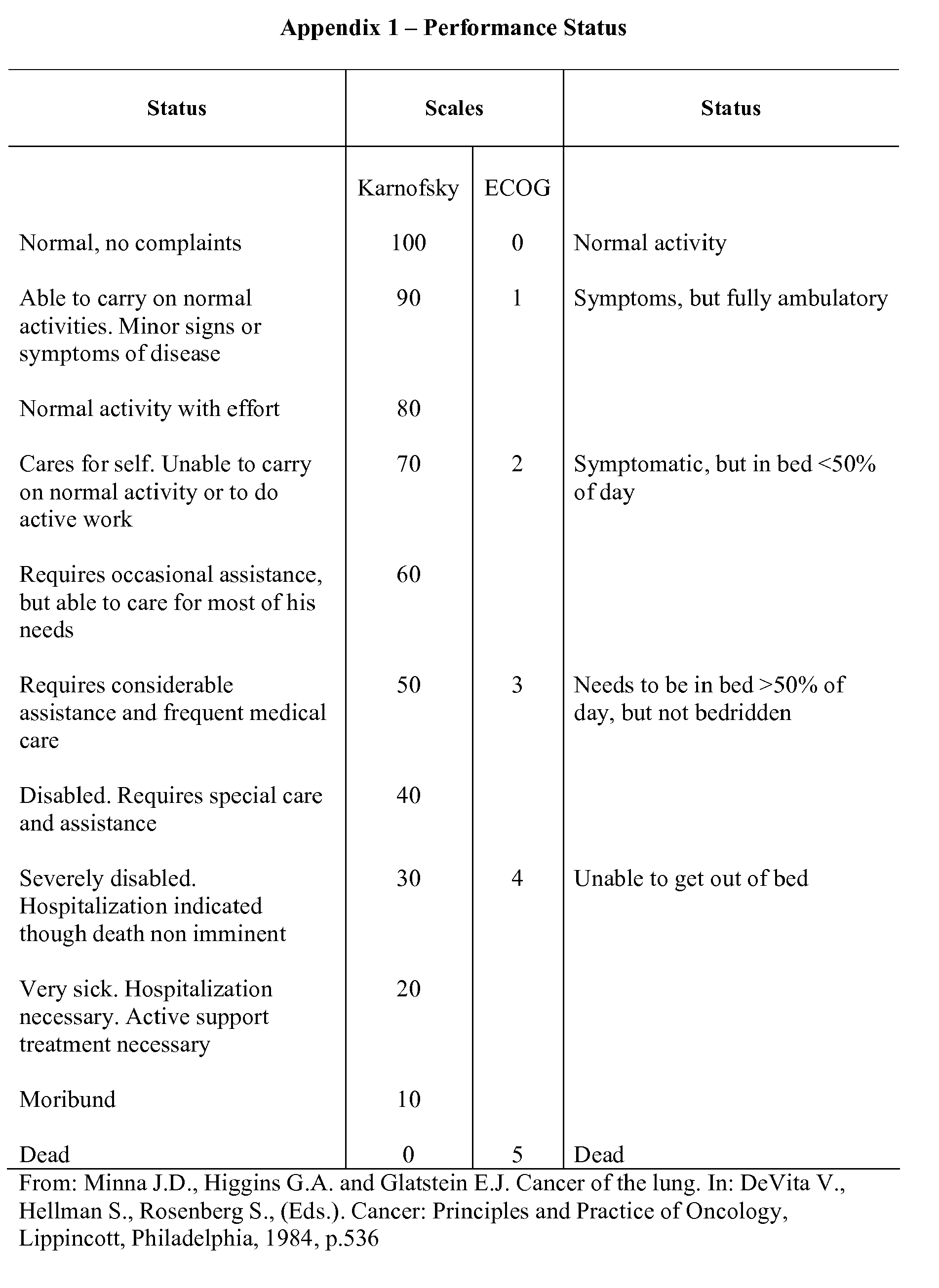

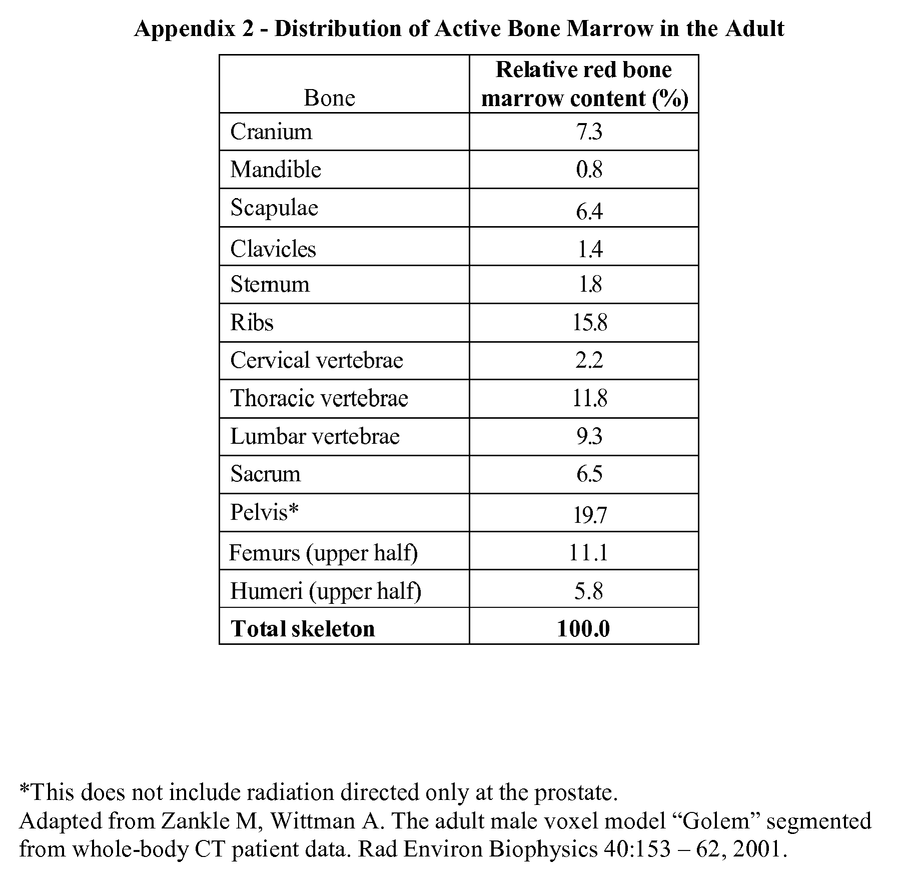

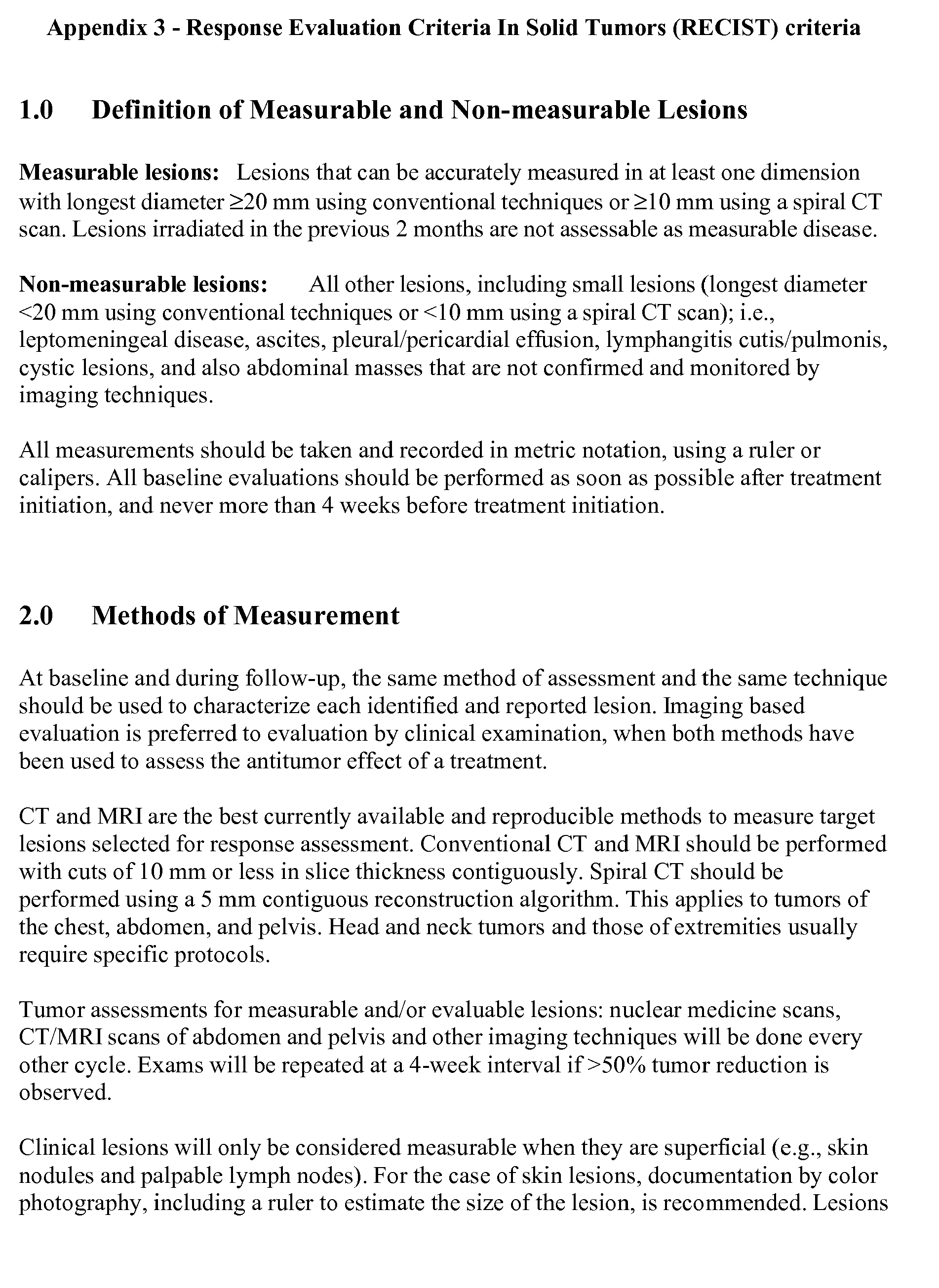

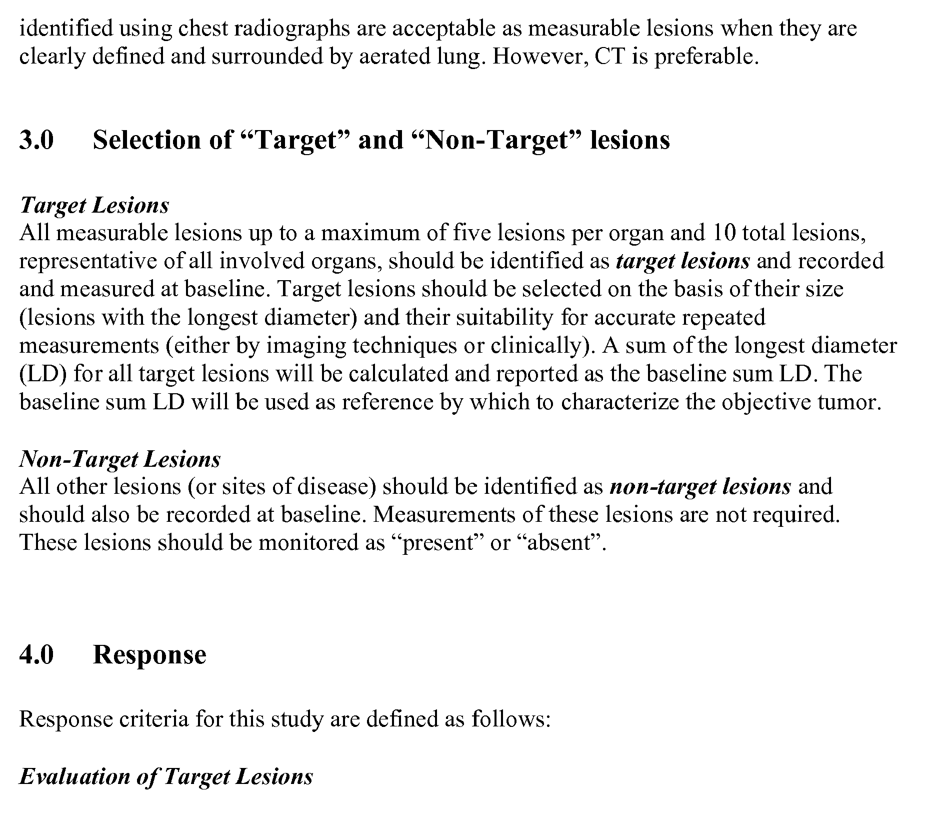

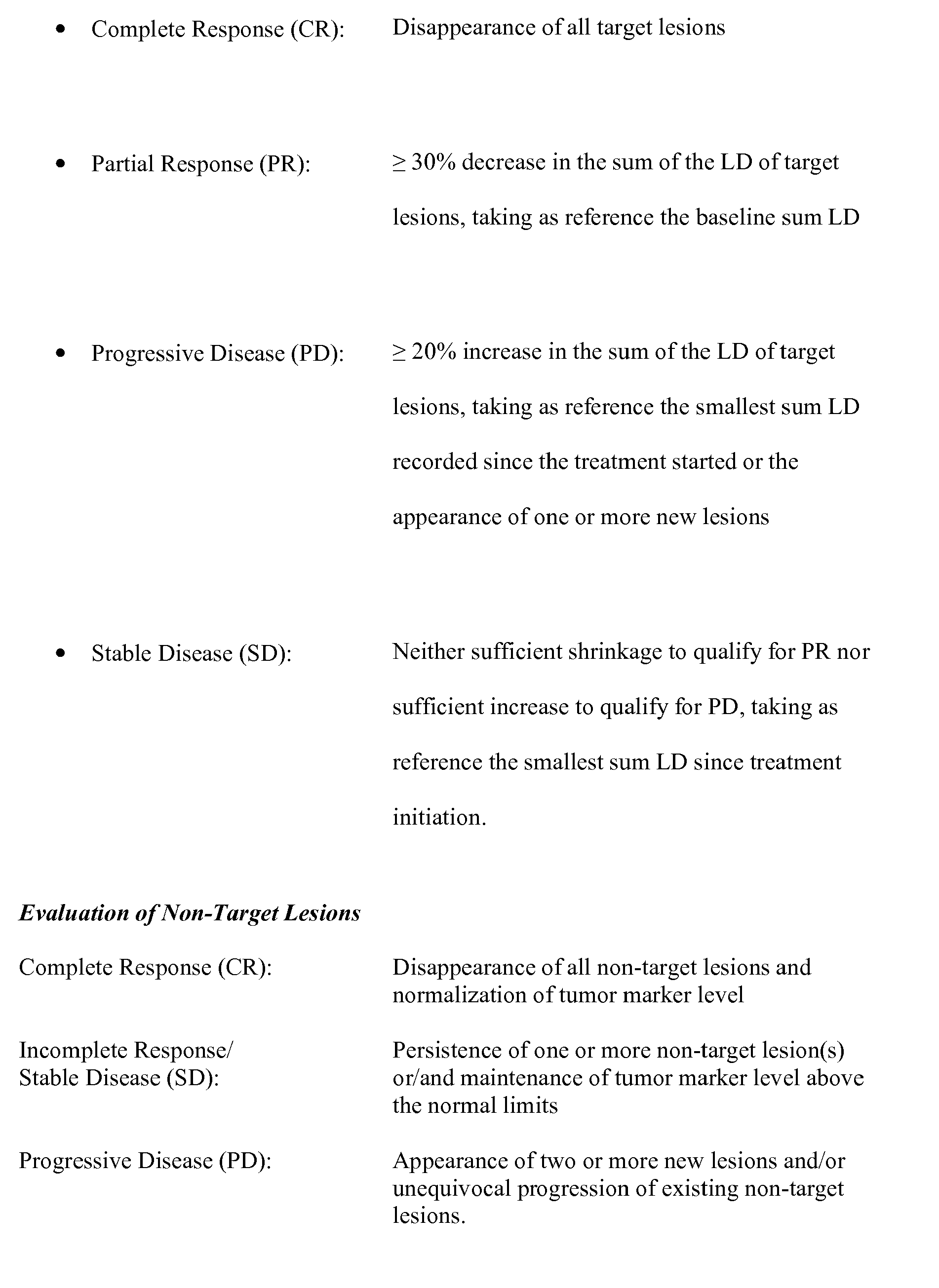

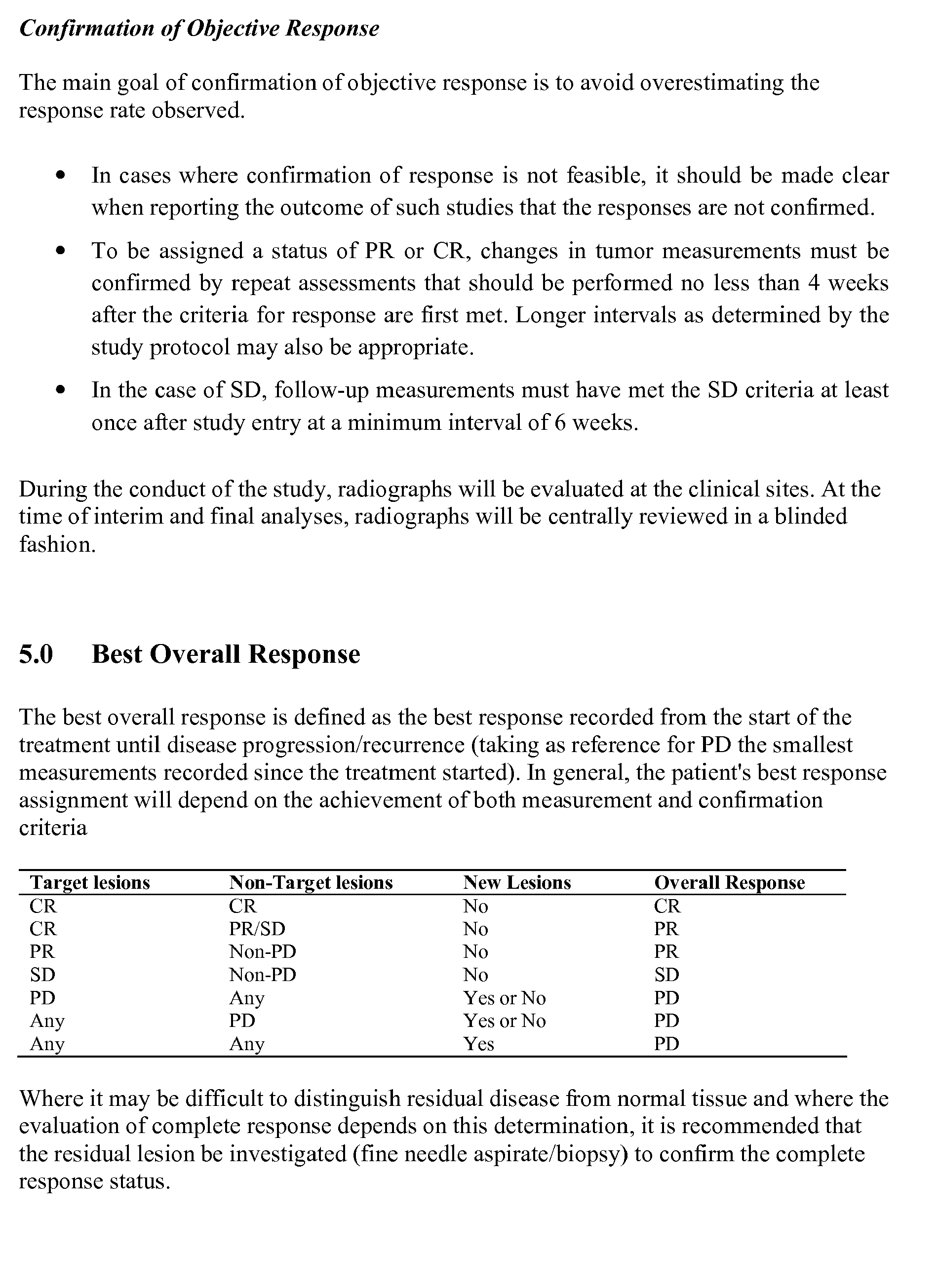

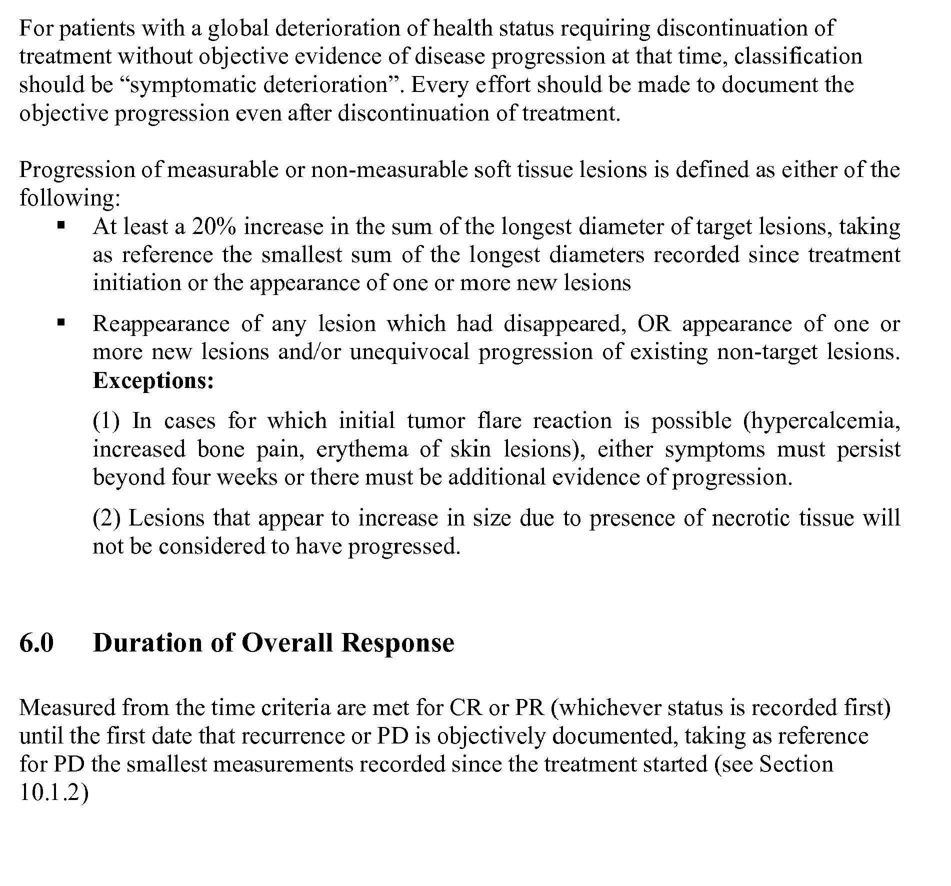

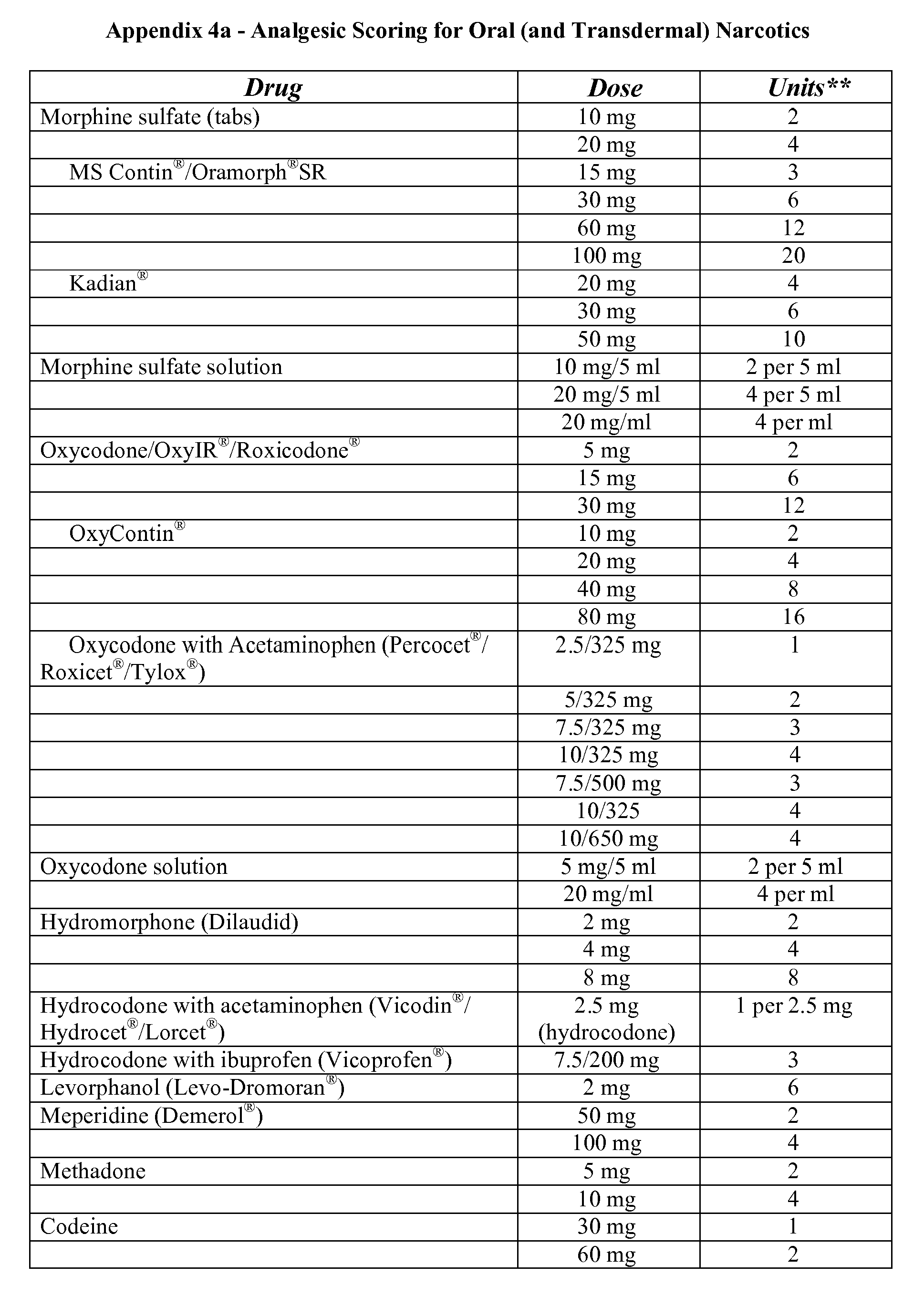

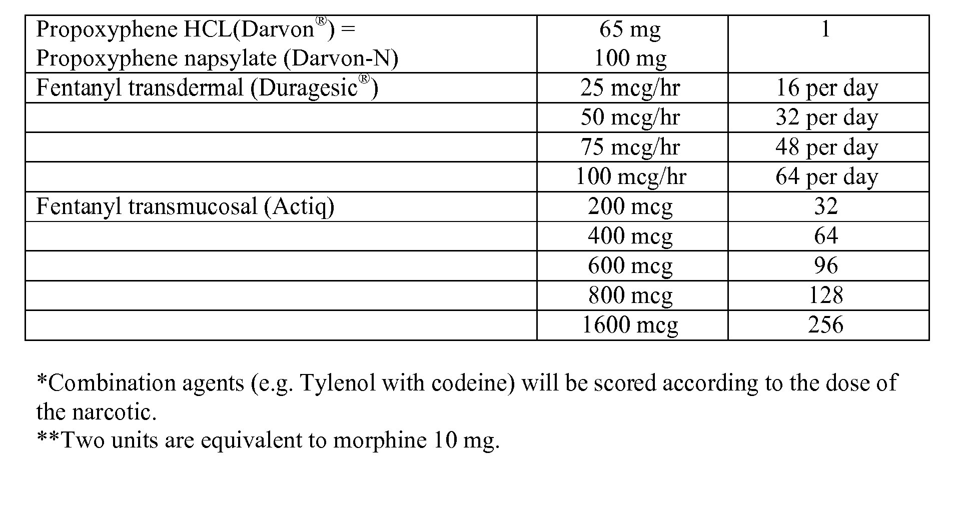

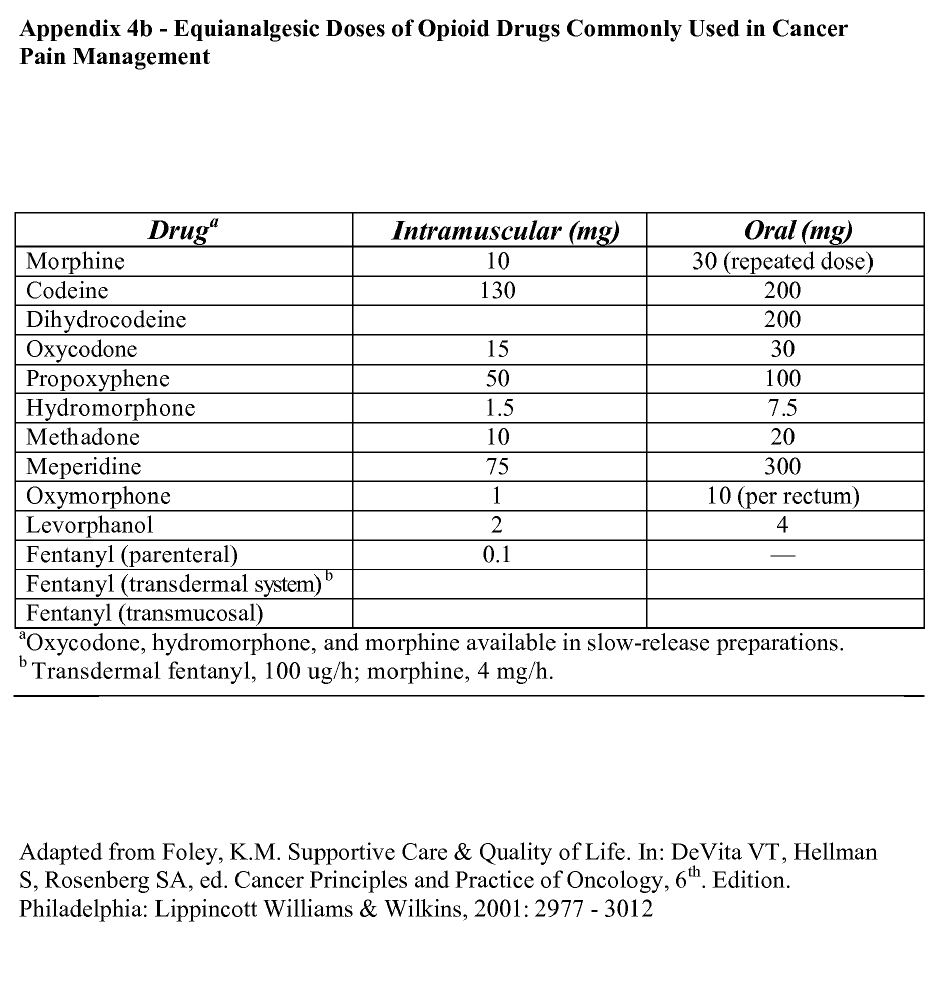

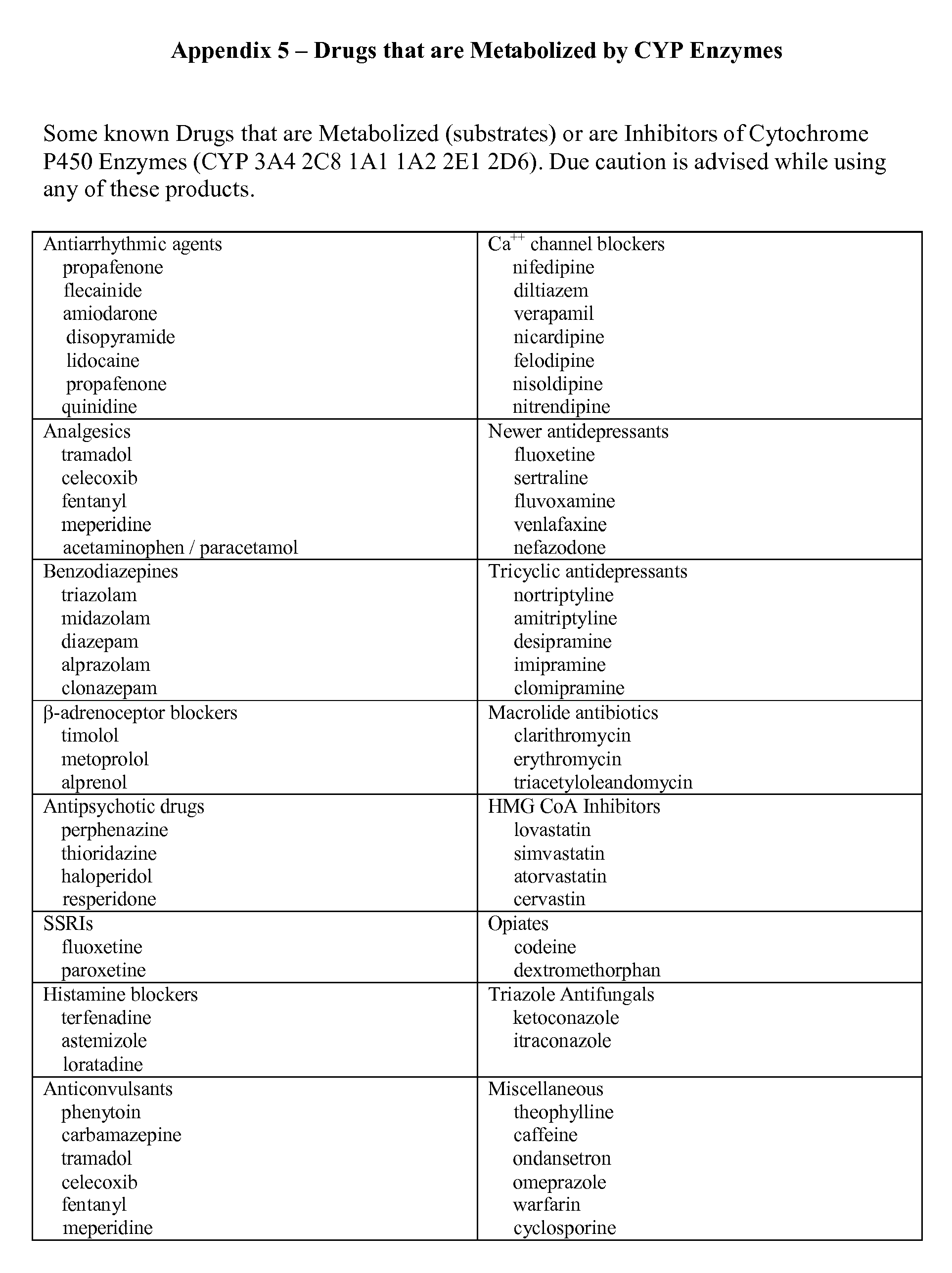

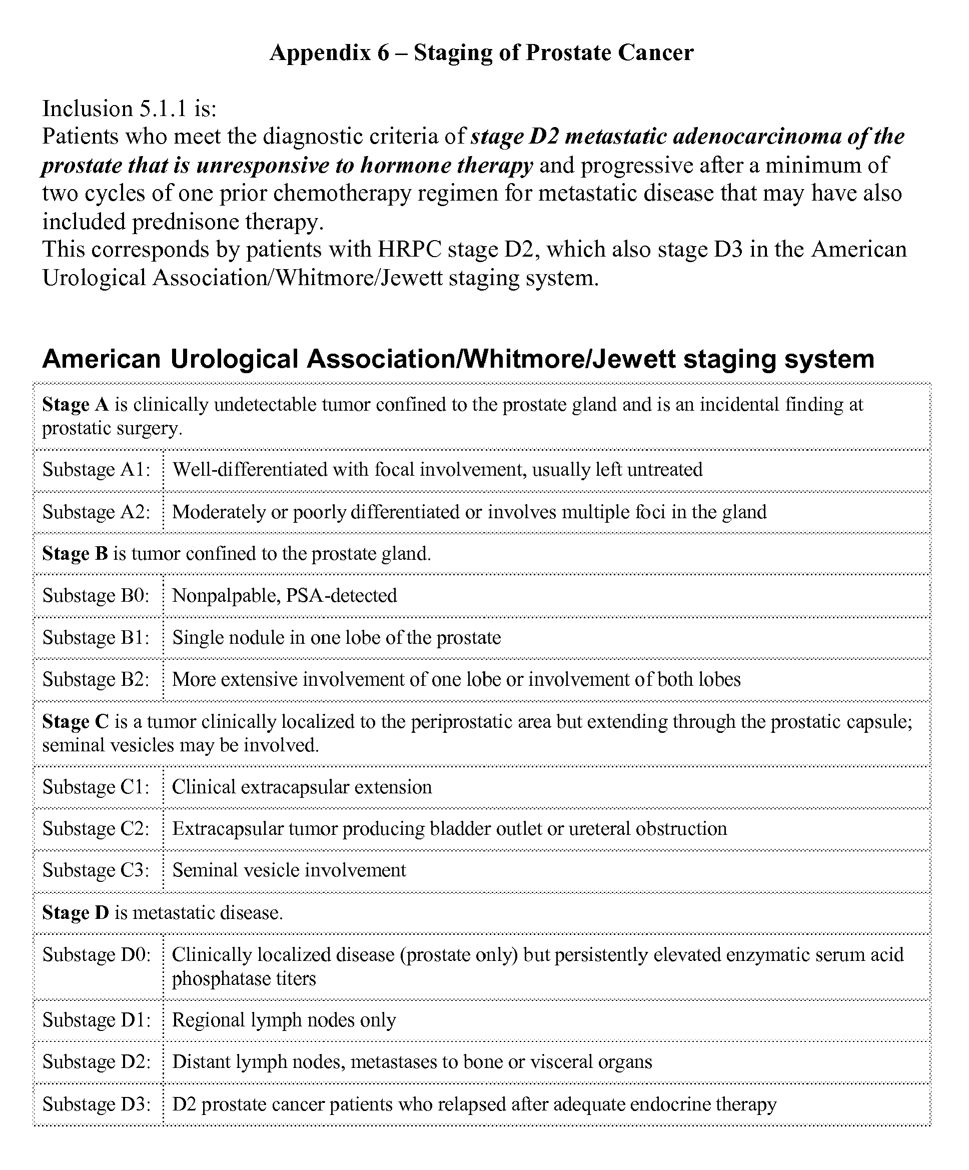

### Data supporting efficacy

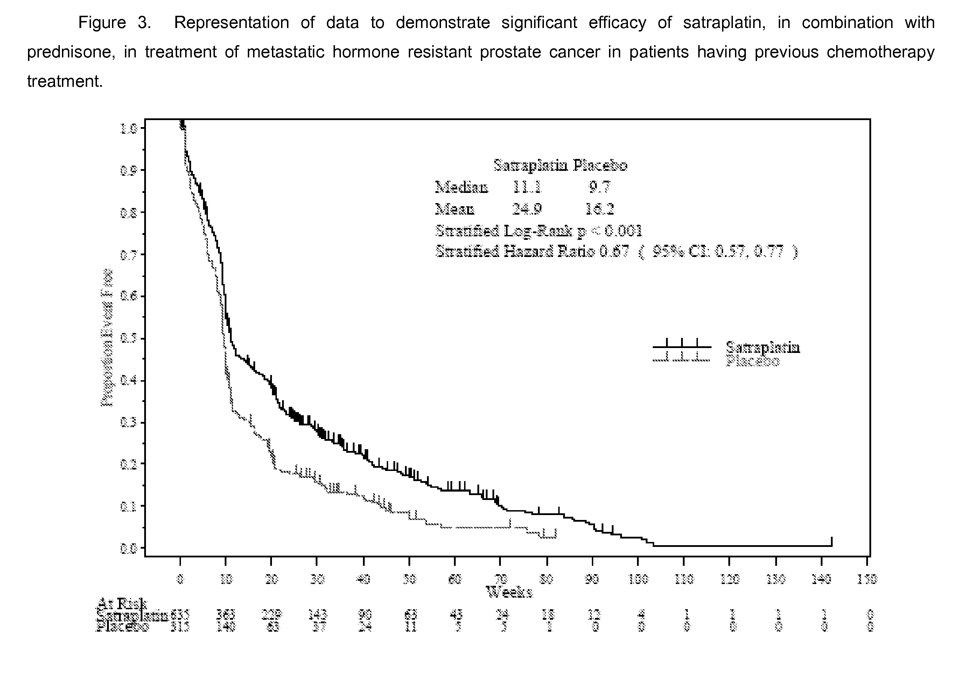

Figure 3 shows exemplary data from the SPARC trial having a statistically significant difference between and in favour of the satraplatin plus prednisone arm compared to the placebo plus prednisone arm, and the conclusions from the SPARC trial are shown in Exhibit A and Exhibit B. The majority of progression events (70% and 80% of progression event in the satraplatin plus prednisone and placebo plus prednisone arms, respectively) comprised of radiographic progression (37% vs 35%) and pain progression (56% vs 41%).

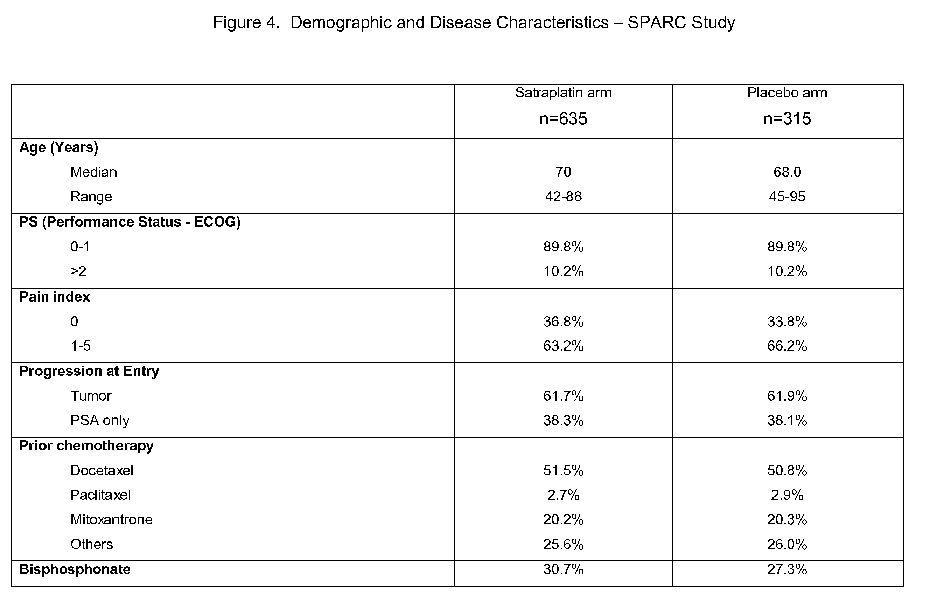

The baseline characteristics of the SPARC trail are summarized in Figure 4. The two arms show balance of the demographics shown upon entry to the SPARC trail. About half the patients received prior docetaxel (Taxotere®) chemotherapy.

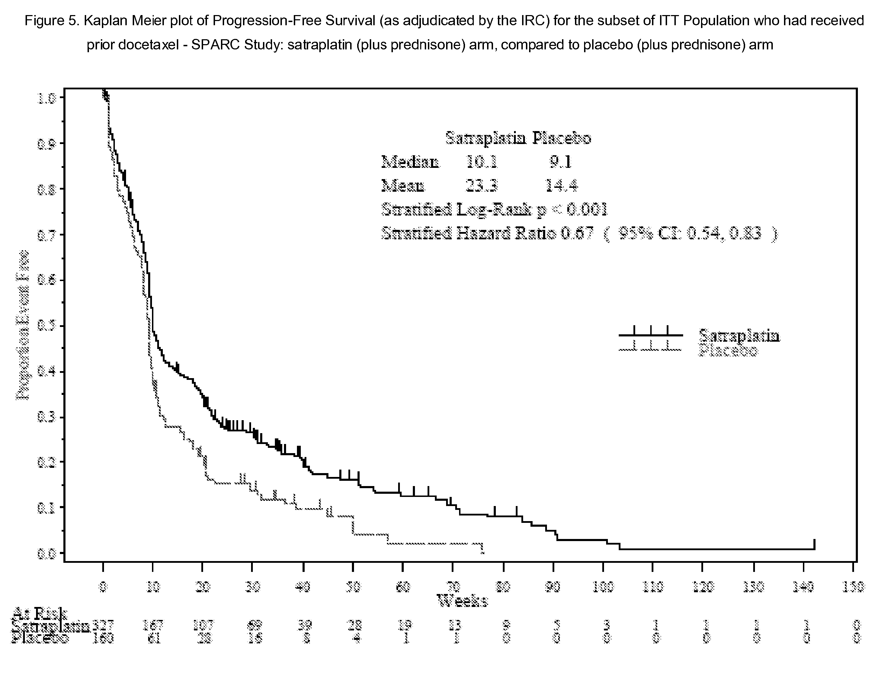

The significant benefit in PFS seen for the satraplatin (plus prednisone) arm, compared to placebo (plus prednisone) arm was maintained irrespective of whether patients had received prior docetaxel (Taxotere®) chemotherapy (Figure 5). These data support that satraplatin (plus prednisone) gives a full treatment effect in advanced (metastatic) HRPC patients who have failed prior Taxotere chemotherapy.

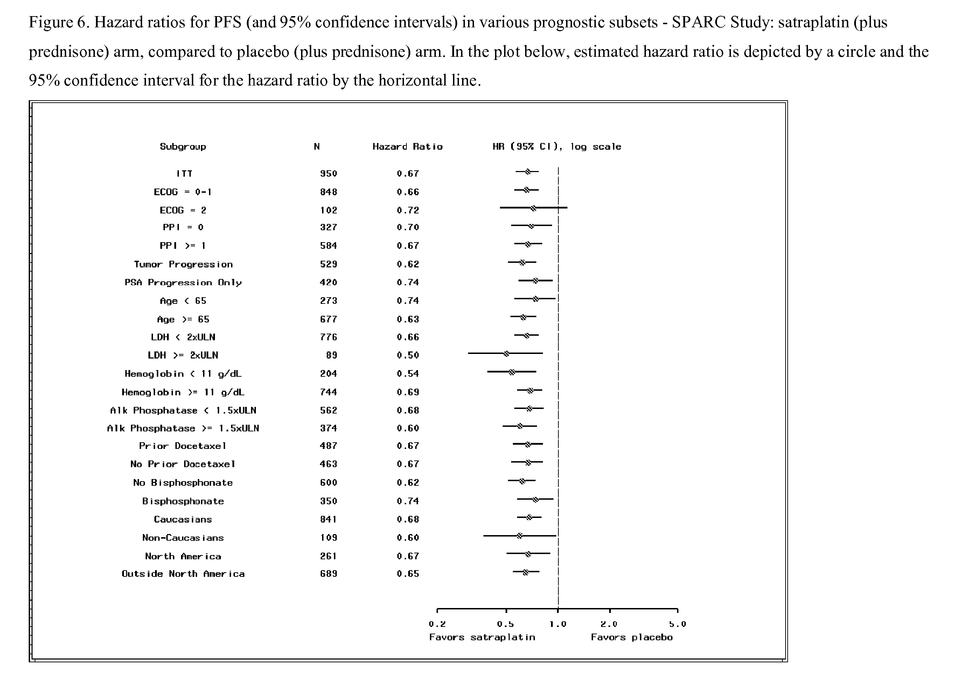

Indeed, the efficacy of the satraplatin (plus prednisone) arm is consistent across patient subsets, showing benefit (as reflected by hazard ratios - "HR" - of less than 1.0) compared to patients on the placebo (plus prednisone) arm, including for those baseline characteristics of patients: highly symptomatic at baseline (pain), PSA progression only, age, haemoglobin and alkaline phosphatase levels, prior docetaxel and biphosphonate use (Figure 6).

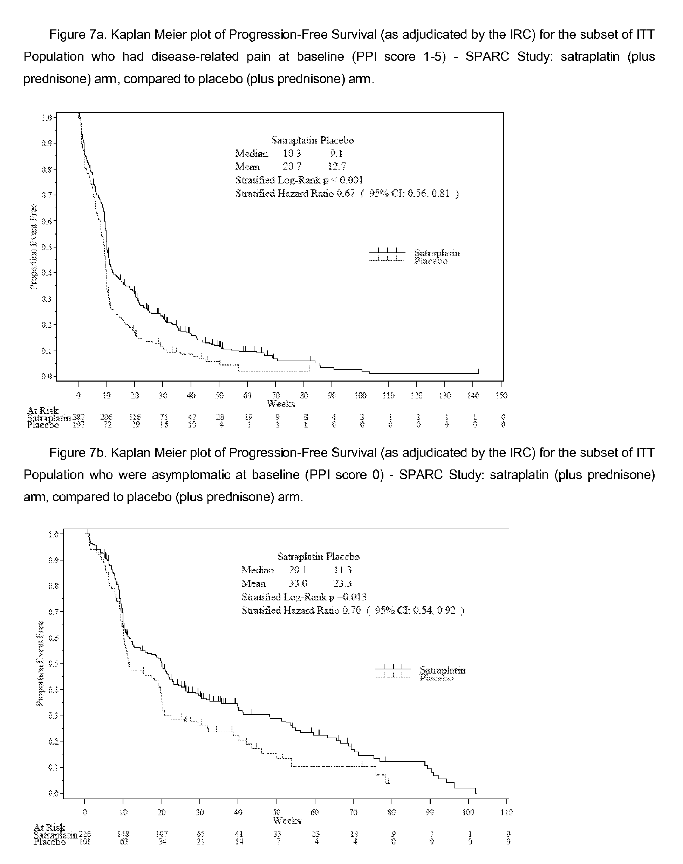

The SPARC study included both symptomatic and asymptomatic patients, as defined by baseline PPI score. For PFS, treatment effects of similar magnitude, favoring the satraplatin (plus prednisone) arm were obtained for the ITT population (HR=0.67, 95% CI: 0.57, 0.77) and the subsets of the ITT population with disease-related pain at baseline (PPI score 1-5; HR=0.67, 95% Cl: 0.56, 0.81) and no pain at baseline (PPI score 0; HR=0.70, 95% Cl: 0.54, 0.92). Kaplan Meier plots for PFS for these ITT subsets are shown in Figures 7a and 7b. Indeed, a benefit in favour of the satraplatin (plus prednisone) arm was seen in the median progression free survival for asymptomatic patients (PPI =0) compared to symptomatic patients (PPI of 1 to 5). Asymptomatic patients on the satraplatin (plus prednisone) arm showed a median PFS of 20.1 weeks compared to 11.3 weeks for patients on the placebo (plus prednisone) arm (a difference of 8.8 weeks), compared to 10.3 to 9.1 weeks for symptomatic patients (a difference of 1.2 weeks).

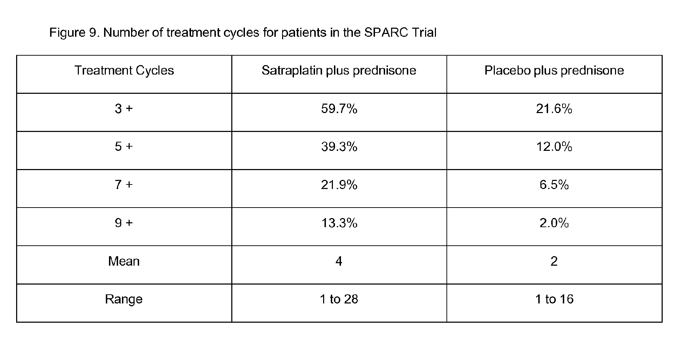

The extension of progression free survival shown by the patients on the satraplatin (plus prednisone) arm is associated with an increase in the increased number of cycles of treatment such patients were administered, compared to the number of cycles administered to patients on the placebo (plus prednisone) arm (Figure 9).

In the SPARC study, there was a 34% lower risk of pain progression for patients on the satraplatin (plus prednisone) arm compared to patients on the placebo (plus prednisone) arm (HR=0.66, 95% Cl: 0.50-0.83). The mean Time to Pain Progression (TPP) for patients on the satraplatin (plus prednisone) arm was 53.4 weeks compared to 36.6 weeks for those on the placebo (plus prednisone) arm (p<0.001). Furthermore, in patients that were symptomatic for pain at baseline, 24% of patients on the satraplatin plus prednisone arm experienced a Pain Response (reduction in pain) compared to 14% on the placebo plus prednisone arm (p=0.0047), with a duration of such Pain Response of 40.1 vs 24.1 weeks respectively.

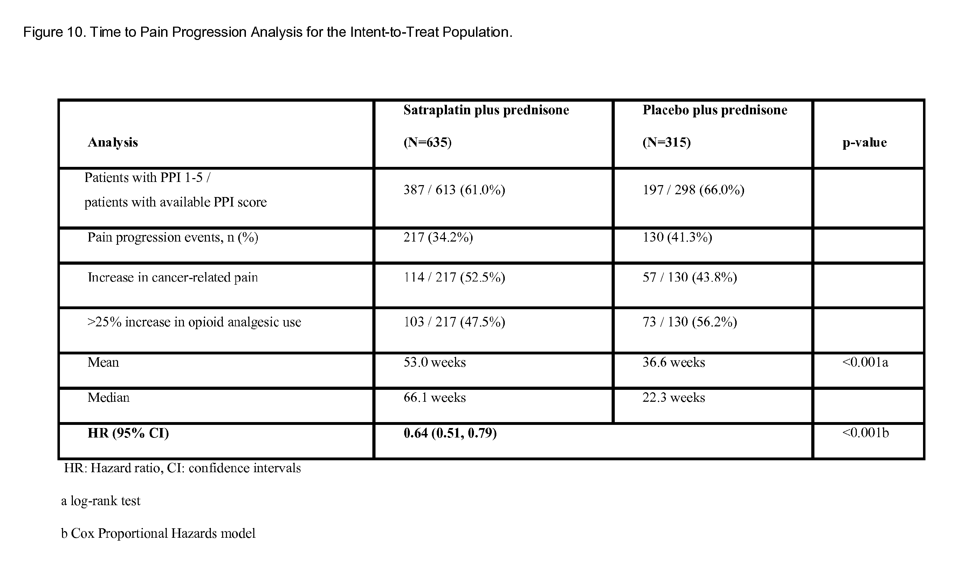

For the ITT population, Figure 10 shows additional analyses of TPP, pain progression or opioid-use data, displaying benefits in favour of the satraplatin plus prednisone arm.

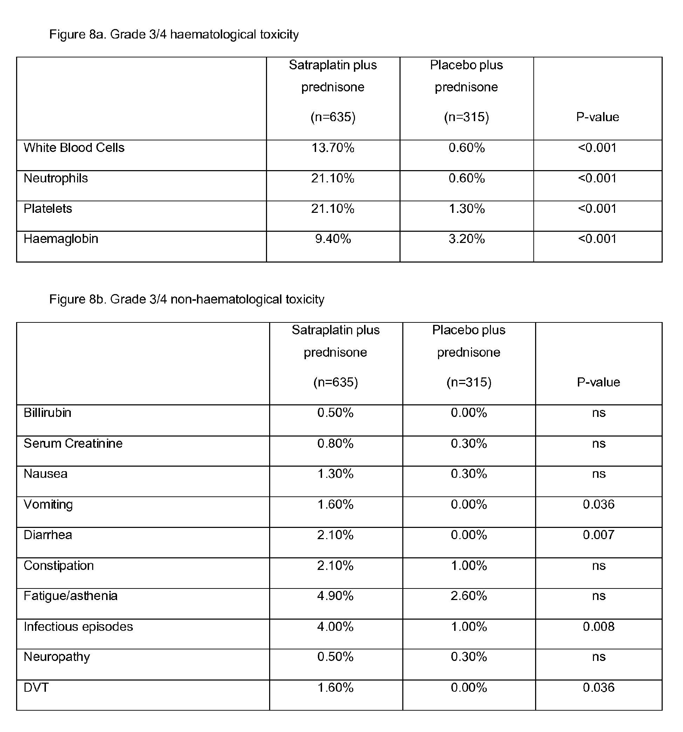

Therapy with satraplatin plus prednisone was well tolerated, with the most common treatment-related adverse events being those haemotological events associated with myelosupression (Figure 8a). Other (non-haematological Grade 3 or Grade 4 toxicities are listed in Figure 8b. Importantly, the incidence of any serious non-haematological toxicity was less than 5% of patients treated with satraplatin plus prednisone. Of note is that, unlike treatment with other platinum compounds, satraplatin plus prednisone did not show a significantly higher incidence of neuropathy and renal events compared to placebo plus prednisone.

### Additional References

Bubley GJ, Carducci M, Dahut W, et al. Eligibility and Response Guidelines for Phase II Clinical trials in Androgen-Independent Prostate Cancer: Recommendations From the Prostate-Specific Antigen Working Group. J Clin Oncol 1999;17:3461-7.

Dagher R et al Docetaxel in Combination with Prednisone for the Treatment of Androgen-Independent Hormone-Refractory Prostate Cancer. Clin Cancer Res 2004, 10: 8147-8151.

Dawson NA. Treatment of progressive metastatic prostate cancer. Oncology 1993;7:17-27.

Eisenberger MA, Simon R, O'Dwyer PJ, Wittes RE, Friedman MA. A reevaluation of non-hormonal cytotoxic chemotherapy in the treatment of prostate cancer. J Clin Oncol 1985;3:827-41.

Eisenberger MA, Nelson WG. How much can we rely on the level of prostate-specific antigen as an end point for evaluation of clinical trials? A word of caution. J Nat Can Inst 1996;88(12):779-81.

Eisenberger M, De Wit R, Berry W, et al. A multicenter phase III comparison of docetaxel (D) + prednisone and mitoxantrone (MTZ) + P in patients with hormone-refractory prostate cancer (HRPC). Proc Am Soc Clin Oncol 2004;23:abstract #4.

Hudes G, Nathan F, Khater C, et al. Phase II trial of 96-hour paclitaxel plus oral estramustine phosphate in metastatic hormone-refractory prostate cancer. J Clin Oncol 1997;15:3156-63.

Hussain M, Petrylak D, Fisher E, Tangen C, Crawford D. Docetaxel (Taxotere) and estramustine versus mitoxantrone and prednisone for hormone-refractory prostate cancer: scientific basis and design of Southwest Oncology Group Study 9916. Semin Oncol 1999;26 (5Suppl 17):55-60.

Kelly W, Slovin S. Chemotherapy for Androgen-independent Prostate Cancer: Myth or Reality. Current Oncol Rep 2000;2:394-401.

Loehrer PJ, Einhorn LH. Cisplatin. Ann Int Med 1984;100:704-13.

Merrin CE. Treatment of genitourinary tumors with cis-dichloroammineplatinum (II): experience in 250 patients. Cancer Treat Rep 1979;63:1579-84.

Miller Jl, Ahmann FR, Drach GW, Emerson SS, Bottaccini MR. The clinical usefulness of serum prostate specific antigen after hormonal therapy of metastatic prostate cancer. J Urol 1992;147:956-61.

Moore MR, B TM, deSimone P, Birch R, Irwin L. Phase II evaluation of weekly cisplatin in metastatic hormone-resistant prostate cancer: A Southeastern Cancer Study Group trial. Cancer Treat Rep 1986;70:541-2.

Petrylak DP, Macarthur R, O'Connor J, et al. Phase I/II studies of docetaxel (Taxotere) combined with estramustine in men with hormone-refractory prostate cancer. Semin Oncol 1999;26(5 Suppl 17):28-33.

Petrylak D, Tangen C, Hussain M, et al. SWOG 99-16: Randomized phase III trial of docetaxel (D)/estramustine (E) versus mitoxantrone (M)/prednisone(p) in men with androgen-independent prostate cancer (AIPCA). Proc Am Soc Clin Oncol 2004;23:abstract #3.

Pienta KJ, Redman B, Hussain M, et al. Phase II evaluation of oral estramustine and oral etoposide in hormone-refractory adenocarcinoma of the prostate. J Clin Oncol 1994;12:2005-21.

Prestayko A, D'Aoust J, Issell B, Crooke S. Cisplatin (cis-diaminedichloroplatinum II). Cancer Treat Rev 1979;6:17-39.

Qazi R, Khandekar J-. Phase II study of cisplatin for metastatic prostatic cancer. Am J Oncol 1983;6:203-5.

Raynaud et al. 1996 Cancer Chemother Phamacol 38: 155-162

Rossof AH, Talley RW, Stephens R, et al. Phase II evaluation of cisdichlorodiammineplatinum (II) in advanced malignancies of the genitourinary and gynecologic organs: a Southwest Oncology Group study. Cancer Treat Rep 1979;63:1557-64.

Rozencweig M, Von Hoff DD, Slavik M, Muggia FM. Cis-diaminedichloroplatinum (II) a new anticancer drug. Ann Int Med 1977;86:803-12.

Soloway M, S B, Brady M, et al. A comparison of estramustine phosphate versus cisplatinum alone versus estramustine phosphate plus cisplatinum in patients with advanced hormone refractory prostate cancer who had extensive irradiation to the pelvis or lumbosacral area. J Urol 1983;129:56-61.

Sternberg C. Hormone refractory metastatic prostate cancer. Ann Oncol 1992;3(5):331-5.

Tannock IF, Osoba D, Stockler MR, et al. Chemotherapy with mitoxantrone plus prednisone or prednisone alone for symptomatic hormone-resistant prostate cancer: A Canadian randomized trial with palliative end points. J Clin Oncol 1996;14(6):1756-64.

Yagoda A. Phase II trials with cis-dichlorodiammineplatinum (II) in the treatment of urothelial cancer. Cancer Treat Rep 1979 (I);63:1565-72.

Yagoda A, Watson R, Natale R. A critical analysis of response criteria in patients with prostate cancer treated with cis-diamminedichlororide platinum (II). Cancer 1979 (II);44:1553-62.

Yagoda A, Petrylak DP. Cytotoxic chemotherapy for advanced hormone-resistant prostate cancer. Cancer 1993;71 (Supplement): 1098-109.

### Equivalents

Those skilled in the art will recognize, or be able to ascertain using no more than routine experimentation, many equivalents to the specific embodiments of the invention described herein. Such equivalents are intended to be encompassed by the following claims.

All of the above-cited references and publications are hereby incorporated by reference.

### Exhibit A. Public disclosure of results from SPARC trial.

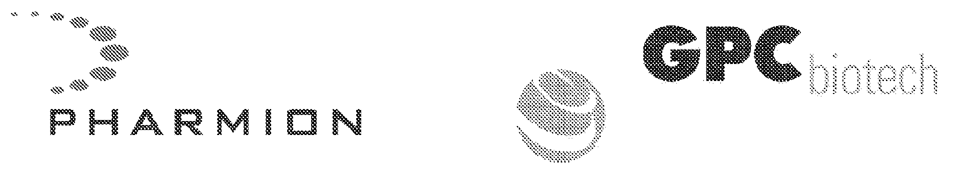

### Press Release

### FOR IMMEDIATE RELEASE

### GPC Biotech and Pharmion Announce Positive Results from the Satraplatin Pivotal Phase 3 Trial and Achievement of the Progression-Free Survival Endpoint

- **40% Reduction in Risk of Disease Progression Seen with Satraplatin Compared to Control**
- **Highly Statistically Significant Results Seen for Progression-Free Survival (p<0.00001)**
- **U.S. Regulatory Submission Expected by Year-End 2006, European Filing in H1 2007**

**Martinsried/Munich (Germany), Waltham, Mass., Princeton, N.J. and Boulder, Colo., September 24, 2006** - GPC Biotech AG (Frankfurt Stock Exchange: GPC; TecDAX index; NASDAQ: GPCB) and Pharmion Corporation (NASDAQ: PHRM) today announced positive topline results for the double-blinded, randomized satraplatin Phase 3 registrational trial, the SPARC trial (Satraplatin and Prednisone Against Refractory Cancer). The trial is evaluating satraplatin plus prednisone versus placebo plus prednisone as a second-line treatment in 950 patients with hormone-refractory prostate cancer (HRPC). The study data show that the results for progression-free survival (PFS) are highly statistically significant (p<0.00001) using the protocol-specified log-rank test. PFS is the primary endpoint for submission for accelerated approval in the U.S. and will also serve as the primary basis for a Marketing Authorization Application (MAA) in Europe.

Using the protocol-specified hazard ratio, which measured the overall risk of disease progression, patients in the SPARC trial who received satraplatin plus prednisone had a 40% reduction in the risk of disease progression (hazard ratio of 0.6; 95% Confidence Interval: 0.5-0.7) compared with patients who received prednisone plus placebo. The improvement seen in progression-free survival by patients treated with satraplatin increased over time. Progression-free survival at the median (50th percentile) demonstrated a 13% improvement in patients who received satraplatin plus prednisone (11 weeks) compared to patients who received prednisone plus placebo (9.7 weeks). Progression-free survival at the 75th percentile showed an 89% improvement for patients in the satraplatin arm (36 weeks) versus patients in the placebo arm (19 weeks). At 6 months, 30% of patients in the satraplatin arm had not progressed, compared to 17% of patients in the control arm. At 12 months, 16% of patients who received satraplatin had not progressed, compared to 7% of patients in the control arm. All of these analyses were conducted on an intent-to-treat basis.

The improvement in PFS in the satraplatin arm was not affected by the type of prior chemotherapy; in particular, the improvement was seen equally for patients who had received prior Taxotere® (docetaxel), as well as those who received other types of chemotherapy treatments. All disease progression events were adjudicated by an independent expert review committee of medical oncologists and radiologists. The vast majority of progression events were based on radiological progressions and pain progressions.

In accordance with the recommendation of the independent Data Monitoring Board for the SPARC trial, patients who have not progressed will continue to be treated, and all patients will be followed for overall survival. With approximately half of the patients from the trial still alive, the companies currently expect to have final overall survival results in the fall of 2007, rather than the previously communicated mid-2007.

As anticipated, the most common adverse reactions consisted of myelosuppression (bone marrow functions, such as lowered platelet count or lowered white blood cell count) and gastrointestinal events, such as nausea, vomiting and diarrhea. These adverse reactions were mostly mild to moderate in severity.

"Patients with advanced hormone-refractory prostate cancer are in urgent need of new treatment options. We are excited about the highly statistically significant improvement in progression-free survival demonstrated by satraplatin in our Phase 3 registrational trial. Importantly, the difference in progression-free survival increases over time in favor of the satraplatin group," said Bernd R. Seizinger, M.D., Ph.D., Chief Executive Officer of GPC Biotech. "Based on the positive data announced today, we will move forward as rapidly as possible with the FDA with the goal of completing the filing for marketing approval of satraplatin in the U.S. before the end of this year."

Dr. Seizinger continued: "Today's results are indeed great news for GPC Biotech and our partners. We look forward to an ongoing productive relationship with Pharmion as they advance satraplatin in Europe. We are delighted that the vision we shared with Spectrum Pharmaceuticals for satraplatin at the time we licensed the compound is now becoming tangible."

"We believe that these results demonstrate the benefits that satraplatin can provide men with advanced prostate cancer. In addition, the oral administration of satraplatin offers the patient the convenience of outpatient therapy and the potential for greater dosing coordination with other cancer therapies," said Patrick J. Mahaffy, President and Chief Executive Officer of Pharmion Corporation. "Pharmion remains focused on preparing its marketing authorization application, which we plan to file with the European regulatory authorities in the first half of 2007. We have worked closely with GPC Biotech since licensing the European and other international rights to satraplatin last December and look forward to continuing our partnership as we move through the regulatory process towards commercialization."

The SPARC trial is a double-blinded, randomized, placebo-controlled multinational Phase 3 trial assessing satraplatin plus prednisone as a second-line chemotherapy treatment for patients with HRPC. A total of 950 patients were accrued to the trial at more than 200 clinical sites in fifteen countries on four continents. The companies plan to submit data from the SPARC trial for presentation at an upcoming major medical conference.

GPC Biotech intends to move forward with the U.S. Food and Drug Administration (FDA) with the goal of completing the submission of the rolling New Drug Application (NDA) by the end of 2006 for approval to market satraplatin. Pharmion Corporation intends to file an MAA to the European Medicines Agency (EMEA) in the first half of 2007.

### About Prostate Cancer

Prostate cancer is the most common cancer among men in the U.S. and Europe. Approximately 234,000 men in the U.S. are expected to be diagnosed with the disease in 2006 and over 27,000 men are expected to die from the disease. In the European Union, over 200,000 new cases are expected to be diagnosed, and over 60,000 patients are expected to die each year. Since the incidence of prostate cancer increases with age, the aging of the overall population is expected to further increase the number of prostate cancer patients.

Most patients diagnosed with prostate cancer initially receive surgery or radiation therapy, and some of these patients are cured. For many others, though, the disease recurs. At this point, the recurrent disease is treated with hormone therapy, and most patients initially respond well to this treatment. Eventually, however, the tumor cells become resistant to the hormones - or "hormone-refractory" - and the tumor again progresses. Increasingly, chemotherapy is being used as an effective first-line treatment for HRPC. However, it is not a cure, and so this is creating a need for effective therapeutic options for these patients once they have progressed.

### About Satraplatin

Satraplatin, an investigational drug, is a member of the platinum family of compounds. Over the past two decades, platinum-based drugs have become a critical part of modern chemotherapy treatments and are used to treat a wide variety of cancers. Unlike the platinum drugs currently on the market, all of which require intravenous administration, satraplatin is an orally bioavailable compound and is given as capsules that patients can take at home.

In December 2005, GPC Biotech signed a co-development and license agreement with Pharmion GmbH, a wholly owned subsidiary of Pharmion Corporation, under which Pharmion was granted exclusive commercialization rights to satraplatin for Europe and certain other territories. GPC Biotech licensed satraplatin from Spectrum Pharmaceuticals, Inc. (NASDAQ: SPPI) in 2002.

GPC Biotech has submitted two of three sections of a rolling submission of an NDA with the U.S. FDA for satraplatin as a second-line chemotherapy treatment for HRPC and expects to complete this filing by the end of 2006. Pharmion plans to file an MAA for Europe in the first half of 2007. In addition, the companies have ongoing a broad oncology development program for satraplatin that includes a number of trials evaluating this compound in combination with radiation therapy, in combination with other cancer therapies and in various other cancers.

### Conference Calls Scheduled

Separate conference calls are planned for GPC Biotech at 8:00 a.m. and for Pharmion Corporation at 8:30 a.m. (both times Eastern), to which participants may listen via live webcast, accessible through each company's Web site at www.pharmion.com, www.gpc-biotech.com, or via telephone. Details, including dial-in numbers, will be provided in separate announcements.

### About Pharmion

Pharmion is a biopharmaceutical company focused on acquiring, developing and commercializing innovative products for the treatment of hematology and oncology patients in the U.S., Europe and additional international markets. Pharmion has a number of products on the market including the world's first approved epigenetic cancer drug, Vidaza®, a DNA demethylating agent. For additional information about Pharmion, please visit Pharmion's website at www.pharmion.com.

### About GPC Biotech

GPC Biotech AG is a biopharmaceutical company discovering and developing new anticancer drugs. GPC Biotech's lead product candidate - satraplatin - has achieved target enrollment in a Phase 3 registrational trial as a second-line chemotherapy treatment in hormone-refractory prostate cancer. The U.S. FDA has granted fast track designation to satraplatin for this indication, and GPC Biotech has begun the rolling NDA submission process for this compound. GPC Biotech is also developing a monoclonal antibody with a novel mechanism-of-action against a variety of lymphoid tumors, currently in Phase 1 clinical development, and has ongoing drug development and discovery programs that leverage its expertise in kinase inhibitors. GPC Biotech AG is headquartered in Martinsried/Munich (Germany), and its wholly owned U.S. subsidiary has sites in Waltham, Massachusetts and Princeton, New Jersey. For additional information, please visit GPC Biotech's Web site at www.gpc-biotech.com.

*This press release contains forward-looking statements, which express the current beliefs and expectations of the management of GPC Biotech AG and Pharmion Corporation, including summary statements relating to top line results of the SPARC trial and summary statements relating to the potential efficacy and safety profile of satraplatin. Such statements are based on current expectations and are subject to risks and uncertainties, many of which are beyond our control, that could cause future results, performance or achievements to differ significantly from the results, performance or achievements expressed or implied by such forward-looking statements. Actual results could differ materially depending on a number of factors, and we caution investors not to place undue reliance on the forward-looking statements contained in this press release. In particular, there can be no guarantee that topline results from the clinical trial discussed in this press release will be confirmed upon full analysis of the results of the trial and additional information relating to the safety, efficacy or tolerability of satraplatin may be discovered upon further analysis of data from the SPARC trial or analysis of additional data from other ongoing clinical trials for satraplatin. Furthermore, even if these topline results are confirmed upon full analysis of the trial, we cannot guarantee that satraplatin will be approved for marketing in a timely manner, if at all, by regulatory authorities nor that, if marketed, satraplatin will be a successful commercial product. We direct you to GPC Biotech's Annual Report on Form 20-F for the fiscal year ended December 31, 2005, Pharmion's Annual Report on Form 10-K for the fiscal year ended December 31, 2005, its most recent filings on Form 10-Q and other reports filed with the U.S. Securities and Exchange Commission for additional details on the important factors that may affect the future results, performance and achievements of either Pharmion or GPC Biotech. Forward-looking statements speak only as of the date on which they are made and neither Pharmion nor GPC Biotech undertakes any obligation to update these forward-looking statements, even if new information becomes available in the future.*

*The scientific information discussed in this press release related to satraplatin is preliminary and investigative. Satraplatin has not yet been approved by the FDA in the U.S., the EMEA in Europe or any other regulatory authority and no conclusions can or should be drawn regarding its safety or effectiveness. Only the relevant regulatory authorities can determine whether satraplatin is safe and effective for the use(s) being investigated.*

*Taxotere® (docetaxel) is a registered trademark of Aventis Pharma S.A.*

***Editor's Note:** A virtual press kit is available for downloading from the News and Media section of the GPC Biotech website at http:llwww.gpc-biotech.com*/*en*/*news_media*/*virtual_press_kit*/*index.html or by contacting one of the GPC Biotech individuals listed below.*

*For further information, please contact:*
**GPC Biotech AG**
Martin Braendle
Director, Investor Relations & Corporate Communications
Phone: +49 (0)89 8565-2693
ir@gpc-biotech.com

**In the U.S.:** Laurie Doyle
Director, Investor Relations & Corporate Communications
Phone: +1 781 890 9007 X267 or +1 609 524 1000
usinvestors@gpc-biotech.com

**Pharmion Corporation**
**Breanna Burkart/Anna Sussman**
Directors, Investor Relations and Corporate Communications
Phone: 720.564.9150
ir@pharmion.com

**Additional Media Contacts for GPC Biotech:**
*In Europe:*
Maitland Noonan Russo
Brian Hudspith
Phone: +44 (0)20 7379 5151
bhudspith@maitland.co.uk

*In the U.S.:*
Noonan Russo
David Schull
Phone:+1 858546-4810
david.schull@eurorscg.com

**Additional Media Contacts for Pharmion:**
*In the U.S.:*
Manning Selvage & Lee
Kelly McKenna
Phone: +1 415 293 2808
kelly.mckenna@mslpr.com

*In Europe:*
ACH & Asociados
Almudena Aguado
Phone:+34 (0)91 745 48 00
almudena.aguado@ach.es

### Exhibit B. Public disclosure II of results from SPARC trial.

### Press Release

### FOR IMMEDIATE RELEASE

### Satraplatin Shown to Significantly Reduce Risk of Disease Progression in Advanced Hormone-Refractory Prostate Cancer Patients

- **Highly Statistically Significant Results for Improvement in Progression-Free Survival**
- **Progression-Free Survival Results Consistent Irrespective of Prior Chemotherapy Treatment, including Taxotere^{®}**
- **Satraplatin was Well Tolerated with Myelosuppression the Most Commonly Observed Toxicity**
- **Data Being Presented Today at ASCO Prostate Cancer Symposium in Orlando, Florida**
- **GPC Biotech Conference Call Scheduled Today at 9:00 AM EST**
- **Pharmion Investor Event Scheduled Today at 6:30 PM EST**

**Martinsried/Munich (Germany), Waltham, Mass., Princeton, N.J. and Boulder, Colo., February 23, 2007 -** GPC Biotech AG (Frankfurt Stock Exchange: GPC; TecDAX index; NASDAQ: GPCB) and Pharmion Corporation (NASDAQ: PHRM) today announced that final progression-free survival (PFS) results for the double-blind, randomized satraplatin Phase 3 registrational trial, the SPARC trial (Satraplatin and Prednisone Against Refractory Cancer) are being presented today at the ASCO Prostate Cancer Symposium in Orlando, Florida. The trial is evaluating satraplatin plus prednisone versus placebo plus prednisone in 950 patients with hormone-refractory prostate cancer (HRPC) who have failed prior chemotherapy. All analyses of PFS being presented were conducted on an intent-to-treat basis.

The study data show that satraplatin significantly reduces the risk of disease progression in these patients using the protocol-specified log-rank test. The hazard ratio of 0.6 (95% Cl: 0.5-0.7, p<0.00001), which was first reported in September 2006, adjusted for nine pre-specified prognostic factors. Using a more conservative analysis, which adjusted only for the three pre-specified stratification factors, the hazard ratio is 0.67 (95% Cl: 0.57-0.77, p=0.0000003). These hazard ratio numbers correspond to a reduction in relative risk of disease progression of 40% and 33%, respectively. Both analyses are being presented today in Orlando.

In accordance with the recommendation of the independent Data Monitoring Board for the SPARC trial, patients who have not progressed continue to be treated and all patients will be followed for overall survival. Overall survival data are expected to be available later this year. GPC Biotech recently completed the New Drug Application (NDA) submission for satraplatin to the U.S. Food and Drug Administration (FDA). Pharmion expects to complete the Marketing Authorization Application (MAA) for Europe in the second quarter of 2007.

Daniel Petrylak, M.D., Associate Professor of Medicine at Columbia University College of Physicians & Surgeons, Director of the Genitourinary Oncology Program at New York-Presbyterian Hospital/Columbia, and a Principal Investigator in the SPARC trial, said: "As there are currently no approved therapies for patients with hormone-refractory prostate cancer whose disease has already failed on one chemotherapy regimen, satraplatin has the potential to address a mounting area of unmet medical need. The data I am presenting today show statistically significant results in progression-free survival in favor of those patients treated with satraplatin. These results are consistent no matter what the prior chemotherapy treatment, including Taxotere^{®}."

All disease progression events were adjudicated by an independent expert review committee of medical oncologists and radiologists. The vast majority of progression events were based on radiological progressions and pain progressions. Pain associated with bone metastases is the dominant cause of morbidity in patients with metastatic HRPC. Increase in prostate specific antigen (PSA) was not part of the progression endpoint. PFS at the median demonstrated a 14% improvement in patients who received satraplatin plus prednisone (11.1 weeks) compared to patients who received prednisone plus placebo (9.7 weeks). The improvement seen in PFS by patients treated with satraplatin increased over time. PFS at the 75th percentile showed an 81% improvement for patients in the satraplatin arm (34.6 weeks) versus patients in the placebo arm (19.1 weeks). At six months, 30% of patients in the satraplatin arm had not progressed, compared to 17% of patients in the control arm. At twelve months, 16% of patients who received satraplatin had not progressed, compared to 7% of patients in the control arm.

The median number of cycles was four for the satraplatin group compared to two for the control group. Nearly 40% of patients treated with satraplatin received five or more cycles of treatment compared to approximately 20% of patients in the control arm.

The improvement in PFS in the satraplatin arm was not affected by the type of prior chemotherapy; importantly, the improvement was similar for patients who had received prior Taxotere® (docetaxel), as well as those who received other types of chemotherapy treatments. Fifty-one percent of patients in the trial were previously treated with Taxotere. The hazard ratio for patients in the SPARC trial who were previously treated with Taxotere was 0.67 (95% CI: 0.54-0.83; p=0.0006, adjusted for the pre-specified stratification factors) and therefore numerically equivalent to the entire study population.

Safety findings were consistent with previous clinical studies involving satraplatin. The reported adverse reactions were mostly mild to moderate in severity. The most common adverse reactions consisted of myelosuppression (bone marrow functions): Twenty-one percent of patients in the satraplatin arm experienced grade 3 or 4 thrombocytopenia; 14 percent had leucopenia and 21 percent had neutropenia. Eight percent of patients in the satraplatin arm experienced grade 3 or 4 gastrointestinal toxicities, including nausea, vomiting, diarrhea and constipation. Five percent or less of patients in the satraplatin arm experienced grade 3 or 4 fatigue, grade 3 or 4 infections and pulmonary/respiratory grade 3 or 4 toxicities.

"We are delighted with the strong detailed results presented today from the satraplatin SPARC Phase 3 trial," said Bernd R. Seizinger, M.D., Ph.D., Chief Executive Officer of GPC Biotech. "Moving forward, we plan to work closely with the FDA regarding our application for marketing approval of satraplatin in the U.S. We also are continuing to aggressively build our marketing and sales organization in the U.S. to prepare for a potential launch of satraplatin later this year."

"We have been very pleased with the response of the prostate cancer treatment community to the SPARC data and believe that satraplatin will become a very important treatment option for men with HRPC," said Patrick J. Mahaffy, President and Chief Executive Officer of Pharmion Corporation. "We are currently preparing our regulatory submission and expect to file a marketing authorization application for satraplatin in the EU by the end of the second quarter."

The SPARC trial is a double-blinded, randomized, placebo-controlled multinational Phase 3 trial assessing satraplatin plus prednisone as a second-line chemotherapy treatment for patients with HRPC. A total of 950 patients were accrued to the trial at approximately 170 clinical sites in sixteen countries on four continents. In addition to the results presented today, the companies expect that more data from the SPARC trial will be presented at future upcoming major medical conferences.

### GPC Biotech and Pharmion investor Events

GPC Biotech will hold a conference call today at 9:00 A.M. EST/15:00 CET to which participants may listen via live webcast, accessible through the Company's Web site at www.gpc-biotech.com, or via telephone. Dr. Daniel Petrylak will participate in the call, in addition to corporate management. The dial-in numbers for the call are as follows:
- Participants from Europe:: 0049 (0)69 2222 2246 or
0044 (0)20 7138 0835
- Participants from the U.S.:: 1-718-354-1172

Pharmion will hold an investor event today at 6:30 P.M. EST in Emerald 4 at the Gaylord Palms Resort and Conference Center in Orlando, Florida. For those unable to attend, the event will be accessible via a teleconference and will be webcast live on our website at www.pharmion.com. The dial in for U.S. participants is 1.800.901.5231 and 1.617.786.2961 for International participants. The passcode is 84420838. Corporate management will briefly review today's data and then a panel of European physicians will discuss their clinical experience with satraplatin and its potential role in the treatment of HRPC in Europe.

## Claims

1. A packaged-pharmaceutical-product comprising a pharmaceutical composition that includes satraplatin, wherein said packaged-pharmaceutical-product further comprises instructions to conduct administration of a therapeutically effective amount of said satraplatin included in said pharmaceutical composition to an individual suffering from metastatic hormone refractory prostate cancer, wherein said instructions further include:
(a) an instruction to conduct said administration of satraplatin to an individual who was treated with previous chemotherapy for metastatic hormone refractory prostate cancer; and
(b) an instruction to conduct said administration of satraplatin in combination with administration of a corticosteroid.

2. The packaged-pharmaceutical-product of claim 1, further comprising a second pharmaceutical composition that includes a corticosteroid.

3. A use of satraplatin for the preparation of a pharmaceutical composition including satraplatin for administration of a therapeutically effective amount of satraplatin to an individual suffering from metastatic hormone refractory prostate cancer, wherein:
(a) said individual was treated with previous chemotherapy for metastatic hormone refractory prostate cancer; and
(b) said individual is administered a corticosteroid in combination with said administration of satraplatin.

4. The packaged-pharmaceutical-product or use of any one of claims 1 to 3, wherein said individual has a diagnosis of Stage D2 adenocarcinoma of the prostate that is unresponsive to hormone therapy.

5. The packaged-pharmaceutical-product or use of any one of claims 1 to 4, wherein said individual has failed treatment with said previous chemotherapy.

6. The packaged-pharmaceutical-product or use of any one of claims 1 to 4, wherein individual has taken a chemotherapy holiday from said previous chemotherapy.

7. The packaged-pharmaceutical-product or use of any one of claims 1 to 6, wherein said chemotherapy was a cytotoxic chemotherapy regime.

8. The packaged-pharmaceutical-product or use of any one of claims 1 to 4, wherein said individual has suffered disease progression or PSA progression after a minimum of two courses of one prior cytotoxic chemotherapy regime for metastatic hormone refractory prostate cancer.

9. The packaged-pharmaceutical-product or use of claim 7, wherein said chemotherapy used a compound selected from mitoxantrone, viniblastine, estramustine and a taxane.

10. The packaged-pharmaceutical-product or use of claim 8, wherein said cytotoxic chemotherapy regime used a compound selected from mitoxantrone, viniblastine, estramustine and a taxane.

11. The packaged-pharmaceutical-product or use of claim 9 or 10, wherein said chemotherapy or cytotoxic chemotherapy regime used a taxane.

12. The packaged-pharmaceutical-product or use of claim 11, wherein said taxane was paclitaxel or docetaxel.

13. The packaged-pharmaceutical-product or use of claim 12, wherein said taxane was docetaxel.

14. The packaged-pharmaceutical-product or use of any one of claims 1 to 13, wherein said individual is administered satraplatin orally at a dose of between about 30 mg/m² and about 140 mg/m² per day over between 3 and 7 days.

15. The packaged-pharmaceutical-product or use of claim 14, wherein said individual is administered satraplatin orally at a dose of between about 40 mg/m² and about 100 mg/m² per day over between 3 and 7 days.

16. The packaged-pharmaceutical-product or use of claim 15, wherein said individual is administered satraplatin orally at a dose of between about 50 mg/m² and about 90 mg/m² per day over between 3 and 7 days.

17. The packaged-pharmaceutical-product or use of claim 14, wherein said individual is administered satraplatin orally at a dose of about 40 mg/m² per day over between 3 and 7 days.

18. The packaged-pharmaceutical-product or use of claim 15, wherein said individual is administered satraplatin orally at a dose of about 60 mg/m² per day over between 3 and 7 days.

19. The packaged-pharmaceutical-product or use of claim 16, wherein said individual is administered satraplatin orally at a dose of about 80 mg/m² per day over between 3 and 7 days.

20. The packaged-pharmaceutical-product or use of any one of claims 1 to 19, wherein said individual is administered satraplatin daily for about five consecutive days, with the cycle repeated about every 35 days.

21. The packaged-pharmaceutical-product or use of any one of claims 1 to 20, wherein no food is taken by said individual for at least about one hour before, and for at least about 2 hours after said administration of satraplatin.

22. The packaged-pharmaceutical-product or use of any one of claims 1 to 21, wherein said administration of satraplatin is to said individual on an empty stomach.

23. The packaged-pharmaceutical-product or use of any one of claims 1 to 22, wherein said individual is administered a corticosteroid orally with an amount of between 2 mg and 10 mg twice per day.

24. The packaged-pharmaceutical-product or use of claim 23, wherein said individual is administered a corticosteroid orally at an amount of 5 mg twice per day.

25. The packaged-pharmaceutical-product or use of any one of claims 14 to 24, wherein said individual is administered a corticosteroid orally about one hour prior to administration of satraplatin orally and about eight hours after administration of satraplatin orally.

26. The packaged-pharmaceutical-product or use of claim 24, wherein said individual is administered a corticosteroid in the morning and the evening on those days of a cycle when satraplatin is not administered.

27. The packaged-pharmaceutical-product or use of any one of claims 1 to 26, wherein said individual is further administered an antiemetic agent on the same day as said administration of satraplatin.

28. The packaged-pharmaceutical-product or use of claim 27, wherein said antiemetic agent is administered about one hour prior to administration of satraplatin orally and about eight hours after administration of satraplatin orally.

29. The packaged-pharmaceutical-product or use of claim 27 or 28, wherein said antiemetic agent is a 5-HT3 blocker or inhibitor.

30. The packaged-pharmaceutical-product or use of claim 29, wherein said 5-HT3 blocker or inhibitor is granisetron.

31. The packaged-pharmaceutical-product or use of claim 30, wherein said granisetron is administered orally with an amount of between 0.2 mg and 5 mg.

32. The packaged-pharmaceutical-product or use of claim 31, wherein said granisetron is administered orally with an amount of 1 mg.

33. The packaged-pharmaceutical-product of any one of claims 1, and 4 to 13, wherein the instructions included in said packaged-pharmaceutical-product comprise instructions to conduct the steps of:
(a) to said individual, on each of days 1 to 5, the administration of prednisone (5 mg) and antiemetic agent (1 mg) orally, followed after about 1 hour by the administration of satraplatin orally at a dose of about 80 mg/m², followed after about 8 hours by the administration of a corticosteroid (5 mg) and antiemetic agent (1 mg) orally;
(b) to said individual, on each of days 6 to 35 the administration of a corticosteroid (5 mg) twice daily in the morning and evening; and
(c) repeating (a) and (b) at least one time.

34. The use of any one of claims 2, and 4 to 13, wherein:
(a) to said individual, on each of days 1 to 5, prednisone (5 mg) and antiemetic agent (1 mg) is administered orally, followed after about 1 hour by administration of satraplatin orally at a dose of about 80 mg/m², followed after about 8 hours by administration of a corticosteroid (5 mg) and antiemetic agent (1 mg) orally;
(b) to said individual, on each of days 6 to 35 a corticosteroid (5 mg) is administered twice daily in the morning and evening; and
(c) repeating (a) and (b) at least one time.

35. The packaged-pharmaceutical-product or use of claim 33 or 34, wherein said administration of satraplatin is to said individual on an empty stomach.

36. The packaged-pharmaceutical-product or use of any one of claims 1 to 35, wherein said administration of satraplatin results in an elongation of the time to disease progression.

37. The packaged-pharmaceutical-product or use of any one of claims 1 to 35, wherein said administration of satraplatin results in an elongation of the progression-free survival.

38. The packaged-pharmaceutical-product or use of any one of claims 1 to 35, wherein said administration of satraplatin results in an elongation of the overall survival.

39. The packaged-pharmaceutical-product or use of any one of claims 1 to 38, wherein said corticosteroid is prednisone.

## Amended claims

### Amended claims in accordance with Rule 137(2) EPC.

**1.** A packaged-pharmaceutical-product comprising a pharmaceutical composition that includes satraplatin, wherein said packaged-pharmaceutical-product further comprises instructions to conduct administration of a therapeutically effective amount of said satraplatin included in said pharmaceutical composition to an individual suffering from metastatic hormone refractory prostate cancer, wherein said instructions further include:
(a) an instruction to conduct said administration of satraplatin to an individual who was treated with previous chemotherapy for metastatic hormone refractory prostate cancer; and
(b) an instruction to conduct said administration of satraplatin in combination with administration of a corticosteroid.

**2.** The packaged-pharmaceutical-product of claim 1, further comprising a second pharmaceutical composition that includes a corticosteroid.

**3.** A use of satraplatin for the preparation of a pharmaceutical composition including satraplatin for administration of a therapeutically effective amount of satraplatin to an individual suffering from metastatic hormone refractory prostate cancer, wherein:
(a) said individual was treated with previous chemotherapy for metastatic hormone refractory prostate cancer; and
(b) said individual is administered a corticosteroid in combination with said administration of satraplatin.

**4.** The packaged-pharmaceutical-product or use of any one of claims 1 to 3, wherein said individual has a diagnosis of Stage D2 adenocarcinoma of the prostate that is unresponsive to hormone therapy.

**5.** The packaged-pharmaceutical-product or use of any one of claims 1 to 4, wherein said individual has failed treatment with said previous chemotherapy.

**6.** The packaged-pharmaceutical-product or use of any one of claims 1 to 4, wherein said individual has taken a chemotherapy holiday from said previous chemotherapy.

**7.** The packaged-pharmaceutical-product or use of any one of claims 1 to 6, wherein said chemotherapy was a cytotoxic chemotherapy regime.

**8.** The packaged-pharmaceutical-product or use of any one of claims 1 to 4, wherein said individual has suffered disease progression or PSA progression after a minimum of two courses of one prior cytotoxic chemotherapy regime for metastatic hormone refractory prostate cancer.

**9.** The packaged-pharmaceutical-product or use of claim 7, wherein said chemotherapy used a compound selected from mitoxantrone, viniblastine, estramustine and a taxane.

**10.** The packaged-pharmaceutical-product or use of claim 8, wherein said cytotoxic chemotherapy regime used a compound selected from mitoxantrone, viniblastine, estramustine and a taxane.

**11.** The packaged-pharmaceutical-product or use of claim 9 or 10, wherein said chemotherapy or cytotoxic chemotherapy regime used a taxane.

**12.** The packaged-pharmaceutical-product or use of claim 11, wherein said taxane was paclitaxel or docetaxel.

**13.** The packaged-pharmaceutical-product or use of claim 12, wherein said taxane was docetaxel.

**14.** The packaged-pharmaceutical-product or use of any one of claims 1 to 13, wherein said individual is administered satraplatin orally at a dose of between about 30 mg/m² and about 140 mg/m² per day over between 3 and 7 days.

**15.** The packaged-pharmaceutical-product or use of claim 14, wherein said individual is administered satraplatin orally at a dose of between about 40 mg/m² and about 100 mg/m² per day over between 3 and 7 days.

**16.** The packaged-pharmaceutical-product or use of claim 15, wherein said individual is administered satraplatin orally at a dose of between about 50 mg/m² and about 90 mg/m² per day over between 3 and 7 days.

**17.** The packaged-pharmaceutical-product or use of claim 14, wherein said individual is administered satraplatin orally at a dose of about 40 mg/m² per day over between 3 and 7 days.

**18.** The packaged-pharmaceutical-product or use of claim 15, wherein said individual is administered satraplatin orally at a dose of about 60 mg/m² per day over between 3 and 7 days.

**19.** The packaged-pharmaceutical-product or use of claim 16, wherein said individual is administered satraplatin orally at a dose of about 80 mg/m² per day over between 3 and 7 days.

**20.** The packaged-pharmaceutical-product or use of any one of claims 1 to 19, wherein said individual is administered satraplatin daily for about five consecutive days, with the cycle repeated about every 35 days.

**21.** The packaged-pharmaceutical-product or use of any one of claims 1 to 20, wherein no food is taken by said individual for at least about one hour before, and for at least about 2 hours after said administration of satraplatin.

**22.** The packaged-pharmaceutical-product or use of any one of claims 1 to 21, wherein said administration of satraplatin is to said individual on an empty stomach.

**23.** The packaged-pharmaceutical-product or use of any one of claims 1 to 22, wherein said individual is administered a corticosteroid orally with an amount of between 2 mg and 10 mg twice per day.

**24.** The packaged-pharmaceutical-product or use of claim 23, wherein said individual is administered a corticosteroid orally at an amount of 5 mg twice per day.

**25.** The packaged-pharmaceutical-product or use of any one of claims 14 to 14, wherein said individual is administered a corticosteroid orally about one hour prior to administration of satraplatin orally and about eight hours after administration of satraplatin orally.

**26.** The packaged-pharmaceutical-product or use of claim 24, wherein said individual is administered a corticosteroid,in the morning and the evening on those days of a cycle when satraplatin is not administered.

**27.** The packaged-pharmaceutical-product or use of any one of claims 1 to 26, wherein said individual is further administered an antiemetic agent on the same day as said administration of satraplatin.

**28.** The packaged-pharmaceutical-product or use of claim 27, wherein said antiemetic agent is administered about one hour prior to administration of satraplatin orally and about eight hours after administration of satraplatin orally.

**29.** The packaged-pharmaceutical-product or use of claim 27 or 28, wherein said antiemetic agent is is a 5-HT3 blocker or inhibitor.

**30.** The packaged-pharmaceutical-product or use of claim 29, wherein said 5-HT3 blocker or inhibitor is granisetron.

**31.** The packaged-pharmaceutical-product or use of claim 30, wherein said granisetron is administered orally with an amount of between 0.2 mg and 5 mg.

**32.** The packaged-pharmaceutical-product or use of claim 31, wherein said granisetron is administered orally with an amount of 1 mg.

**33.** The packaged-pharmaceutical-product of any one of claims 1, and 4 to 13, wherein the instructions included in said packaged-pharmaceutical-product comprise instructions to conduct the steps of:
(a) to said individual, on each of days 1 to 5, the administration of a corticosteroid (5 mg) and antiemetic agent (1 mg) orally, followed after about 1 hour by the administration of satraplatin orally at a dose of about 80 mg/m², followed after about 8 hours by the administration of a corticosteroid (5 mg) and antiemetic agent (1 mg) orally;
(b) to said individual, on each of days 6 to 35 the administration of a corticosteroid (5 mg) twice daily in the morning and evening; and
(c) repeating (a) and (b) at least one time.

**34.** The use of any one of claims 2, and 4 to 13, wherein:
(a) to said individual, on each of days 1 to 5, a corticosteroid (5 mg) and antiemetic agent (1 mg) is administered orally, followed after about 1 hour by administration of satraplatin orally at a dose of about 80 mg/m², followed after about 8 hours by administration of a corticosteroid (5 mg) and antiemetic agent (1 mg) orally;
(b) to said individual, on each of days 6 to 35 a corticosteroid (5 mg) is administered twice daily in the morning and evening; and
(c) repeating (a) and (b) at least one time.

**35.** The packaged-pharmaceutical-product or use of claim 33 or 34, wherein said administration of satraplatin is to said individual on an empty stomach.

**36.** The packaged-pharmaceutical-product or use of any one of claims 1 to 35, wherein said administration of satraplatin results in an elongation of the time to disease progression.

**37.** The packaged-pharmaceutical-product or use of any one of claims 1 to 35, wherein said administration of satraplatin results in an elongation of the progression-free survival.

**38.** The packaged-pharmaceutical-product or use of any one of claims 1 to 35, wherein said administration of satraplatin results in an elongation of the overall survival.

**39.** The packaged-pharmaceutical-product or use of any one of claims 1 to 38, wherein said corticosteroid is prednisone.
